(19) Europäisches Patentamt
European Patent Office
Office européen des brevets

(11) EP 3 160 957 B1

(12) **EUROPEAN PATENT SPECIFICATION**

(45) Date of publication and mention of the grant of the patent:
**06.06.2018 Bulletin 2018/23**

(21) Application number: **15730199.5**

(22) Date of filing: **23.06.2015**

(51) Int Cl.:
***C07D 401/14*** (2006.01) ***C07D 413/14*** (2006.01)
***C07D 403/14*** (2006.01) ***C07D 409/14*** (2006.01)
***C07D 417/14*** (2006.01) ***C07D 471/04*** (2006.01)
***A61K 31/404*** (2006.01) ***A61K 31/42*** (2006.01)
***A61K 31/4245*** (2006.01) ***A61K 31/415*** (2006.01)
***A61K 31/4164*** (2006.01) ***A61K 31/44*** (2006.01)
***A61K 31/505*** (2006.01) ***A61P 25/00*** (2006.01)

(86) International application number:
**PCT/EP2015/064016**

(87) International publication number:
**WO 2015/197567 (30.12.2015 Gazette 2015/52)**

(54) **INDOLIN-2-ONE OR PYRROLO-PYRIDIN-2-ONE DERIVATIVES**

INDOLIN-2-ON ODER PYRROLO-PYRIDIN-2-ON-DERIVATE

DÉRIVÉS D'INDOLIN-2-ONE OU DE PYRROLOPYRIDIN-2-ONE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Validation States:
**MA**

(30) Priority: **26.06.2014 EP 14174559**

(43) Date of publication of application:
**03.05.2017 Bulletin 2017/18**

(73) Proprietor: **F. Hoffmann-La Roche AG**
**4070 Basel (CH)**

(72) Inventors:
- **HILPERT, Hans**
  **4142 Muenchenstein (CH)**
- **KOLCZEWSKI, Sabine**
  **79540 Loerrach (DE)**
- **HUMM, Roland**
  **79424 Auggen (DE)**
- **STOLL, Theodor**
  **4102 Binningen (CH)**
- **MUSER, Thorsten**
  **79541 Loerrach (DE)**
- **PLANCHER, Jean-Marc**
  **68220 Hagenthal-le-Bas (FR)**
- **GAUFRETEAU, Delphine**
  **68680 Kembs (FR)**

(74) Representative: **Salud, Carlos E. et al**
**F. Hoffmann-La Roche AG**
**Patent Department**
**Grenzacherstrasse 124**
**4070 Basel (CH)**

(56) References cited:
**WO-A1-02/00217** **WO-A1-2009/124692**
**WO-A1-2009/132774** **WO-A1-2014/040969**
**WO-A2-2008/046083**

EP 3 160 957 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

The present invention is concerned with indolin-2-one or pyrrolo-pyridin-2-one derivatives of general formula

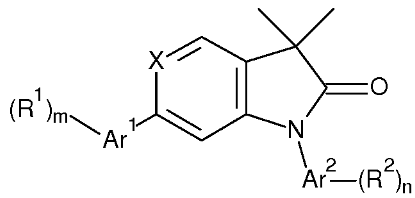

I

wherein

| | |
|---|---|
| $Ar^1$ | is phenyl or a five or six membered heteroaryl group, containing one, two or three heteroatoms, selected from N, S or O, wherein the N-heteroatom in the heteroaryl group may be oxidized to $N^+$-($O^-$); |
| $R^1$ | is lower alkyl, halogen, cyano or cycloalkyl; |
| $Ar^2$ | is a five or six membered heteroaryl group, containing one, two, three or four heteroatoms, selected from N, S or O, wherein the N-heteroatom in the heteroaryl group may be oxidized to $N^+$-($O^-$), or is benzo[b]thiophenyl; |
| $R^2$ | is hydrogen, lower alkyl, halogen, cyano, lower alkyl substituted by hydroxyl, lower alkyl substituted by halogen, lower alkyl substituted by amino, lower alkyl substituted by alkoxy, lower alkyl substituted by amide, or is cycloalkyl; |
| X | is CH or N; |
| n | is 1 or 2; |
| m | is 1 or 2; |

as well as with a pharmaceutically acceptable salt thereof, with a racemic mixture, or with its corresponding enantiomer and/or optical isomer and/or stereoisomer thereof

WO9106545 describes a close structure containing a phenyl substituted imidazole moiety for $Ar^1$, but without a heteroaryl group in the position of $Ar^2$, for prevention of clumping of both erythrocytes and thrombocytes. EP2108641 and WO2008046083 disclose a very broad scope of similar compounds which are inhibitors of the p38 nitrogen activated protein kinase for the treatment of inflammation diseases and benign prostatic hyperplasia, respectively.

Now it has been found that the compounds of formula I may be used for the treatment of CNS diseases. The described compounds have been shown to reverse the L-687,414 ((3R,4R)-3 amino-1-hydroxy-4-methyl-pyrrolidin-2-one, a NMDA glycine site antagonist) induced hyperlocomotion, a behavioral pharmacodynamic mouse model for schizophrenia, described by D. Alberati et al. in Pharmacology, Biochemistry and Behavior, 97 (2010), 185 - 191. The authors described that hyperlocomotion induced by L-687,414 was inhibited by a series of known antipsychotic drugs. The compounds of formula I demonstrate marked activity in this model. These findings predict antipsychotic activity for the present compounds, making them useful for the treatment of positive (psychosis) and negative symptoms of schizophrenia, substance abuse, alcohol and drug addiction, obsessive-compulsive disorders, cognitive impairment, bipolar disorders, mood disorders, major depression, resistant depression, anxiety disorders, Alzheimer's disease, autism, Parkinson's disease, chronic pain, borderline personality disorder, sleep disturbances, chronic fatigue syndrome, stiffness, antiinflammatory effects in arthritis and balance problems.

In addition to the reversal of L-687,414 induced hyperlocomotion experiment as described above, some compounds of the present invention have been tested in SmartCube®, an automated system in which the behaviors of compound-treated mice in response to multiple challenges are captured by digital video and analyzed with computer algorithms (Roberds et al., Frontiers in Neuroscience, 2011, Vol. 5, Art. 103, 1-4; Vadim Alexandrov, Dani Brunner, Taleen Hanania, Emer Leahy Eur. J. Pharmacol. 2015, 750, 82-99). In this way, the neuro-pharmacological effects of a test compound can be predicted by similarity to major classes of compounds, such as antipsychotics, anxiolytics and antidepressants. Examples 9, 25, 48 and 53 show similarity to atypical antipsychotics. The results are shown in Table 2.

In addition to the above-mentioned experiments, it has been shown that some of the compounds of formula I are also ENT1 inhibitors (equilibrative nucleoside transporter 1 protein). Therapeutic potential of ENT1 inhibitors is directly or indirectly (via effects of adenosine and/or adenosine receptor modulation) described in the literature for the treatment of the following diseases:

autoimmune disease (US 2006/253263), cancer (WO9857643), viral infections and fungal infections (WO2004060902), neurodegenerative disease, Parkinson's disease, Alzheimer's disease, Huntington's disease, amyotrophic lateral sclerosis, psychiatric diseases, substance abuse, ADHD, depression, epilepsy, anxiety, schizophrenia (WO0168105, EP 1252910, EP1612210, WO2009018275), autism spectrum disorders (Susan A. Masinoa, Masahito Kawamura Jr., Jessica L. Cotea, Rebecca B. Williams, David N. Ruskina, Neuropharmacology, 2013, 68, 116-121., pain (WO2009062990, WO2009064497), inflammation, asthma, (US 2007213296, Inflammation research, 2011, 60, 75-76), cardiovascular diseases (Trends in Pharmacological science, 2006, 27, 416-425), sleep disorders, (Psychopharmacology, 1987, 91, 434-439), and ophthalmology and inflammatory retinal diseases (World Journal of Diabetes, vol. 1, 12 - 18).

Schizophrenia is a complex mental disorder typically appearing in late adolescence or early adulthood with a world-wide prevalence of approximately 1 % of the adult population, which has enormous social and economic impact. The criteria of the Association of European Psychiatrists (ICD) and the American Psychiatric Association (DSM) for the diagnosis of schizophrenia require two or more characteristic symptoms to be present: delusions, hallucinations, disorganized speech, grossly disorganized or catatonic behavior (positive symptoms), or negative symptoms (alogia, affective flattening, lack of motivation, anhedonia). As a group, people with schizophrenia have functional impairments that may begin in childhood, continue throughout adult life and make most patients unable to maintain normal employment or otherwise have normal social function. They also have a shortened lifespan compared to the general population, and suffer from an increased prevalence of a wide variety of other neuropsychiatric syndromes, including substance abuse, obsessive-compulsive symptoms and abnormal involuntary movements. Schizophrenia is also associated with a wide range of cognitive impairments, bipolar disorders, major depression and anxiety disorders, the severity of which limits the functioning of patients, even when psychotic symptoms are well controlled. The primary treatment of schizophrenia is antipsychotic medications. Antipsychotics, for example risperidone, olanzapine, however, fail to significantly ameliorate the negative symptoms and cognitive dysfunction.

Antipsychotic drugs have shown clinical efficacy for the treatment of the following diseases:

Fibromyalgia, which is a syndrome characterized by chronic generalized pain associated with different somatic symptoms, such as sleep disturbances, fatigue, stiffness, balance problems, hypersensitivity to physical and psychological environmental stimuli, depression and anxiety *(*CNS Drugs, 2012, 26, 2, 135-53).

Schizoaffective disorders: includes psychotic and affective symptoms, this disorder falls on a spectrum between bipolar disorders (with depressive and manic episodes, alcohol and drug addiction, substance abuse) and schizophrenia. J. Clin. Psychiatry, 2010, 71, S2, 14-9, Pediatr. Drugs 2011, 13, 5, 291-302

Major depression: BMC Psychiatry 2011, 11, 86

Treatment resistent depression*:* Journal of Psychopharmacology, 0(0) 1- 16

Anxiety: European Neuropsychopharmacology, 2011, 21, 429-449

Bipolar disorders: Encephale, International J. of Neuropsychopharmacology, 2011, 14, 1029-104, International J. of Neuropsychopharmacology, 2012, 1-12; J. of Neuropsychopharmacology, 2011, 0, 0, 1- 15

Mood disorders: J. Psychopharmacol. 2012, Jan 11, CNS Drugs, 2010, 2, 131-61

Autism: Current opinion in pediatrics, 2011, 23, 621 - 627; J. Clin. Psychiatry, 2011, 72, 9, 1270-1276

Alzheimer's disease: J. Clin. Psychiatry, 2012, 73, 1, 121-128

Parkinson's disease: Movement Disorders, 2011, 26, 6

Chronic fatigue syndrome: European Neuropsychopharmacology, 2011, 21, 282-286

Borderline Personality disorder: J. Clin. Psychiatry, 2011, 72, 10, 1363-1365 J. Clin. Psychiatry, 2011, 72, 10, 1353-1362

Anti-inflammatory effects in arthritis: European J. of Pharmacology, 2012, 678, 55-60

Objects of the present invention are novel compounds of formula I and the use of compounds of formula I and their pharmaceutically acceptable salts for the treatment of CNS diseases related to positive (psychosis) and negative symptoms of schizophrenia, substance abuse, alcohol and drug addiction, obsessive-compulsive disorders, cognitive impairment, bipolar disorders, mood disorders, major depression, treatment resistant depression, anxiety disorders, Alzheimer's disease, autism, Parkinson's disease, chronic pain, borderline personality disorder, neurodegenerative disease, sleep disturbances, chronic fatigue syndrome, stiffness, inflammatory disease, asthma, Huntington's disease, ADHD, amyotrophic lateral sclerosis, epilepsy, effects in arthritis, autoimmune disease, viral and fungal infections, cardiovascular diseases, ophthalmology and inflammatory retinal diseases and balance problems.

Further objects of the present invention are medicaments containing such novel compounds as well as methods for preparation of compounds of formula I, a combination of compounds of formula I with marketed antipsychotics, antidepressants, anxiolytics or mood stabilizers, and methods for the treatment of CNS disorders as mentioned above.

Encompassed by the present invention are corresponding prodrugs of compounds of formula I.

A common antipsychotic drug for the treatment of schizophrenia is olanzapine. Olanzapine (Zyprexa) belongs to a drug class known as atypical antipsychotics. Other members of this class include for example clozapine (Clozaril), risperidone (Risperdal), aripiprazole (Abilify) and ziprasidone (Geodon).

Olanzapine is approved for the treatment of psychotic disorders, long term treatment of bipolar disorders and in combination with fluoxetine for the treatment of depressive episodes associated with bipolar disorders and for the treatment of resistant depression. The compounds of the present invention may be combined with antipsychotic drugs

like olanzapine (Zyprexa), clozapine (Clozaril), risperidone (Risperdal), aripiprazole (Abilify), amisulpride (Solian), asenapine (Saphris), blonanserin (Lonasen), clotiapine (Entumine), iloperidone (Fanapt), lurasidone (Latuda), mosapramine (Cremin), paliperidone (Invega), perospirone (Lullan), quetiapine (Seroquel), remoxipride (Roxiam), sertindole (Serdolect), sulpiride (Sulpirid, Eglonyl), ziprasidone (Geodon, Zeldox), zotepine (Nipolept), haloperidol (Haldol, Serenace), droperidol (Droleptan), chlorpromazine (Thorazine, Largactil), fluphenazine (Prolixin), perphenazine (Trilafon), prochlorperazine (Compazine), thioridazine (Mellaril, Melleril), trifluoperazine (Stelazine), triflupromazine (Vesprin), levomepromazine (Nozinan), promethazine (Phenergan), pimozide (Orap) and cyamemazine (Tercian).

One preferred embodiment of the invention is a combination, wherein the marketed antipsychotic drug is olanzapine (Zyprexa), clozapine (Clozaril), risperidone (Risperdal), aripiprazole (Abilify) or ziprasidone.

Furthermore, the compounds of the present invention can be combined with antidepressants such as selective serotonin reuptake inhibitors [Citalopram (Celexa), Escitalopram (Lexapro, Cipralex), Paroxetine (Paxil, Seroxat), Fluoxetine (Prozac), Fluvoxamine (Luvox), Sertraline (Zoloft, Lustral)], serotonin-norepinephrine reuptake inhibitors [Duloxetine (Cymbalta), Milnacipran (Ixel, Savella), Venlafaxine (Effexor), Desvenlafaxine (Pristiq), Tramadol (Tramal, Ultram), Sibutramine (Meridia, Reductil)], serotonin antagonist and reuptake inhibitors [Etoperidone (Axiomin, Etonin), Lubazodone (YM-992, YM-35,995), Nefazodone (Serzone, Nefadar), Trazodone (Desyrel)], norepinephrine reuptake inhibitors [Reboxetine (Edronax), Viloxazine (Vivalan), Atomoxetine (Strattera)], norepinephrine-dopamine reuptake inhibitors [Bupropion (Wellbutrin, Zyban), Dexmethylphenidate (Focalin), Methylphenidate (Ritalin, Concerta)], norepinephrine-dopamine releasing agents [Amphetamine (Adderall), Dextroamphetamine (Dexedrine), Dextromethamphetamine (Desoxyn), Lisdexamfetamine (Vyvanse)], tricyclic antidepressants [Amitriptyline (Elavil, Endep), Clomipramine (Anafranil), Desipramine (Norpramin, Pertofrane), Dosulepin [Dothiepin] (Prothiaden), Doxepin (Adapin, Sinequan), Imipramine (Tofranil), Lofepramine (Feprapax, Gamanil, Lomont), Nortriptyline (Pamelor), Protriptyline (Vivactil), Trimipramine (Surmontil)], tetracyclic antidepressants [Amoxapine (Asendin), Maprotiline (Ludiomil), Mianserin (Bolvidon, Norval, Tolvon), Mirtazapine (Remeron)], monoamine oxidase inhibitors [Isocarboxazid (Marplan), Moclobemide (Aurorix, Manerix), Phenelzine (Nardil), Selegiline [L-Deprenyl] (Eldepryl, Zelapar, Emsam), Tranylcypromine (Parnate), Pirlindole (Pirazidol)], 5-HT1A Receptor Agonists [Buspirone (Buspar), Tandospirone (Sediel), Vilazodone (Viibryd)], 5-HT2 Receptor Antagonists [Agomelatine (Valdoxan), Nefazodone (Nefadar, Serzone), selective Serotonin Reuptake Enhancers [Tianeptine].

A preferred embodiment of this invention is a combination, wherein the marketed anti-depressive drug is citalopram (Celexa), escitalopram (Lexapro, Cipralex), paroxetine (Paxil, Seroxat), fluoxetine (Prozac), sertraline (Zoloft, Lustral) duloxetine (Cymbalta), milnacipran (Ixel, Savella), venlafaxine (Effexor), or mirtazapine (Remeron).

Compounds can also be combined with anxiolytics such as Alprazolam (Helex, Xanax, Xanor, Onax, Alprox, Restyl, Tafil, Paxal), Bretazenil, Bromazepam (Lectopam, Lexotanil, Lexotan, Bromam), Brotizolam (Lendormin, Dormex, Sintonal, Noctilan), Chlordiazepoxide (Librium, Risolid, Elenium), Cinolazepam (Gerodorm), Clonazepam (Rivotril, Klonopin, Iktorivil, Paxam), Clorazepate (Tranxene, Tranxilium), Clotiazepam (Veratran, Clozan, Rize), Cloxazolam (Sepazon, Olcadil), Delorazepam (Dadumir), Diazepam (Antenex, Apaurin, Apzepam, Apozepam, Hexalid, Pax, Stesolid, Stedon, Valium, Vival, Valaxona), Estazolam (ProSom), Etizolam (Etilaam, Pasaden, Depas), Flunitrazepam (Rohypnol, Fluscand, Flunipam, Ronal, Rohydorm), Flurazepam (Dalmadorm, Dalmane), Flutoprazepam (Restas), Halazepam (Paxipam), Ketazolam (Anxon), Loprazolam (Dormonoct), Lorazepam (Ativan, Temesta, Tavor, Lorabenz), Lormetazepam (Loramet, Noctamid, Pronoctan), Medazepam (Nobrium), Midazolam (Dormicum, Versed, Hypnovel, Dormonid), Nimetazepam (Erimin), Nitrazepam (Mogadon, Alodorm, Pacisyn, Dumolid, Nitrazadon), Nordazepam (Madar, Stilny), Oxazepam (Seresta, Serax, Serenid, Serepax, Sobril, Oxabenz, Oxapax), Phenazepam (Phenazepam), Pinazepam (Domar), Prazepam (Lysanxia, Centrax), Premazepam, Quazepam (Doral), Temazepam (Restoril, Normison, Euhypnos, Temaze, Tenox), Tetrazepam (Mylostan), Triazolam (Halcion, Rilamir), Clobazam (Frisium, Urbanol), Eszopiclone (Lunesta), Zaleplon (Sonata, Starnoc), Zolpidem (Ambien, Nytamel, Stilnoct, Stilnox, Zoldem, Zolnod), Zopiclone (Imovane, Rhovane, Ximovan; Zileze; Zimoclone; Zimovane; Zopitan; Zorclone), Pregabalin (Lyrica) and Gabapentin (Fanatrex, Gabarone, Gralise, Neurontin, Nupentin).

One preferred embodiment of the invention is a combination, wherein the marketed anxiolytic drug is alprazolam (Helex, Xanax, Xanor, Onax, Alprox, Restyl, Tafil, Paxal), chlordiazepoxide (Librium, Risolid, Elenium), clonazepam (Rivotril, Klonopin, Iktorivil, Paxam), diazepam (Antenex, Apaurin, Apzepam, Apozepam, Hexalid, Pax, Stesolid, Stedon, Valium, Vival, Valaxona), Estazolam (ProSom), eszopiclone (Lunesta), zaleplon (Sonata, Starnoc), zolpidem (Ambien, Nytamel, Stilnoct, Stilnox, Zoldem, Zolnod), pregabalin (Lyrica) or gabapentin (Fanatrex, Gabarone, Gralise, Neurontin, Nupentin).

A further object of the invention is a combination with mood stabilizers such as Carbamazepine (Tegretol), Lamotrigine (Lamictal), Lithium (Eskalith, Lithane, Lithobid), and Valproic Acid (Depakote).

Compounds can also be combined with procognitive compounds such as donepezil (Aricept), galantamine (Razadyne), rivastigmine (Exelon) and memantine (Namenda).

The preferred indications using the compounds of the present invention are psychotic diseases like schizophrenia.

As used herein, the term "lower alkyl" denotes a saturated straight- or branched-chain group containing from 1 to 7 carbon atoms, for example, methyl, ethyl, propyl, isopropyl, n-butyl, i-butyl, 2-butyl, t-butyl and the like. Preferred alkyl groups are groups with 1 - 4 carbon atoms.

As used herein, the term "lower alkoxy" denotes an alkyl group as defined above, wherein the alkyl residue is attached via an oxygen atom.

As used herein, the term "lower alkyl substituted by hydroxy" denotes a group wherein the alkyl residue is as defined above, wherein at least one hydrogen atom is replaced by a hydroxy group.

As used herein, the term "lower alkyl substituted by halogen" denotes a group wherein the alkyl residue is as defined above, wherein at least one hydrogen atom is replaced by a halogen atom.

As used herein, the term "lower alkyl substituted by amino" denotes a group wherein the alkyl residue is as defined above, wherein at least one hydrogen atom is replaced by $NH_2$.

As used herein, the term "lower alkyl substituted by amide" denotes a group wherein the alkyl residue is as defined above, wherein at least one hydrogen atom is replaced by $C(O)N(CH_3)_2$ or $C(O)NH_2$.

As used herein, the term "lower alkyl substituted by alkoxy" denotes a group wherein the alkyl residue is as defined above, wherein at least one hydrogen atom is replaced an alkoxy group.

The term "cycloalkyl" denotes an alkyl ring with 3 - 6 carbon ring atoms.

The term "halogen" denotes chlorine, iodine, fluorine and bromine.

The term "five or six membered heteroaryl group, containing one, two, three or four heteroatoms, selected from N, S or O" denotes aromatic rings, selected from the group consisting of pyridinyl, pyrimidinyl, pyridazinyl, pyrazinyl, imidazolyl, pyrazolyl, thiazolyl, 1,3,4-oxadiazolyl, 1,2,4-oxadiazolyl, 1,2,4-triazolyl, 1,2,3-triazolyl, isoxazolyl, tetrazolyl, 1,2,4-thiadiazolyl, isothiazolyl or oxazolyl.

The term "wherein the N-heteroatom in the heteroaryl group may be oxidized to $N^{+}$-($O^{-}$)" denotes for example the following groups

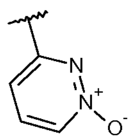 or 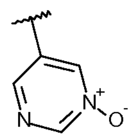 .

The term "pharmaceutically acceptable acid addition salts" embraces salts with inorganic and organic acids, such as hydrochloric acid, nitric acid, sulfuric acid, phosphoric acid, citric acid, formic acid, fumaric acid, maleic acid, acetic acid, succinic acid, tartaric acid, methanesulfonic acid, p-toluenesulfonic acid and the like.

One embodiment of the invention are compounds, wherein X is CH. A further embodiment of the invention are compounds from this group, wherein $Ar^1$ and $Ar^2$ are both a six membered heteroaryl group, containing one, two or three heteroatoms, selected from N, S or O, for example the compounds

3,3-Dimethyl-6-(2-methylpyrimidin-5-yl)-1-(pyridin-4-yl)indolin-2-one
3,3-Dimethyl-6-(2-methylpyrimidin-5-yl)-1-(pyridin-3-yl)indolin-2-one
3,3-Dimethyl-1-(2-methylpyridin-4-yl)-6-(2-methylpyrimidin-5-yl)indolin-2-one
3,3-Dimethyl-1-(6-methylpyrimidin-4-yl)-6-(2-methylpyrimidin-5-yl)indolin-2-one
3,3-Dimethyl-6-(2-methylpyrimidin-5-yl)-1-(pyrimidin-5-yl)indolin-2-one
3,3-Dimethyl-1,6-bis(2-methylpyrimidin-5-yl)indolin-2-one
3,3-Dimethyl-1-(6-methylpyridin-3-yl)-6-(2-methylpyrimidin-5-yl)indolin-2-one
3,3-Dimethyl-6-(6-methylpyridin-3-yl)-1-(2-methylpyridin-4-yl)indolin-2-one
3,3-Dimethyl-1,6-bis(2-methylpyridin-4-yl)indolin-2-one
6-(4-Fluoropyridin-3-yl)-3,3-dimethyl-1-(2-methylpyridin-4-yl)indolin-2- one
1-(5-Fluoro-2-methylpyridin-4-yl)-3,3-dimethyl-6-(2-methylpyrimidin-5-yl)indolin-2-one
5-(3,3-Dimethyl-6-(2-methylpyrimidin-5-yl)-2-oxoindolin-1-yl)picolinonitrile
1-(6-(Hydroxymethyl)pyridin-3-yl)-3,3-dimethyl-6-(2-methylpyrimidin-5-yl)indolin-2-one
1-(6-Cyclopropylpyridin-3-yl)-3,3-dimethyl-6-(2-methylpyrimidin-5-yl)indolin-2-one
3,3-Dimethyl-6-(2-methylpyrimidin-5-yl)-1-(pyridin-2-yl)indolin-2-one
1-(2-Fluoropyridin-4-yl)-3,3-dimethyl-6-(2-methylpyrimidin-5-yl)indolin-2-one
1-(3-Fluoropyridin-4-yl)-3,3-dimethyl-6-(2-methylpyrimidin-5-yl)indolin-2-one
1-(2-Fluoro-5-methylpyridin-4-yl)-3,3-dimethyl-6-(2-methylpyrimidin-5-yl)indolin-2-one
3,3-Dimethyl-1-(6-methylpyridazin-3-yl)-6-(2-methylpyrimidin-5-yl)indolin-2-one
1-(3-Chloropyridin-4-yl)-3,3-dimethyl-6-(2-methylpyrimidin-5-yl)indolin-2-one
3,3-Dimethyl-1-(5-methylpyrimidin-2-yl)-6-(2-methylpyrimidin-5-yl)indolin-2-one

3,3-Dimethyl-1-(5-methylpyridin-2-yl)-6-(2-methylpyrimidin-5-yl)indolin-2-one
3,3-Dimethyl-1-(5-methylpyrazin-2-yl)-6-(2-methylpyrimidin-5-yl)indolin-2-one
3,3-Dimethyl-1-(5-methylpyridin-3-yl)-6-(2-methylpyrimidin-5-yl)indolin-2-one
3,3-Dimethyl-1-(4-methylpyridin-2-yl)-6-(2-methylpyrimidin-5-yl)indolin-2-one
3,3-Dimethyl-1-(6-methylpyridin-2-yl)-6-(2-methylpyrimidin-5-yl)indolin-2-one
3,3-Dimethyl-1-(2-methylpyrimidin-4-yl)-6-(2-methylpyrimidin-5-yl)indolin-2-one
3,3-Dimethyl-1-(6-methylpyrazin-2-yl)-6-(2-methylpyrimidin-5-yl)indolin-2-one
3,3-Dimethyl-1-(4-methylpyrimidin-2-yl)-6-(2-methylpyrimidin-5-yl)indolin-2-one
1-(2,6-Dimethylpyridin-4-yl)-3,3-dimethyl-6-(2-methylpyrimidin-5-yl)indolin-2-one
1-(4,6-Dimethylpyrimidin-2-yl)-3,3-dimethyl-6-(2-methylpyrimidin-5-yl)indolin-2-one
1-(2,6-Dimethylpyrimidin-4-yl)-3,3-dimethyl-6-(2-methylpyrimidin-5-yl)indolin-2-one
1-(4,5-Dimethylpyridin-2-yl)-3,3-dimethyl-6-(2-methylpyrimidin-5-yl)indolin-2-one
1-(5,6-dimethylpyridin-2-yl)-3,3-dimethyl-6-(2-methylpyrimidin-5-yl)indolin-2-one
1-(5,6-dimethylpyridin-3-yl)-3,3-dimethyl-6-(2-methylpyrimidin-5-yl)indolin-2-one
3,3-dimethyl-6-(2-methylpyrimidin-5-yl)-1-(pyridazin-3-yl)indolin-2-one
3,3-dimethyl-6-(2-methylpyrimidin-5-yl)-1-(pyrazin-2-yl)indolin-2-one
3,3-dimethyl-6-(2-methylpyrimidin-5-yl)-1-(pyrimidin-2-yl)indolin-2-one
3,3-dimethyl-6-(2-methylpyrimidin-5-yl)-1-(4-methylpyrimidin-5-yl)indolin-2-one
3,3-dimethyl-1,6-bis(5-methylpyrimidin-2-yl)indolin-2-one
3,3-dimethyl-1,6-bis(5-methylpyrazin-2-yl)mdolm-2-one
3,3-dimethyl-6-(2-methylpyrimidin-5-yl)-1-(pyrimidin-4-yl)indolin-2-one
1-(5-cyclopropylpyrazin-2-yl)-3,3-dimethyl-6-(2-methylpyrimidin-5-yl)indolin-2-one
5-(3,3-dimethyl-6-(2-methylpyrimidin-5-yl)-2-oxoindolin-1-yl)pyrazine-2-carbonitrile
1-(6-cyclopropylpyrazin-2-yl)-3,3-dimethyl-6-(2-methylpyrimidin-5-yl)indolin-2-one
1-(4,5-dimethylpyridin-3-yl)-3,3-dimethyl-6-(2-methylpyrimidin-5-yl)indolin-2-one
1-(4,5-dimethylpyridin-3-yl)-3,3-dimethyl-6-(2-methylpyrimidin-5-yl)indolin-2-one
1-(4,6-dimethylpyridin-3-yl)-3,3-dimethyl-6-(2-methylpyrimidin-5-yl)indolin-2-one
5-(3,3-dimethyl-1-(5-methylpyrimidin-2-yl)-2-oxoindolin-6-yl)-2-methylpyrimidine 1-oxide
1-(2-(hydroxymethyl)pyrimidin-5-yl)-3,3-dimethyl-6-(2-methylpyrimidin-5-yl)indolin-2-one
1-[2-(aminomethyl)pyrimidin-5-yl]-3,3-dimethyl-6-(2-methylpyrimidin-5-yl)indol-2-one
3-(3,3-dimethyl-6-(2-methylpyrimidin-5-yl)-2-oxoindolin-1-yl)-6-methylpyridazine 1-oxide
3-(3,3-dimethyl-6-(2-methyl-1-oxidopyrimidin-5-yl)-2-oxoindolin-1-yl)-6-methylpyridazine 1-oxide
1-(2-(fluoromethyl)pyrimidin-5-yl)-3,3-dimethyl-6-(2-methylpyrimidin-5-yl)indolin-2-one
3,3-dimethyl-1-(6-methylpyridazin-3-yl)-6-(2-methylpyridin-4-yl)-1H-pyrrolo[3,2-c]pyridin-2(3H)-one
6-(4-fluorophenyl)-3,3-dimethyl-1-(2-methylpyrimidin-5-yl)-1H-pyrrolo[3,2-c]pyridin-2(3H)-one
1-(5-chloropyrimidin-2-yl)-3,3-dimethyl-6-(2-methylpyrimidin-5-yl)indolin-2-one
1-(2-chloropyrimidin-5-yl)-3,3-dimethyl-6-(2-methylpyrimidin-5-yl)indolin-2-one
1-(2,6-dichloropyridin-4-yl)-3,3-dimethyl-6-(2-methylpyrimidin-5-yl)indolin-2-one
1-(2-cyclopropylpyrimidin-5-yl)-3,3-dimethyl-6-(2-methylpyrimidin-5-yl)indolin-2-one
1-(5-chloropyrazin-2-yl)-3,3-dimethyl-6-(2-methylpyrimidin-5-yl)indol-2-one
1-(6-chloropyridazin-3-yl)-3,3-dimethyl-6-(2-methylpyrimidin-5-yl)indolin-2-one
1-(2-chloro-6-methylpyridin-4-yl)-3,3-dimethyl-6-(2-methylpyrimidin-5-yl)indolin-2-one
3,3-dimethyl-6-(1-methylpyrimidin-5-yl)-1-(pyridazin-4-yl)indolin-1-one
1-(6-chloro-2-methylpyrimidin-4-yl)-3,3-dimethyl-6-(2-methylpyrimidin-5-yl)indolin-2-one
3,3-dimethyl-1-(5-methylpyridazin-3-yl)-6-(2-methylpyrimidin-5-yl)indolin-2-one
1-(6-chloropyridazin-4-yl)-3,3-dimethyl-6-(2-methylpyrimidin-5-yl)indolin-2-one
3,3-dimethyl-1-(3-methylpyrazin-2-yl)-6-(2-methylpyrimidin-5-yl)indolin-2-one
1-(4-chloropyrimidin-2-yl)-3,3-dimethyl-6-(2-methylpyrimidin-5-yl)indolin-2-one
1-(6-(methoxymethyl)pyridin-3-yl)-3,3-dimethyl-6-(2-methylpyrimidin-5-yl)indolin-2-one
1-(5-cyclopropylpyridazin-3-yl)-3,3-dimethyl-6-(2-methylpyrimidin-5-yl)indolin-2-one
3,3-dimethyl-6-(5-methylpyrazin-2-yl)-1-(6-methylpyrazin-2-yl)indolin-2-one
3,3-dimethyl-6-(5-methylpyrazin-2-yl)-1-(2-methylpyrimidin-5-yl)indolin-2-one
1-(6-cyclopropylpyridazin-3-yl)-3,3-dimethyl-6-(2-methylpyrimidin-5-yl)indolin-2-one
3,3-dimethyl-1-(6-methylpyrazin-2-yl)-6-(6-methyl-2-pyridyl)indolin-2-one
3,3-dimethyl-1-(5-methylpyrazin-2-yl)-6-(2-methylpyrimidin-4-yl)indolin-2-one
3,3-dimethyl-6-(5-methylpyrazin-2-yl)-1-(6-methylpyridazin-3-yl)indolin-2-one
1-(5-fluoropyridin-3-yl)-3,3-dimethyl-6-(2-methylpyrimidin-5-yl)indolin-2-one
5-(3,3-dimethyl-6-(2-methylpyrimidin-5-yl)-2-oxoindolin-1-yl)nicotinonitrile

3,3-dimethyl-1-(6-methylpyrazin-2-yl)-6-(2-methyl-4-pyridyl)indolin-2-one
3,3-dimethyl-1-(6-methylpyrazin-2-yl)-6-(5-methyl-3-pyridyl)indolin-2-one
6-(5-fluoro-3-pyridyl)-3,3-dimethyl-1-(6-methylpyrazin-2-yl)indolin-2-one
3,3-dimethyl-1-(5-methylpyrazin-2-yl)-6-(2-methyl-4-pyridyl)indolin-2-one
3,3-dimethyl-1-(5-methylpyrazin-2-yl)-6-(6-methyl-3-pyridyl)indolin-2-one
3,3-dimethyl-1-(5-methylpyrazin-2-yl)-6-(5-methyl-3-pyridyl)indolin-2-one
6-(5-fluoro-3-pyridyl)-3,3-dimethyl-1-(5-methylpyrazin-2-yl)indolin-2-one
6-(5-fluoro-6-methyl-3-pyridyl)-3,3-dimethyl-1-(5-methylpyrazin-2-yl)indolin-2-one
3,3-dimethyl-6-(2-methylpyrimidin-5-yl)-1-(5-(trifluoromethyl)pyridin-3-yl)indolin-2-one
1-(5-(hydroxymethyl)pyridin-3-yl)-3,3-dimethyl-6-(2-methylpyrimidin-5-yl)indolin-2-one
3,3-dimethyl-1-(6-methylpyrazin-2-yl)-6-(2-methylpyrimidin-4-yl)indolin-2-one
5-[3,3-dimethyl-1-(6-methylpyrazin-2-yl)-2-oxo-indolin-6-yl]pyrimidine-2-carbonitrile or
1-(5-ethylpyridin-3-yl)-3,3-dimethyl-6-(2-methylpyrimidin-5-yl)indolin-2-one.

A further embodiment of the invention are compounds from the group (X=CH), wherein $Ar^1$ is a six membered heteroaryl group, containing one, two or three heteroatoms, selected from N, S or O, and $Ar^2$ is five membered heteroaryl group, containing one, two or three heteroatoms, selected from N, S or O, for example the compounds

3,3-Dimethyl-1-(1-methyl-1H-imidazol-4-yl)-6-(2-methylpyrimidin-5-yl)indolin-2-one
3,3-Dimethyl-1-(1-methyl-1H-pyrazol-3-yl)-6-(2-methylpyrimidin-5-yl)indolin-2-one
1-(1,5-Dimethyl-1H-pyrazol-3-yl)-3,3-dimethyl-6-(2-methylpyrimidin-5-yl)indolin-2-one
3,3-Dimethyl-1-(1-methyl-1H-imidazol-4-yl)-6-(6-methylpyridin-3-yl)indolin-2-one
3,3-Dimethyl-1-(1-methyl-1-methyl-1H-imidazol-4-yl)-6-(2-methylpyridin-4-yl)indolin-2-one
3,3-Dimethyl-1-(1-methyl-1H-pyrazol-3-yl)-6-(6-methylpyridin-3-yl)indolin-2-one
6-(4-Fluoropyridin-3-yl)-3,3-dimethyl-1-(1-methyl-1H-imidazol-4-yl)indolin-2-one
6-(4-Fluoropyridin-3-yl)-3,3-dimethyl-1-(1-methyl-1H-pyrazol-3-yl)indolin-2-one
3,3-Dimethyl-6-(2-methylpyrimidin-5-yl)-1-(5-methylthiophen-2-yl)indolin-2-one
3,3-Dimethyl-1-(1-methyl-1H-imidazol-5-yl)-6-(2-methylpyrimidin-5-yl)indolin-2-one
3,3-Dimethyl-1-(5-methyl-1,3,4-oxadiazol-2-yl)-6-(2-methylpyrimidin-5-yl)indolin-2-one
3,3-Dimethyl-6-(2-methylpyrimidin-5-yl)-1-(1H-pyrazol-3-yl)indolin-2-one
1-(1,2-Dimethyl-1H-imidazol-4-yl)-3,3-dimethyl-6-(2-methylpyrimidin-5-yl)indolin-2-one
1-(5-ethylpyridin-3-yl)-3,3-dimethyl-6-(2-methylpyrimidin-5-yl)indolin-2-one
1-(1,5-dimethyl-1H-1,2,4-triazol-3-yl)-3,3-dimethyl-6-(2-methylpyrimidin-5-yl)indolin-2-one
3,3-dimethyl-1-(1-methyl-1H-pyrazol-4-yl)-6-(2-methylpyrimidin-5-yl)indolin-2-one
3,3-dimethyl-1-(2-methyl-1H-imidazol-4-yl)-6-(2-methylpyrimidin-5-yl)indolin-2-one
3,3-dimethyl-1-(1-methyl-1H-1,2,4-triazol-3-yl)-6-(2-methylpyrimidin-5-yl)indolin-2-one
3,3-dimethyl-6-(2-methylpyrimidin-5-yl)-1-(1H-pyrazol-4-yl)indolin-2-one
1-(1H-imidazol-4-yl)-3,3-dimethyl-6-(2-methylpyrimidin-5-yl)indolin-2-one
3,3-dimethyl-1-(3-methyl-1H-pyrazol-5-yl)-6-(2-methylpyrimidin-5-yl)indolin-2-one
3,3-dimethyl-1-(1-methyl-1H-1,2,3-triazol-4-yl)-6-(2-methylpyrimidin-5-yl)indolin-2-one
3,3-dimethyl-1-(4-methyl-1H-imidazol-2-yl)-6-(2-methylpyrimidin-5-yl)indolin-2-one
3,3-dimethyl-1-(3-methylisoxazol-5-yl)-6-(2-methylpyrimidin-5-yl)indolin-2-one
1-(1H-imidazol-2-yl)-3,3-dimethyl-6-(2-methylpyrimidin-5-yl)indolin-2-one
1-(1-ethyl-1H-pyrazol-3-yl)-3,3-dimethyl-6-(2-methylpyrimidin-5-yl)indolin-2-one
3,3-dimethyl-6-(2-methylpyrimidin-5-yl)-1-(2H-tetrazol-5-yl)indolin-2-one
3,3-dimethyl-1-(2-methyl-2H-tetrazol-5-yl)-6-(2-methylpyrimidin-5-yl)indolin-2-one
3,3-dimethyl-1-(1-methyl-1H-pyrazol-4-yl)-6-(2-methylpyridin-4-yl)-1H-pyrrolo[3,2-c]pyridin-2(3H)-one
3,3-dimethyl-1-(1-methyl-1H-imidazol-4-yl)-6-(2-methylpyridin-4-yl)-1H-pyrrolo[3,2-c]pyridin-2(3H)-one
3,3-dimethyl-1-(3-methyl-1,2,4-thiadiazol-5-yl)-6-(2-methylpyrimidin-5-yl)indolin-2-one
3,3-dimethyl-1-(3-methylisothiazol-5-yl)-6-(2-methylpyrimidin-5-yl)indolin-2-one
3,3-dimethyl-6-(2-methylpyrimidin-5-yl)-1-(5-methylthiazol-2-yl)indolin-2-one
1-(1-isopropyl-1H-pyrazol-3-yl)-3,3-dimethyl-6-(2-methylpyrimidin-5-yl)indolin-2-one
3,3-dimethyl-1-(1-methyl-1H-pyrazol-3-yl)-6-(5-methylpyrazin-2-yl)indolin-2-one
3,3-dimethyl-6-(2-methylpyrimidin-5-yl)-1-(1-(2,2,2-trifluoroethyl)-1H-pyrazol-3-yl)indolin-2-one
1-(1-(2-methoxyethyl)-1H-pyrazol-3-yl)-3,3-dimethyl-6-(2-methylpyrimidin-5-yl)indolin-2-one or
2-(3-(3,3-dimethyl-6-(2-methylpyrimidin-5-yl)-2-oxoindolin-1-yl)-1H-pyrazol-1-yl)-N,N-dimethylacetamide.

A further embodiment of the invention are compounds from the group (X=CH), wherein $Ar^1$ is a five membered

heteroaryl group, containing one, two or three heteroatoms, selected from N, S or O, and $Ar^2$ is a six membered heteroaryl group, containing one, two or three heteroatoms, selected from N, S or O, for example the compounds

3,3-Dimethyl-6-(4-methylimidazol-1-yl)-1-(6-methyl-3-pyridyl)indolin-2-one
6-(4-Cyclopropylimidazol-1-yl)-3,3-dimethyl-1-(2-methyl-4-pyridyl)indolin-2-one
3,3-Dimethyl-6-(4-methylimidazol-1-yl)-1-(2-methyl-4-pyridyl)indolin-2-one
3,3-Dimethyl-6-(1-methyl-1H-pyrazol-3-yl)-1-(2-methylpyrimidin-5-yl)indolin-2-one
3,3-Dimethyl-6-(1-methyl-1H-imidazol-4-yl)-1-(2-methylpyrimidin-5-yl)indolin-2-one
6-(4-Isopropylimidazol-1-yl)-3,3-dimethyl-1-(6-methyl-3-pyridyl)indolin-2-one
3,3-Dimethyl-6-(5-methyl-1,3,4-oxadiazol-2-yl)-1-(6-methyl-3-pyridyl)indolin-2-one
3,3-Dimethyl-6-(5-methyl-1,3,4-oxadiazol-2-yl)-1-(2-methyl-4-pyridyl)indolin-2-one
6-(4-Isopropylimidazol-1-yl)-3,3-dimethyl-1-(2-methyl-4-pyridyl)indolin-2-one
6-(4-Isopropylimidazol-1-yl)-3,3-dimethyl-1-(2-methylpyrimidin-5-yl)indolin-2-one
6-(4-Cyclopropylimidazol-1-yl)-3,3-dimethyl-1-(2-methylpyrimidin-5-yl)indolin-2-one
3,3-Dimethyl-6-(5-methyl-1,3,4-oxadiazol-2-yl)-1-(2-methylpyrimidin-5-yl)indolin-2-one
3,3-Dimethyl-6-(4-methylimidazol-1-yl)-1-(2-methylpyrimidin-5-yl)indolin-2-one
3,3-dimethyl-6-(2-methyloxazol-5-yl)-1-(2-methylpyrimidin-5-yl)indolin-2-one or
3,3-dimethyl-6-(3-methyl-1,2,4-oxadiazol-5-yl)-1-(2-methylpyrimidin-5-yl)indolin-2-one.

A further embodiment of the invention are compounds from the group (X=CH), wherein $Ar^1$ and $Ar^2$ are both a five membered heteroaryl group, containing one, two or three heteroatoms, selected from N, S or O, for example the compounds

6-(4-Isopropylimidazol-1-yl)-3,3-dimethyl-1-(1-methylpyrazol-3-yl)indolin-2-one
6-(4-Cyclopropylimidazol-1-yl)-3,3-dimethyl-1-(1-methylimidazol-4-yl)indolin-2-one
6-(4-Cyclopropylimidazol-1-yl)-3,3-dimethyl-1-(1-methylpyrazol-3-yl)indolin-2-one
3,3-Dimethyl-1-(1-methylimidazol-4-yl)-6-(2-methyloxazol-5-yl)indolin-2-one
3,3-Dimethyl-6-(2-methyloxazol-5-yl)-1-(1-methylpyrazol-3-yl)indolin-2-one
3,3-Dimethyl-1-(1-methylimidazol-4-yl)-6-(5-methyl-1,3,4-oxadiazol-2-yl)indolin-2-one or
3,3-dimethyl-6-(4-methyl-1H-imidazol-1-yl)-1-(1-methyl-1H-imidazol-4-yl)indolin-2-one.

A further embodiment of the invention are compounds from the group (X=CH), wherein $Ar^2$ is benzo[b]thiophenyl, and the other substituents are as described above, for example the compound
1-(Benzo[b]thiophen-4-yl)-3,3-dimethyl-6-(2-methylpyrimidin-5-yl)indolin-2-one.

One embodiment of the invention are compounds, wherein X is N and the other substituents are as described above, for example the compounds

3,3-Dimethyl-1-(1-methyl-1H-imidazol-4-yl)-6-(2-methylpyrimidin-5-yl)-1H-pyrrolo[3,2-c]pyridin-2(3H)-one
3,3-Dimethyl-1-(1-methyl-1H-pyrazol-3-yl)-6-(2-methylpyrimidin-5-yl)-1H-pyrrolo[3,2-c]pyridin-2(3H)-one
3,3-Dimethyl-1-(2-methylpyridin-4-yl)-6-(2-methylpyrimidin-5-yl)-1H-pyrrolo[3,2-c]pyridin-2(3H)-one
3,3-dimethyl-1-(6-methylpyridazin-3-yl)-6-(2-methylpyridin-4-yl)-1H-pyrrolo[3,2-c]pyridin-2(3H)-one
3,3-dimethyl-1-(1-methyl-1H-pyrazol-4-yl)-6-(2-methylpyridin-4-yl)-1H-pyrrolo[3,2-c]pyridin-2(3H)-one
3,3-dimethyl-1-(1-methyl-1H-imidazol-4-yl)-6-(2-methylpyridin-4-yl)-1H-pyrrolo[3,2-c]pyridin-2(3H)-one or
6-(4-fluorophenyl)-3,3-dimethyl-1-(2-methylpyrimidin-5-yl)-1H-pyrrolo[3,2-c]pyridin-2(3H)-one.

The present compounds of formula I and their pharmaceutically acceptable salts can be prepared by methods known in the art, for example, by processes described below, which processes comprise

a) reacting a compound of formula

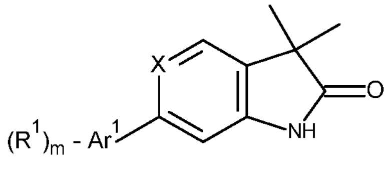

7

with a compound of formula

$(R^2)_n$-$Ar^2$-Y 8

to a compound of formula

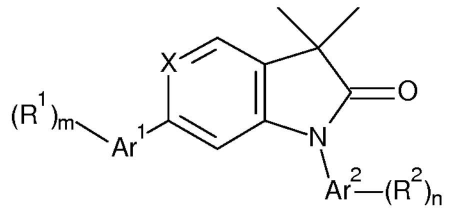

**I**

wherein Y is Cl, Br or I and the other groups have the meaning as described above and,
if desired, converting the compounds obtained into pharmaceutically acceptable acid addition salts;

The preparation of compounds of formula **I** of the present invention may be carried out in sequential or convergent synthetic routes. Syntheses of the compounds of the invention are shown in the following schemes. The skills required for carrying out the reaction and purification of the resulting products are known to those skilled in the art. The substituents and indices used in the following description of the processes have the significance given herein before unless indicated to the contrary.

In more detail, the compounds of formula **I** can be manufactured by the methods given below, by the methods given in the examples or by analogous methods. Appropriate reaction conditions for the individual reaction steps are known to a person skilled in the art. The reaction sequence is not limited to the one displayed in the schemes, however, depending on the starting materials and their respective reactivity the sequence of reaction steps can be freely altered. Starting materials are either commercially available or can be prepared by methods analogous to the methods given below, by methods described in the examples, or by methods known in the art.

Scheme 1

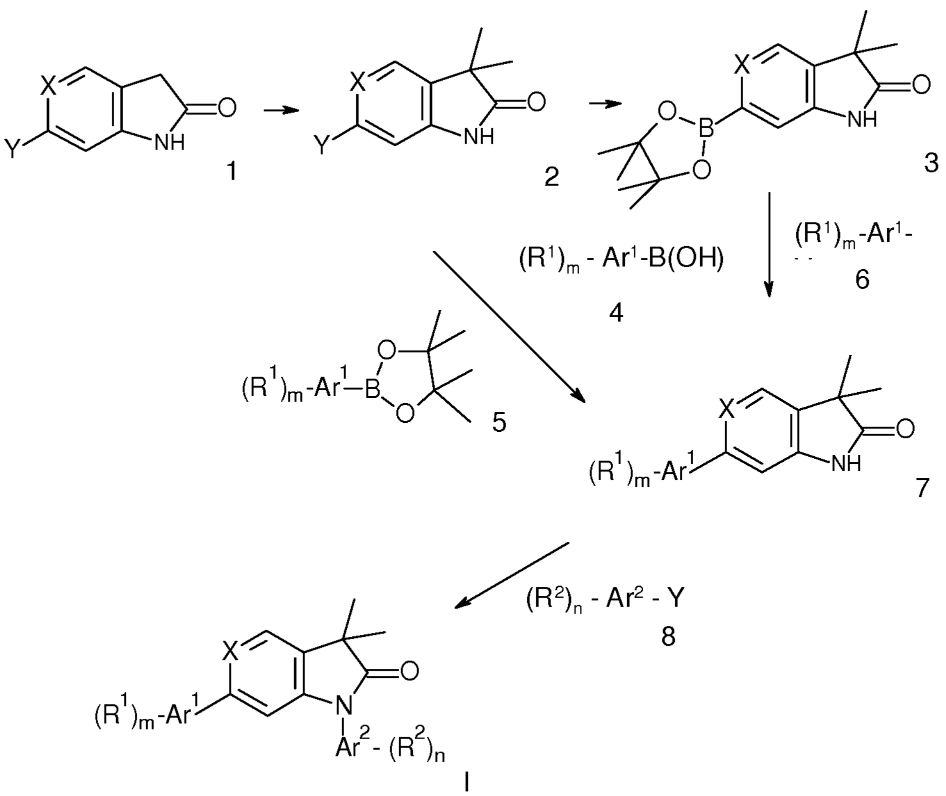

Compound of formula **I** with $Ar^1$ = unsubstituted and substituted pyrimidines, pyridines, pyrazole and imidazoles and X = CH or N (Scheme 1) can be prepared by dimethylation of 6-halo-oxindoles **1** (Y = Cl, Br, I) with Me-LG (LG being a leaving group like iodide, bromide, chloride, tosylate) in the presence of a base like potassium tert-butoxide and copper(I)bromide-dimethylsulfide complex to give compounds **2.** Compounds of general formula **2** can be coupled with boronic acids **4** or boronic esters **5** in the presence of a palladium catalyst, e.g [1,1'-bis(diphenylphosphino)ferrocene] dichloropalladium(II) and a base, e.g. potassium acetate or sodium carbonate to give arylated compounds **7.** Alternatively, compounds **7** can be prepared via boronic esters **3** followed by coupling with aryl-halogenides **6** (Y = Cl, Br, I). Introduction of the second aryl residue ($Ar^2$-$(R^2)_n$) can be accomplished by coupling compounds **7** with aryl-halogenides **8** in the presence of copper(I)iodide, a ligand such as N,N'-dimethylethylendiamine and a base, e.g. potassium carbonate to give compounds of formula **I.**

Scheme 2

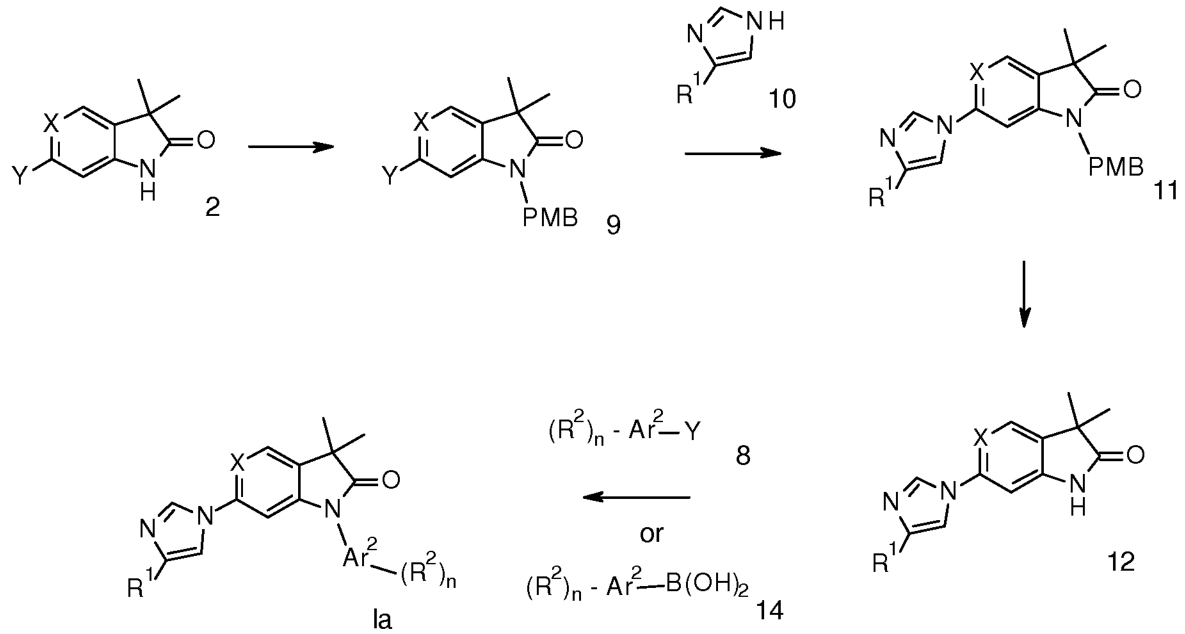


Imidazoles of formula **I**a (Scheme 2) can be prepared from halogen-oxindoles **2** (Y = Cl, Br, I) by protection of the amine group using the p-methoxybenzyl protecting group (PMB), which is followed by coupling of the protected product **9** with a substituted imidazole **10** ($R^1$ = alkyl or cycloalkyl) in the presence of copper bromide, a ketone such as 2-acetyl-cyclohexanone and a base, e.g. potassium carbonate to give the PMB-protected imidazoles **11.** Deprotection of the PMB group can be effected with a strong acid, e.g. TFA furnishing the indolinone **12.** Coupling of **12** with a substituted aryl halogenide **8** (Y = Cl, Br, I) can be effected in the presence of copper iodide, a ligand such as N,N'-dimethylethylenediamine and a base, e.g potassium carbonate or with a boronic acid **14,** copper acetate and a base such as sodium bis(trimethylsilyl)amide affording the targeted imidazoles **Ia.**

Scheme 3

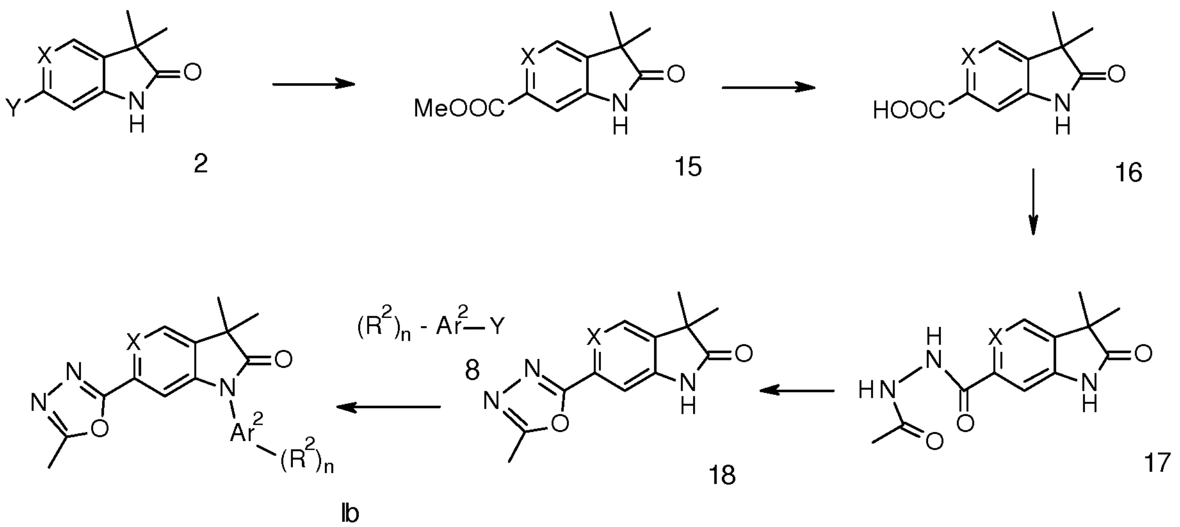


Oxadiazoles of formula **Ib** (Scheme 3) can be prepared from halogen-oxindoles **2** (Y = Cl, Br, I) by carbonylation with carbon monoxide in methanol and in the presence of a ferrocene-palladium catalyst. Hydrolysis of the methyl ester **15** using e.g. sodium hydroxide yields acid **16,** which can be reacted with acetyl hydrazide in the presence of EDCI and 1H-benzo[d][1,2,3]triazol-1-ol furnishing acetylhydrazide **17.** Cyclization of **17** using p-toluensulfonyl cloride affords oxadiazole **18,** which can be reacted with a substituted aryl halogenide (Y = Cl, Br, I) furnishing compounds of formula **Ib.**

Scheme 4

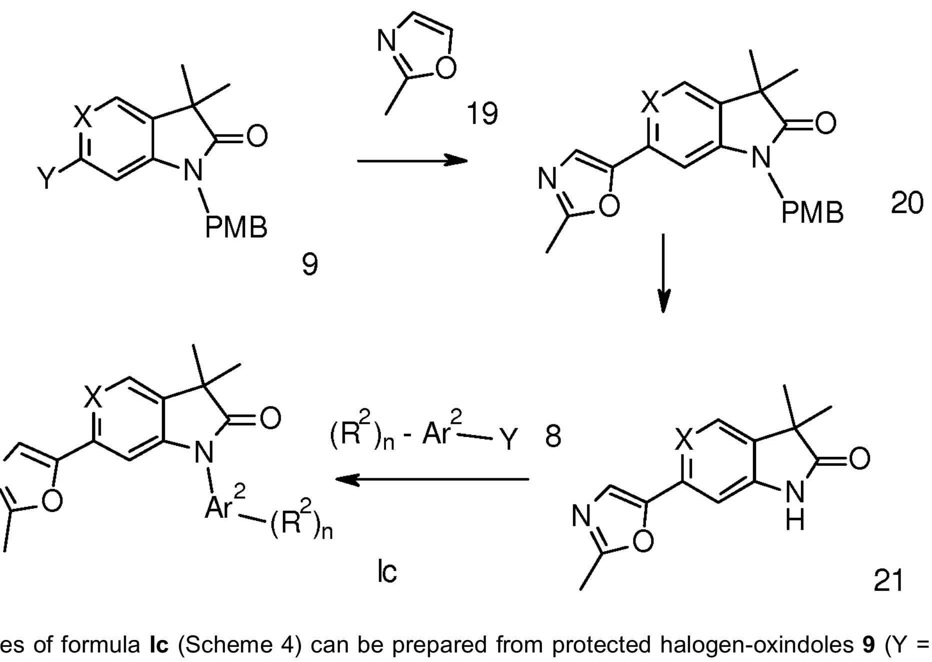


Oxazoles of formula **Ic** (Scheme 4) can be prepared from protected halogen-oxindoles **9** (Y = Cl, Br, I) and oxazole **19** in the presence of palladium diacetate and 2-dicyclohexylphosphino)biphenyl and a base, e.g. potassium carbonate to give the protected oxazole **20.** Deprotection of the PMB group can be effected with a strong acid, e.g. TFA affording the indolinone **21.** Coupling of **21** with a substituted aryl halogenide **8** (Y = Cl, Br, I) can be accomplished in the presence of copper iodide, a ligand such as N,N'-dimethylethylenediamine and a base, e.g. potassium carbonate affording the targeted oxazoles **Ic.**

Scheme 5

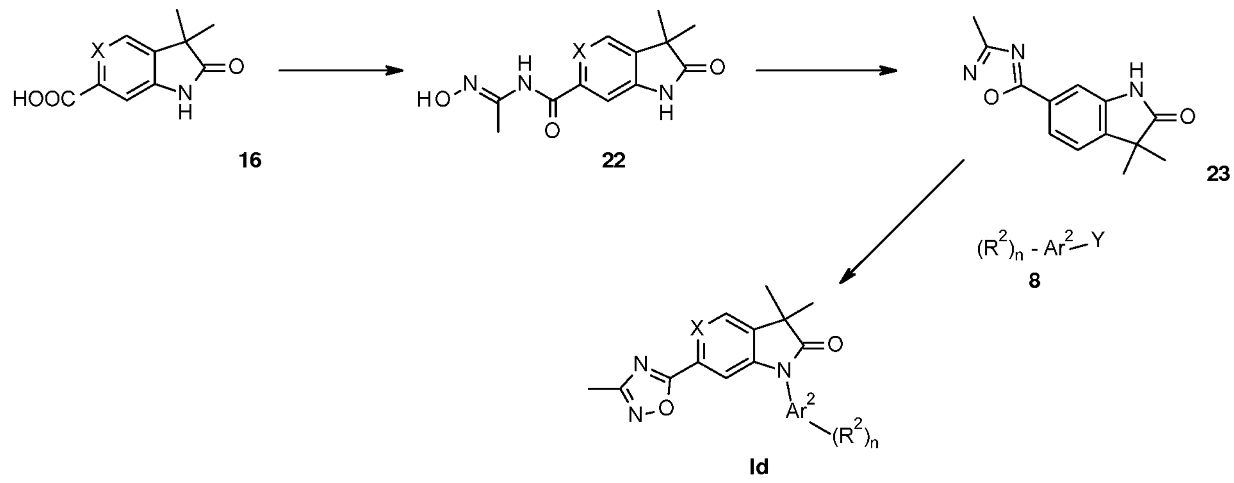

16 22 23 8 Id

The carboxylic acid **16,** described above in Scheme 3, can converted to the N-hydroxy-C-methyl-carbonimidoyl derivative **22** for example by addition of N-hydroxy-acetamidine and subsequently deshydrated for example upon heating to the corresponding -[1,2,4]oxadiazole **23.** Coupling of **23** with a substituted aryl halogenide **8** (Y = Cl, Br, I) can be accomplished in the presence of copper iodide, a ligand such as N,N'-dimethylethylenediamine and a base, e.g. potassium carbonate affording the targeted oxadiazoles **Id.**

Scheme 6

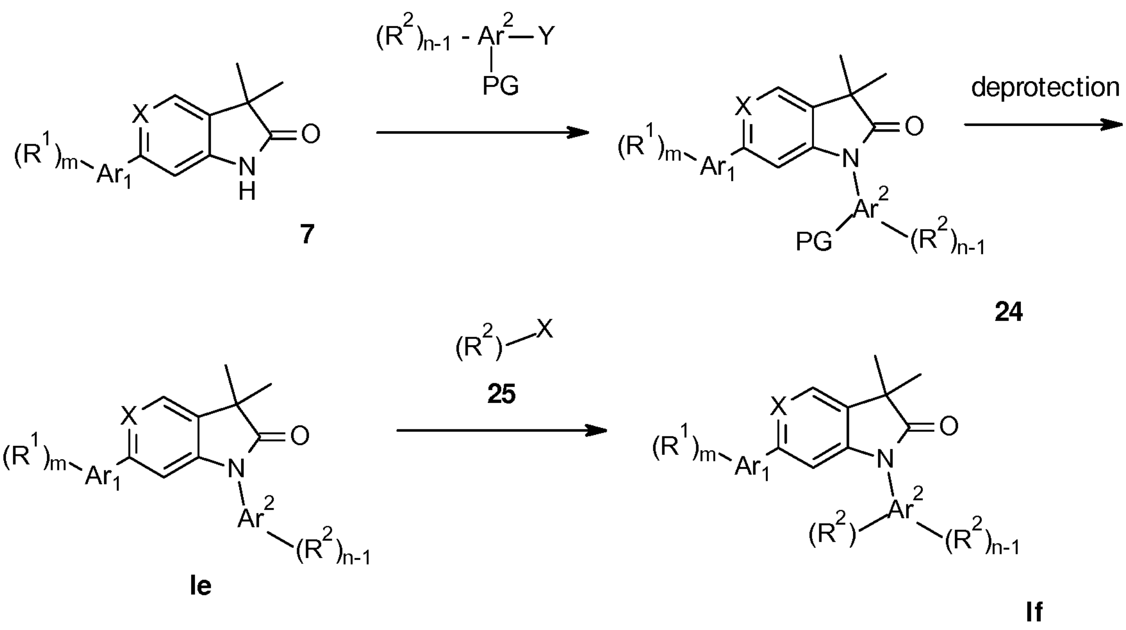


7 24 25 Ie If

Presence of exchangable/acidic proton on the some heteroaromatic rings (e.g. 5-member rings) can be detrimental for the coupling reaction to **7.** In such cases, protecting groups (PP, eg. Tetrahydropyranyl, tert-butylcarbamate, trimethylsilylethoxymethyl etc.) can be use, facilitating the production of compounds **24.**

Introduction and cleavage of these protecting groups are following methods known by those skilled in the Art (cf. Protective groups in organic synthesis, third edition, Wiley interscience, from T. H. Greene and P. G. M. Wuts).

Subsequent cleavage of the protecting group afford compound **Ie,** which can optionally be further substituted by alkylation with alkyl halogenide (X=Cl, Br, I) affording compound of general formula **If.**

Scheme 7

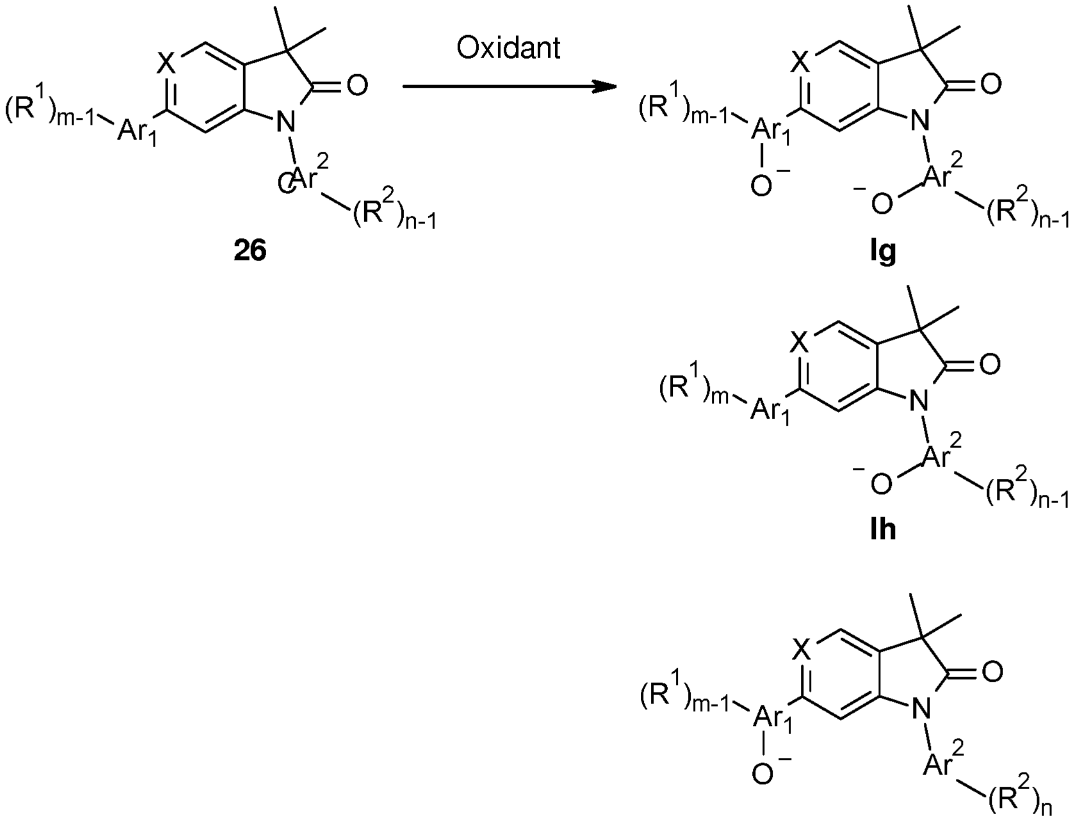


26

Ig and/or

Ih and/or

Ii

Using an oxidant on **26** (e.g. metachloroperbenzoic acid) may afford the N-oxide derivatives of general formula **Ig, Ih** and/or **Ii.** Regioselectivity and number of such N-oxidation depend on the relative electron density of the heteroaromatics and stoechiometry of the reaction condition. Separation of the various products might require the use of HPLC.

Scheme 8

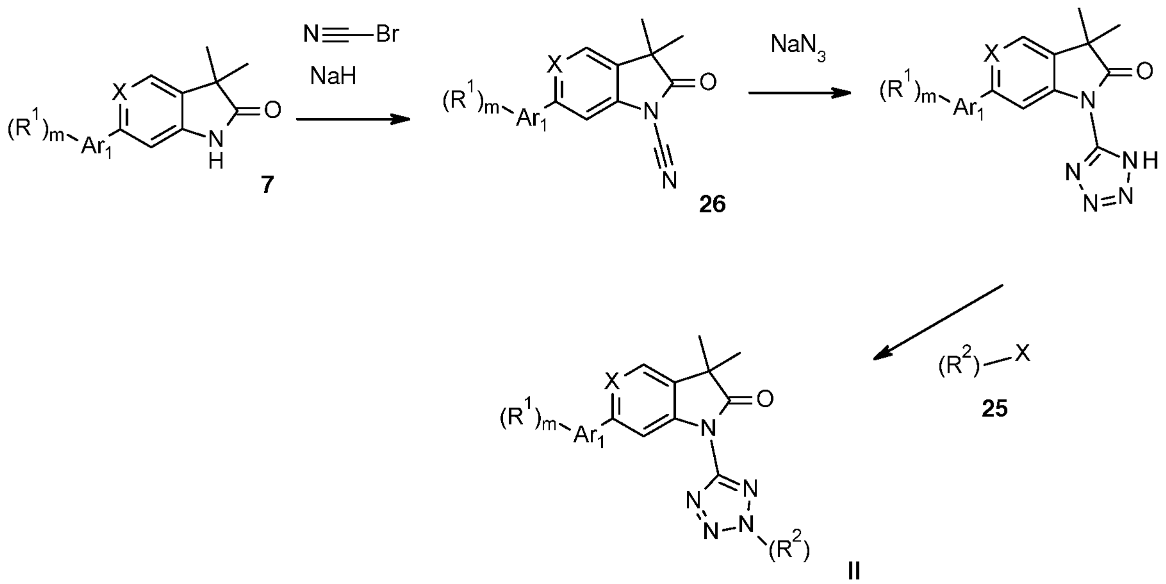


7

26

Ik

25

Il

Addition of cyanobromide on compound of general formula **7** can be achieved with the use of a strong base

e.g. sodium hydride. Sodium azide can react on **26** in presence a copper or zinc catalyst to afford the tetrazole of general formula **Ik.** This process is known as "click-chemistry". The tetrazole can be alkylated by alkyl halide **25** (X=C, Br, I) and a base such as potassium carbonate, affording compounds of general formula **Il.**

Experimental Part

The following examples are provided for illustration of the invention. They should not be considered as limiting the scope of the invention, but merely as being representative thereof.

Abbreviations:

Boc, t-butyloxycarbonyl;
DIPEA, diisopropylethylamine;
DMAP, dimethylaminopyridine;
DMF, dimethylformamide;
DMSO, dimethylsulfoxide;
EDCI, 1-ethyl-3-(3-dimethylaminopropyl)carbodiimid;
EtOAc, ethyl acetate;
HOBt, 1-hydroxybenzotriazole;
MeOH, methanol;
NMP, N-methyl-2-pyrrolidon;
PMB, p-methoxybenzyl;
TFA, trifluoroacetic acid;
THF, tetrahydrofuran.

General: Silica gel chromatography was either performed using cartridges packed with silica gel (ISOLUTE® Columns, TELOSTM Flash Columns) or silica-NH2 gel (TELOSTM Flash NH2 Columns) on ISCO Combi Flash Companion or on glass columns on silica gel 60 (32-60 mesh, 60 Å). MS: Mass spectra (MS) were measured with ion spray positive or negative method on a Perkin-Elmer SCIEX API 300.

**Example 1**

**3,3-Dimethyl-6-(2-methylpyrimidin-5-yl)-1-(pyridin-4-yl)indolin-2-one**

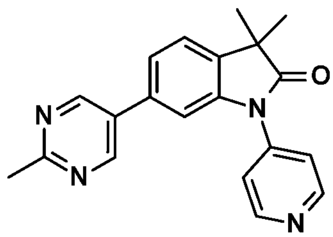

a) 6-Bromo-3,3-dimethyl-indolin-2-one

To a suspension of potassium tert-butylate (12.8 g) in dry THF (80 ml) was added portion wise at 0 °C 6-bromoindolin-2-one (5.0 g,) followed by copper (I) bromide-dimethylsulfide complex (470 mg). MeI (6.82 g) was added drop wise within 45 min keeping the internal temperature below 8 °C, the mixture was warmed to 22 °C and stirring was continued for 16 hours. The mixture was quenched at 0 °C with saturated aqueous ammonium chloride solution and diluted with TBME and water. The organic layer was dried, evaporated and the residue purified by flash chromatography (silica gel, EtOAc/ n-heptane, 1:1) to give the title compound (5.17 g) as a brown solid (5.17 g, 91%). MS (m/z): 240.4/ 242.4 [$(M+H)^+$].

b) 3,3-Dimethyl-6-(4,4,5,5-tetramethyl-1,3,2-dioxaborolan-2-yl)indolin-2-one

A suspension of 6-bromo-3,3-dimethyl-indolin-2-one (1.00 g), bis(pinacolato)diboron (1.60 g), potassium acetate (0.83 g) in DMSO (14 ml) was flushed with argon, then treated with [1,1'-bis(diphenylphosphino)ferrocene] dichloropalladium(II) (152 mg) and stirring was continued at 110 °C for 16 h. The mixture was partitioned between aqueous hydrochloric acid (0.1 M) and EtOAc, the organic layer was dried, evaporated and the residue purified by flash chromatography (silica gel, gradient, 0% to 80% EtOAc in n-heptane) to give the title compound (0.92 g, 77%) as a light yellow solid. MS (m/z): 288.2 [$(M+H)^+$].

c) 3,3-Dimethyl-6-(2-methylpyrimidin-5-yl)indolin-2-one

A suspension of 3,3-dimethyl-6-(4,4,5,5-tetramethyl-1,3,2-dioxaborolan-2-yl)indolin-2-one (200 mg) and 5-bromo-2-methylpyrimidine (181 mg) in 1,4-dioxane (2 ml) and aqueous sodium carbonate (2 M) was flushed with argon, then [1,1'-bis(diphenylphosphino)ferrocene] dichloropalladium(II) (26 mg) was added and stirring was continued at 115 °C for 3 h. The mixture was evaporated and the residue purified by flash chromatography (silica gel, gradient, 0% to 10% MeOH in dichloromethane) to give the title compound (148 mg, 84%) as a brown solid. MS (m/z): 254.2 [$(M+H)^+$].

d) 3,3-Dimethyl-6-(2-methylpirimidin-5-yl)-1-(pyridin-4-yl)indolin-2-one (Example 1)

A suspension of 3,3-dimethyl-6-(2-methylpyrimidin-5-yl)indolin-2-one (100 mg) and 4-iodopyridine (97 mg) in acetonitrile (1.5 ml) was flushed with argon, then potassium carbonate (120 mg), copper(I)iodide (8 mg) and N,N'-dimethylethylendiamine (7 mg) were added and stirring was continued in a microwave oven at 120 °C for 1.5 h. The mixture was partitioned between water and EtOAc, the organic layer was dried, evaporated and the residue purified by flash chromatography (silica gel, 30% to 100% EtOAc in n-heptane). The compound containing fractions were evaporated and the residue crystallized from n-heptane/EtOAc to give the title compound (64 mg, 49%) as a light brown solid. MS (m/z): 331.2 [$(M+H)^+$].

**Example 2**

**3,3-Dimethyl-1-(1-methyl-1H-imidazol-4-yl)-6-(2-methylpyrimidin-5-yl)indolin-2-one**

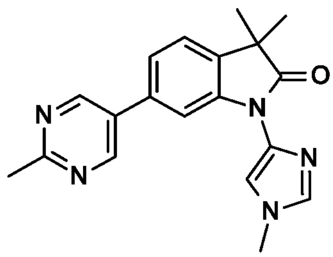

Example 2 was prepared in analogy to example 1d using 4-bromo-1-methyl-1H-imidazole to give the title compound (59%) as an off-white solid. MS (m/z): 334.3 [$(M+H)^+$].

**Example 3**

**3,3-Dimethyl-6-(2-methylpyrimidin-5-yl)-1-(pyridin-3-yl)indolin-2-one**

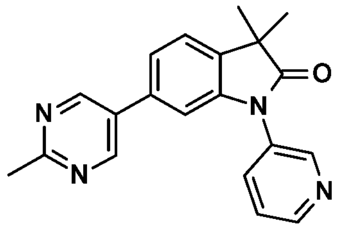

Example 3 was prepared in analogy to example 1d using 3-iodopyridine to give the title compound (50%) as white needles. MS (m/z): 331.3 [(M+H)$^+$].

**Example 4**

**3,3-Dimethyl-1-(1-methyl-1H-pyrazol-3-yl)-6-(2-methylpyrimidin-5-yl)indolin-2-one**

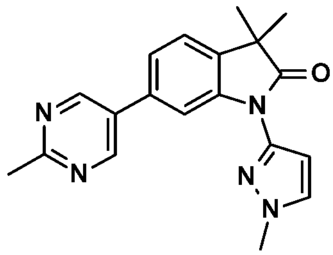

Example 4 was prepared in analogy to example 1d using 3-iodo-1-methyl-1H-pyrazole to give the title compound (75%) as an off-white solid. MS (m/z): 334.2 [(M+H)$^+$].

**Example 5**

**1-(1,5-Dimethyl-1H-pyrazol-3-yl)-3,3-dimethyl-6-(2-methylpyrimidin-5-yl)indolin-2-one**

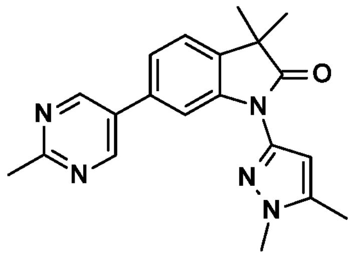

Example 5 was prepared in analogy to example 1d using 3-bromo-1,5-dimethyl-1H-pyrazole to give the title compound (67%) as an off-white solid. MS (m/z): 348.3 [(M+H)$^+$],

**Example 6**

**3,3-Dimethyl-1-(2-methylpyridin-4-yl)-6-(2-methylpyrimidin-5-yl)indolin-2-one**

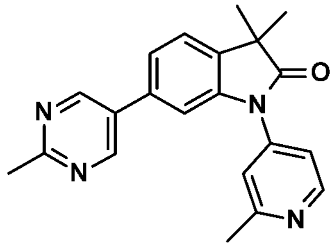

Example 6 was prepared in analogy to example 1d using 4-bromo-2-methylpyridine to give the title compound (79%) as a white foam. MS (m/z): 345.3 [$(M+H)^+$].

**Example 7**

**3,3-Dimethyl-1-(6-methylpyrimidin-4-yl)-6-(2-methylpyrimidin-5-yl)indolin-2-one**

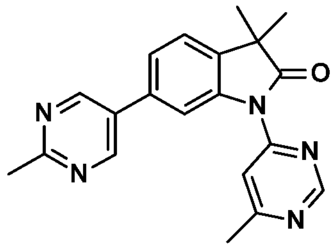

Example 7 was prepared in analogy to example 1d using 4-bromo-6-methylpyrimidine to give the title compound (27%) as a yellow solid. MS (m/z): 346.2 [$(M+H)^+$].

**Example 8**

**3,3-Dimethyl-6-(2-methylpyrimidin-5-yl)-1-(pyrimidin-5-yl)indolin-2-one**

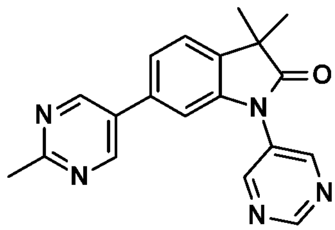

Example 8 was prepared in analogy to example 1d using 5-bromopyrimidine to give the title compound (55%) as a light yellow solid. MS (m/z): 332.2 [$(M+H)^+$].

**Example 9**

**3,3-Dimethyl-1,6-bis(2-methylpyrimidin-5-yl)indolin-2-one**

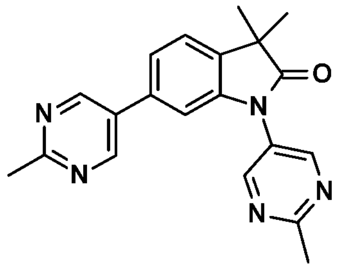

Example 9 was prepared in analogy to example 1d using 5-bromo-2-methylpyrimidine to give the title compound (28%) as a white solid. MS (m/z): 346.2 [$(M+H)^+$].

**Example 10**

**3,3-Dimethyl-1-(1-methyl-1H-imidazol-4-yl)-6-(6-methylpyridin-3-yl)indolin-2-one**

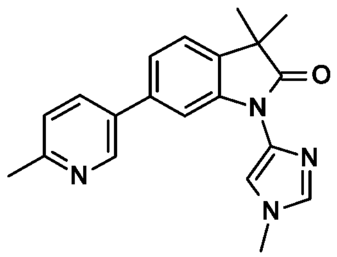

a) 3,3-Dimethyl-6-(6-methylpyridin-3-yl)indolin-2-one

A mixture of 6-bromo-3,3-dimethylindolin-2-one (250 mg) from example 1a and 6-methylpyridine-3-boronic acid (214 mg) in 1,4-dioxane (4 ml) and aqueous sodium carbonate (1.3 ml) was flushed with argon, then [1,1'-bis(diphenylphosphino)ferrocene] dichloropalladium(II) (38 mg) was added and stirring was continued at 115 °C for 6 h. The mixture was evaporated and the residue purified by flash chromatography (silica gel, gradient, 10% to 100% EtOAc in n-heptane) to give the title compound (205 mg, 78%) as an off-white solid. MS (m/z): 253.3 [$(M+H)^+$].

b) 3,3-Dimethyl-1-(1-methyl-1H-imidazol-4-yl)-6-(6-methylpyridin-3-yl)indolin-2-one (Example 10)

The title compound was prepared from 3,3-dimethyl-6-(6-methylpyridin-3-yl)indolin-2-one and 4-bromo-1-methyl-1H-imidazole in analogy to example Id and obtained (55%) as a light yellow solid. MS (m/z): 333.3 [$(M+H)^+$].

**Example 11**

**3,3-Dimethyl-1-(1-methyl-1H-imidazol-4-yl)-6-(2-methylpyridin-4-yl)indolin-2-one**

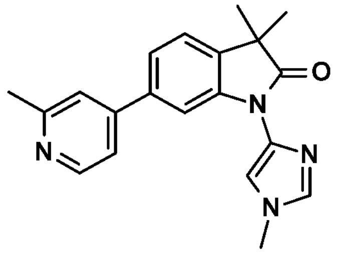

a) 3,3-Dimethyl-6-(2-methylpyridin-4-yl)indolin-2-one

The title compound was prepared in analogy to example 10a using 2-methylpyrimidine-5-boronic acid and obtained (49%) as a light brown solid. MS (m/z): 253.3 [(M+H)+].

b) 3,3-Dimethyl-1-(1-methyl-1H-imidazol-4-yl)-6-(2-methylpyridin-4-yl)indolin-2-one (Example 11)

The title compound was prepared from 3,3-dimethyl-6-(2-methylpyridin-4-yl)indolin-2-one and 4-bromo-1-methyl-1H-imidazole in analogy to example 1d and obtained (43%) as a brown oil. MS (m/z): 333.2 [(M+H)+].

**Example 12**

**3,3-Dimethyl-1-(6-methylpyridin-3-yl)-6-(2-methylpyrimidin-5-yl)indolin-2-one**

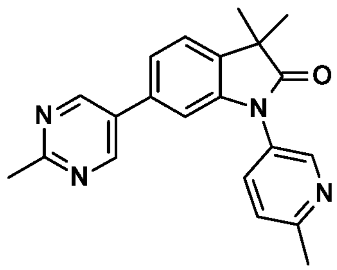

Example 12 was prepared in analogy to example 1d using 5-bromo-2-methylpyridine to give the title compound (65%) as a white solid. MS (m/z): 345.2 [(M+H)+].

**Example 13**

**3,3-Dimethyl-1-(1-methyl-1H-pyrazol-3-yl)-6-(6-methylpyridin-3-yl)indolin-2-one**

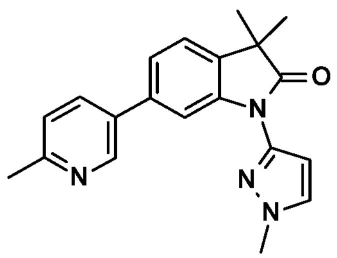

Example 13 was prepared in analogy to example 10b using 3-iodo-1-methyl-1H-pyrazole to give the title compound (76%) as a light yellow oil. MS (m/z): 333.2 [(M+H)+].

**Example 14**

**6-(4-Fluoropyridin-3-yl)-3,3-dimethyl-1-(1-methyl-1H-imidazol-4-yl)indolin-2-one**

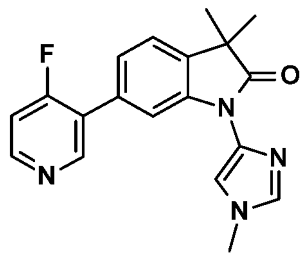

a) 6-(4-Fluoropyridin-3-yl)-3,3-dimethylindolin-2-one

The title compound was prepared in analogy to example 10a using 4-fluoropyridine-3-boronic acid pinacol ester and obtained (19%) as a light brown solid. MS (m/z): 257.3 [$(M+H)^+$].

b) 6-(4-Fluoropyridin-3-yl)-3,3-dimethyl-1-(1-methyl-1H-imidazol-4-yl)indolin-2-one (Example 14)

The title compound was prepared from 6-(4-fluoropyridin-3-yl)-3,3-dimethylindolin-2-one and 4-bromo-1-methyl-1H-imidazole in analogy to example Id and obtained (39%) as a brown solid. MS (m/z): 337.2 [$(M+H)^+$].

**Example 15**

**3,3-Dimethyl-1-(1-methyl-1H-imidazol-4-yl)-6-(2-methylpyrimidin-5-yl)-1H-pyrrolo[3,2-c]pyridin-2(3H)-one**

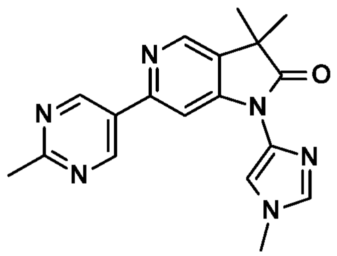

a) 3,3-Dimethyl-6-(2-methylpyrimidin-5-yl)-1H-pyrrolo[3,2-c]pyridin-2(3H)-one

A suspension of 6-chloro-3,3-dimethyl-1H-pyrrolo[3,2-c]pyridin-2(3H)-one (200 mg, prepared according to Woolford et al., WO 2012143726) and 2-methyl-5-(4,4,5,5-tetramethyl-1,3,2-dioxaborolan-2-yl)pyrimidine (336 mg) in 1,4-dioxane (4 ml) and aqueous sodium carbonate solution (2 M, 1 ml) was flushed with argon, then [1,1'-bis(diphenylphosphino)ferrocene] dichloropalladium(II) (37 mg) was added and stirring was continued at 110 °C for 5 h. The mixture was evaporated and the residue purified by flash chromatography (silica gel, gradient, 0% to 10% MeOH in dichloromethane) to give the title compound (221 mg, 85%) as a brown solid. MS (m/z): 255.1 [$(M+H)^+$].

b) 3,3-Dimethyl-1-(1-methyl-1H-imidazol-4-yl)-6-(2-methylpyrimidin-5-yl)-1H-pyrrolo[3,2-c]pyridin-2(3H)-one (Example 15)

The title compound was prepared from 3,3-dimethyl-6-(2-methylpyrimidin-5-yl)-1H-pyrrolo[3,2-c]pyridin-2(3H)-one and 4-bromo-1-methyl-1H-imidazole in analogy to example Id and obtained (29%) as a white solid. MS (m/z): 335.2 [$(M+H)^+$].

**Example 16**

**3,3-Dimethyl-1-(1-methyl-1H-pyrazol-3-yl)-6-(2-methylpyrimidin-5-yl)-1H-pyrrolo[3,2-c]pyridin-2(3H)-one**

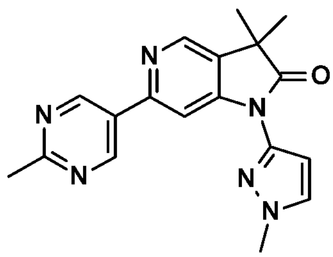

Example 16 was prepared from 3,3-dimethyl-6-(2-methylpyrimidin-5-yl)-1H-pyrrolo[3,2-c]pyridin-2(3H)-one from example 15a and 3-iodo-1-methyl-1H-pyrazole in analogy to example Id to give the title compound (91%) as a light brown solid. MS (m/z): 335.2 [(M+H)$^+$].

**Example 17**

**3,3-Dimethyl-1-(2-methylpyridin-4-yl)-6-(2-methylpyrimidin-5-yl)-1H-pyrrolo[3,2-c]pyridin-2(3H)-one**

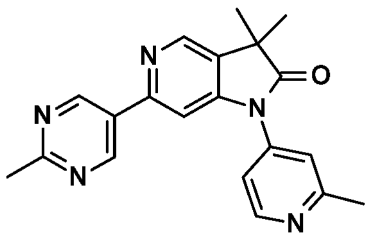

Example 17 was prepared from 3,3-dimethyl-6-(2-methylpyrimidin-5-yl)-1H-pyrrolo[3,2-c]pyridin-2(3H)-one from example 15a and 4-bromo-2-methylpyridine in analogy to example Id to give the title compound (74%) as a brown solid. MS (m/z): 346.2 [(M+H)$^+$].

**Example 18**

**3,3-Dimethyl-6-(4-methylimidazol-1-yl)-1-(6-methyl-3-pyridyl)indolin-2-one**

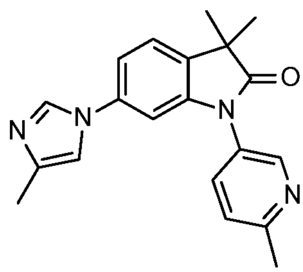

a) 6-Bromo-1-(4-methoxybenzyl)-3,3-dimethyl-1,3-dihydroindol-2-one

To a solution of 6-bromo-3,3-dimethyl-1,3-dihydroindol-2-one from example 1a (4.00 g) in DMF (40 ml) were added cesium carbonate (3.50 g) and 4-methoxy benzyl chloride (5.00 g) and stirring was continued at 80 °C for 16 h. The mixture was partitioned between water and EtOAc, the organic layer was dried, evaporated and the residue purified by flash chromatography (silica gel, EtOAc/ n-heptane, 1:4) to give the title compound (3.50 g, 58%) as an off-white solid. MS (m/z): 362.0 [(M+H)$^+$].

b) 1-(4-Methoxybenzyl)-3,3-dimethyl-6-(4-methyl-imidazol-1-yl)-1,3-dihydroindol-2-one

A mixture of 6-bromo-1-(4-methoxybenzyl)-3,3-dimethyl-1,3-dihydroindol-2-one (0.50 g), potassium carbonate (0.21 g) and 4-methyl imidazole (0.57 g) in NMP (2.5 ml) was flushed with argon, then CuBr (20 mg) and 2-acetylcyclohexanone (39 mg) were added and and stirring was continued at 135 °C for 16 h. The mixture was partitioned between water and EtOAc, the organic layer was dried, evaporated and the residue purified by flash chromatography (silica gel, EtOAc/ n-heptane, 3:2) to give the title compound (0.17 g, 35%) as a light yellow solid. MS (m/z): 361.8 [$(M+H)^+$].

c) 3,3-Dimethyl-6-(4-methyl-imidazol-1-yl)-1,3-dihydroindol-2-one

A solution of 1-(4-methoxybenzyl)-3,3-dimethyl-6-(4-methyl-imidazol-1-yl)-1,3-dihydroindol-2-one (170 mg) in TFA (10 ml) was heated at 110 °C for 72 h. The mixture was evaporated, the residue partitioned between aqueous saturated sodium bicarbonate and EtOAc, the organic layer was dried, evaporated and the residue purified by flash chromatography (silica gel, EtOAc) to give the title compound (70 mg, 61%) as a light yellow solid. MS (m/z): 241.8 [$(M+H)^+$].

d) 3,3-Dimethyl-6-(4-methylimidazol-1-yl)-1-(6-methyl-3-pyridyl)indolin-2-one (Example 18)

To a suspension of 3,3-dimethyl-6-(4-methyl-imidazol-1-yl)-1,3-dihydroindol-2-one (70 mg), 2-methylpyridine-5-boronic acid (80 mg), DMAP (106 mg) and copper acetate (56 mg) in dry toluene (2 ml) was added sodium bis(trimethylsilyl)amide (1 M in THF, 0.06 ml) at 25 °C while bubbling dry air through the mixture and stirring was continued at 95 °C for 16h. The mixture was partitioned between aqueous hydrochloric acid (2 M) and EtOAc, the organic layer was dried, evaporated and the residue purified by flash chromatography (silica gel, EtOAc/MeOH, 95:5) to give the title compound (36 mg, 37%) as a brown solid. MS (m/z): 333.1 [$(M+H)^+$].

**Example 19**

**3,3-Dimethyl-6-(6-methylpyridin-3-yl)-1-(2-methylpyridin-4-yl)indolin-2-one**

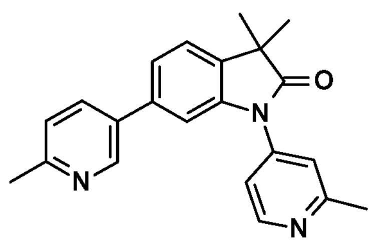

Example 19 was prepared in analogy to example 10b using 4-bromo-2-methylpyridine to give the title compound (51%) as a light yellow oil. MS (m/z): 344.2 [$(M+H)^+$].

**Example 20**

**3,3-Dimethyl-1,6-bis(2-methylpyridin-4-yl)indolin-2-one**

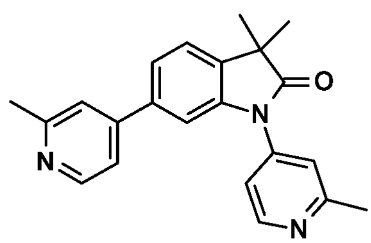

Example 20 was prepared in analogy to example 11b using 4-bromo-2-methylpyridine to give the title compound (65%) as a white foam. MS (m/z): 344.3 [(M+H)$^+$].

**Example 21**

**6-(4-Cyclopropylimidazol-1-yl)-3,3-dimethyl-1-(2-methyl-4-pyridyl)indolin-2-one**

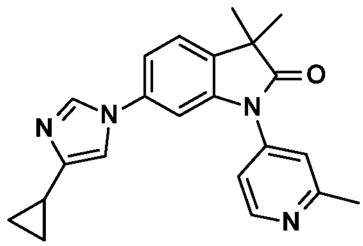

a) 6-(4-Cyclopropyl-imidazol-1-yl)-3,3-dimethyl-1,3-dihydro-indol-2-one

The title compound was prepared in analogy to example 18a-c using 4-cyclopropyl imidazole (prepared according to Chen, Y., WO2010096395) in step 18b and obtained (58%) as a yellow solid. MS (m/z): 267.9 [(M+H)$^+$],

b) 6-(4-Cyclopropylimidazol-1-yl)-3,3-dimethyl-1-(2-methyl-4-pyridyl)indolin-2-one (Example 21)

The title compound was prepared form 6-(4-cyclopropyl-imidazol-1-yl)-3,3-dimethyl-1,3-dihydro-indol-2-one and 2-methyl-pyridine-4-boronic in analogy to example 18d to give the title compound (9%) as a yellow solid. MS (m/z): 358.9 [(M+H)$^+$].

**Example 22**

**3,3-Dimethyl-6-(4-methylimidazol-1-yl)-1-(2-methyl-4-pyridyl)indolin-2-one**

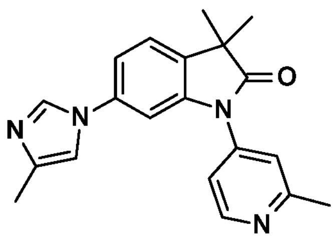

Example 22 was prepared in analogy to example 18d using 2-methylpyridine-4-boronic acid to give the title compound (22%) as an off-white solid. MS (m/z): 332.9 [(M+H)$^+$].

**Example 23**

**6-(4-Fluoropyridin-3-yl)-3,3-dimethyl-1-(1-methyl-1H-pyrazol-3-yl)indolin-2-one**

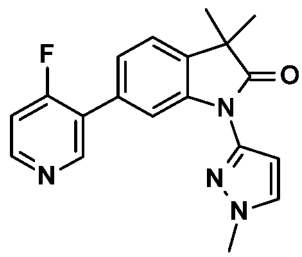

Example 23 was prepared in analogy to example 14b using 3-iodo-1-methyl-1H-pyrazole to give the title compound (74%) as a light yellow oil. MS (m/z): 337.2 [(M+H)$^+$].

**Example 24**

**6-(4-Fluoropyridin-3-yl)-3,3-dimethyl-1-(2-methylpyridin-4-yl)indolin-2-one**

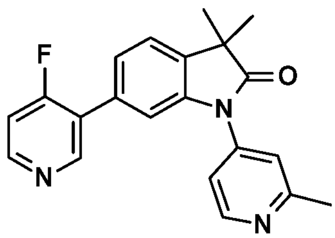

Example 24 was prepared in analogy to example 14b using 4-bromo-2-methylpyridine to give the title compound (37%) as a light yellow foam. MS (m/z): 348.1 [(M+H)$^+$].

**Example 25**

**1-(5-Fluoro-2-methylpyridin-4-yl)-3,3-dimethyl-6-(2-methylpyrimidin-5-yl)indolin-2-one**

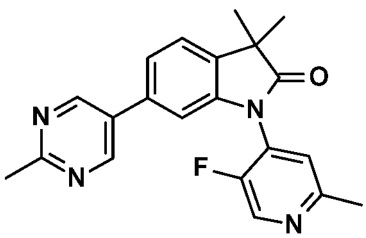

Example 25 was prepared in analogy to example 1d using 4-bromo-5-fluoro-2-methylpyridine to give the title compound (21%) as a colorless oil. MS (m/z): 363.2 [(M+H)$^+$].

**Example 26**

**3,3-Dimethyl-6-(1-methyl-1H-pyrazol-3-yl)-1-(2-methylpyrimidin-5-yl)indolin-2-one**

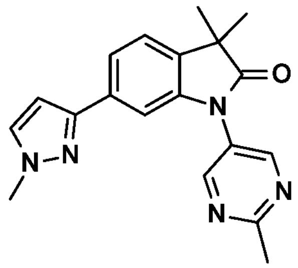

a) 3,3-Dimethyl-6-(1-methyl-1H-pyrazol-3-yl)indolin-2-one

The title compound was prepared in analogy to example 1c using 3,3-dimethyl-6-(4,4,5,5-tetramethyl-1,3,2-dioxaborolan-2-yl)indolin-2-one from example 1b and 3-iodo-1-methyl-1H-pyrazole to give the title compound (26%) as an off-white solid. MS (m/z): 242.1 [(M+H)$^+$].

b) 3,3-Dimethyl-6-(1-methyl-1H-pyrazol-3-yl)-1-(2-methylpyrimidin-5-yl)indolin-2-one (Example 26)

The title compound was prepared from 3,3-dimethyl-6-(1-methyl-1H-pyrazol-3-yl)indolin-2-one in analogy to example Id using 5-bromo-2-methylpyrimidine to give the title compound (59%) as a colorless oil. MS (m/z): 334.2 [(M+H)$^+$].

**Example 27**

**3,3-Dimethyl-6-(2-methylpyrimidin-5-yl)-1-(5-methylthiophen-2-yl)indolin-2-one**

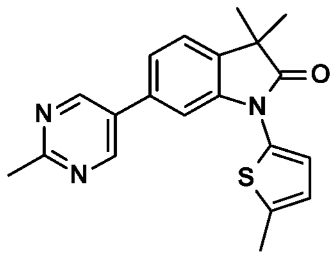

Example 27 was prepared in analogy to example 1d using 2-iodo-5-methylthiophene to give the title compound (38%) as a white solid. MS (m/z): 350.2 [(M+H)$^+$].

**Example 28**

**3,3-Dimethyl-1-(1-methyl-1H-imidazol-5-yl)-6-(2-methylpyrimidin-5-yl)indolin-2-one**

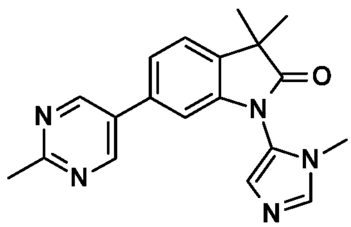

Example 28 was prepared in analogy to example 1d using 5-bromo-1-methyl-1H-imidazole to give the title compound (18%) as a yellow solid. MS (m/z): 334.2 [(M+H)$^+$].

**Example 29**

**3,3-Dimethyl-6-(1-methyl-1H-imidazol-4-yl)-1-(2-methylpyrimidin-5-yl)indolin-2-one**

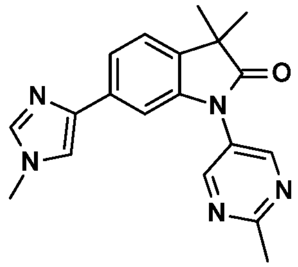

a) 3,3-Dimethyl-6-(1-methyl-1H-pyrazol-3-yl)indolin-2-one

The title compound was prepared in analogy to example 1c using 3,3-dimethyl-6-(4,4,5,5-tetramethyl-1,3,2-dioxaborolan-2-yl)indolin-2-one from example 1b and 4-Iodo-1-methyl-1H-imidazole to give the title compound (16%) as a brown solid. MS (m/z): 242.2 [$(M+H)^+$].

b) 3,3-Dimethyl-6-(1-methyl-1H-imidazol-4-yl)-1-(2-methylpyrimidin-5-yl)indolin-2-one (Example 29)

The title compound was prepared from 3,3-dimethyl-6-(1-methyl-1H-pyrazol-3-yl)indolin-2-one and 5-bromo-2-methylpyrimidine in analogy to example 1d to give the title compound (34%) as an off-white solid. MS (m/z): 334.2 [$(M+H)^+$].

**Example 30**

**5-(3,3-Dimethyl-6-(2-methylpyrimidin-5-yl)-2-oxoindolin-1-yl)picolinonitrile**

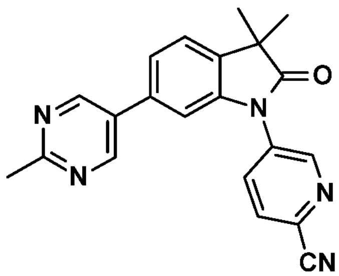

Example 30 was prepared in analogy to example 1d using 5-bromopicolinonitrile to give the title compound (66%) as a white foam. MS (m/z): 356.2 [$(M+H)^+$].

**Example 31**

**1-(6-(Hydroxymethyl)pyridin-3-yl)-3,3-dimethyl-6-(2-methylpyrimidin-5-yl)indolin-2-one**

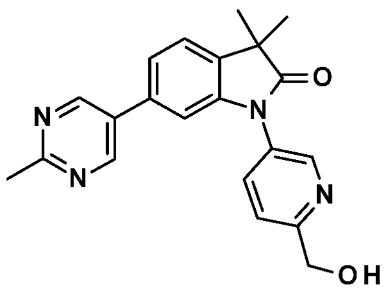

Example 31 was prepared in analogy to example 1d using (5-bromopyridin-2-yl)methanol to give the title compound (55%) as a white solid. MS (m/z): 361.2 [(M+H)$^+$].

**Example 32**

**1-(6-Cyclopropylpyridin-3-yl)-3,3-dimethyl-6-(2-methylpyrimidin-5-yl)indolin-2-one**

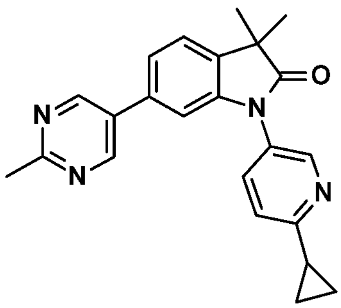

Example 32 was prepared in analogy to example 1d using 5-bromo-2-cyclopropylpyridine to give the title compound (26%) as a white solid. MS (m/z): 371.2 [(M+H)$^+$].

**Example 33**

**6-(4-Isopropylimidazol-1-yl)-3,3-dimethyl-1-(6-methyl-3-pyridyl)indolin-2-one**

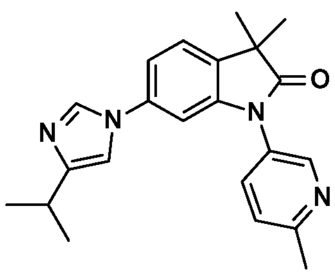

a) <u>6-(4-Isopropyl-imidazol-1-yl)-3,3-dimethyl-1,3-dihydro-indol-2-one</u>

The title compound was prepared in analogy to example 18a-c using 4-isopropyl imidazole (prepared according to Dolby et al., US20050101785) in step 18b and obtained (67%) as an off-white solid. MS (m/z): 269.9 [(M+H)$^+$].

b) <u>6-(4-Isopropylimidazol-1-yl)-3,3-dimethyl-1-(6-methyl-3-pyridyl)indolin-2-one (Example 33)</u>

Example 33 was prepared from 6-(4-isopropyl-imidazol-1-yl)-3,3-dimethyl-1,3-dihydro-indol-2-one and 2-methyl-pyridine-4-boronic acid in analogy to example 18d to give the title compound (25%) as yellow solid. MS (m/z): 360.8 [(M+H)$^+$].

**Example 34**

**3,3-Dimethyl-6-(5-methyl-1,3,4-oxadiazol-2-yl)-1-(6-methyl-3-pyridyl)indolin-2-one**

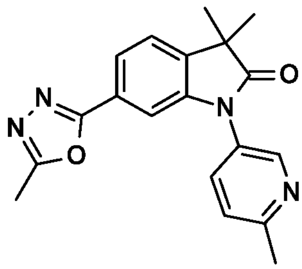

a) 3,3-Dimethyl-2-oxo-2,3-dihydro-1H-indole-6-carboxylic acid methyl ester

A solution of 6-bromo-3,3-dimethyl-1,3-dihydroindol-2-one (5.00 g), DIPEA (26.9) and DMF (5 ml) in methanol (40 ml) was flushed with argon, then [1,1'-bis(diphenylphosphino)ferrocene] dichloropalladium(II) (1.20 g) was added and stirring was continued in an autoclave at a 150 psi CO pressure at 100 °C for 18 h. The mixture was evaporated and the residue partitioned between water and EtOAc, the organic layer was dried, evaporated and the residue purified by flash chromatography (silica gel, gradient, 30% EtOAc in n-heptane) to give the title compound (3.0 g, 66%) as a brown solid. MS (m/z): 220.0 [(M+H)$^+$].

b) 3,3-Dimethyl-2-oxo-2,3-dihydro-1H-indole-6-carboxylic acid

To a solution of 3,3-dimethyl-2-oxo-2,3-dihydro-1H-indole-6-carboxylic acid methyl ester (6.00 g) in THF (60 ml) were added LiOH (11.5 g) and water (10 ml) and stirring was continued at 25 °C for 18 h. The mixture was evaporated, the residue dissolved in ice cold water, the pH was adjusted to 5-6 using aqueous hydrochloric acid (6 N, 40 ml), the suspension was filtered and the residue dried to give the title compound (3.50 g, 62%) as a light yellow solid. MS (m/z): 204.1 [(M-H)$^-$].

c) 3,3-Dimethyl-2-oxo-2,3-dihydro-1H-indole-6-carboxylic acid N'-acetyl-hydrazide

To a solution of 3,3-dimethyl-2-oxo-2,3-dihydro-1H-indole-6-carboxylic acid (2.00 g) and acetic acid hydrazide (0.81 g) in dry DMF (5 ml) were added HOBt (1.58 g), EDCI (2.20 g) and DIPEA (4.3 ml) and stirring was continued 25° C for 18 h. The mixture was evaporated to give the crude title compound (2.20 g, 82%), which was used in the next step without further purification. MS (m/z): 262.1 [(M+H)$^+$].

d) 3,3-Dimethyl-6-(5-methyl-[1,3,4]oxadiazol-2-yl)-1,3-dihydroindol-2-one

To a solution of crude 3,3-dimethyl-2-oxo-2,3-dihydro-1H-indole-6-carboxylic acid N'-acetylhydrazide (43) (1.40 g) in DMF (5 ml) and actonitrile (10 ml) were added tosyl chloride (1.50 g) and triethylamine (2.2 ml) and stirring was continued 25 °C for 18 h. The mixture was evaporated and the residue partitioned between water and EtOAc, the organic layer was dried, evaporated and the residue purified by flash chromatography (silica gel, gradient, 30% EtOAc in n-heptane) to give the title compound (0.50 g, 38%). MS (m/z): 244.2 [(M+H)$^+$].

e) 3,3-Dimethyl-6-(5-methyl-1,3,4-oxadiazol-2-yl)-1-(6-methyl-3-pyridyl)indolin-2-one (Example 34)

The title compound was prepared from 3,3-dimethyl-6-(5-methyl-[1,3,4]oxadiazol-2-yl)-1,3-dihydroindol-2-one and 5-bromo-2-methyl-pyridine in analogy to example 1d and obtained as an off-white solid (29%). MS (m/z): 335.1 [(M+H)$^+$].

**Example 35**

**3,3-Dimethyl-6-(5-methyl-1,3,4-oxadiazol-2-yl)-1-(2-methyl-4-pyridyl)indolin-2-one**

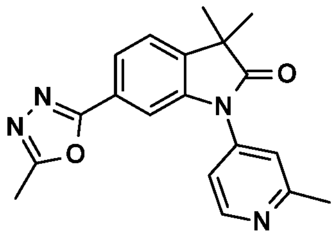

Example 35 was prepared from 3,3-dimethyl-6-(5-methyl-[1,3,4]oxadiazol-2-yl)-1,3-dihydroindol-2-one from example 34d and 4-bromo-2-methyl-pyridine in analogy to example Id to give the title compound (27%) as an off-white solid. MS (m/z): 335.2 [$(M+H)^+$].

**Example 36**

**6-(4-Isopropylimidazol-1-yl)-3,3-dimethyl-1-(2-methyl-4-pyridyl)indolin-2-one**

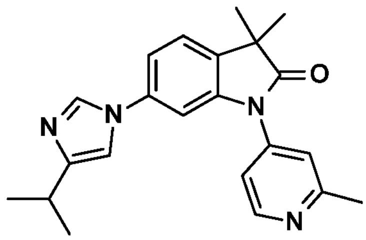

A mixture of 6-(4-isopropyl-imidazole-1-yl)-3,3-dimethyl-1,3-dihydro-indol-2-one (150 mg) from example 33a, 4-bromo-2-methyl-pyridine (116 mg) and potassium carbonate (169 mg) in acetonitrile (10 ml) was flushed with argon, then CuI (10 mg) and N,N'-dimethylethylenediamine (16 mg) were added and stirring was continued at 110 °C for 5 h. The mixture was partitioned between water and EtOAc, the organic layer was dried, evaporated and the residue purified by flash chromatography (silica gel, EtOAc) to give the title compound (24%) as a yellow solid. MS (m/z): 361.0 [$(M+H)^+$].

**Example 37**

**6-(4-Isopropylimidazol-1-yl)-3,3-dimethyl-1-(1-methylpyrazol-3-yl)indolin-2-one**

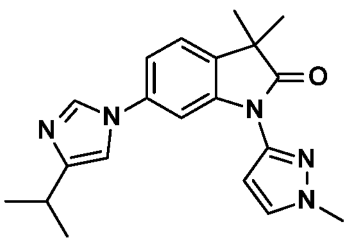

Example 37 was prepared from 6-(4-isopropyl-imidazole-1-yl)-3,3-dimethyl-1,3-dihydro-indol-2-one from example 33a and 3-bromo-1-methyl-1H-pyrazole in analogy to example 36 to give the title compound (31%) as an off-white solid. MS (m/z): 349.8 [$(M+H)^+$].

**Example 38**

**6-(4-Isopropylimidazol-1-yl)-3,3-dimethyl-1-(2-methylpyrimidin-5-yl)indolin-2-one**

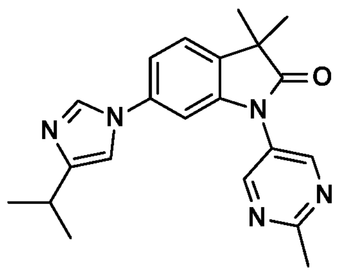

Example 38 was prepared from 6-(4-isopropyl-imidazole-1-yl)-3,3-dimethyl-1,3-dihydro-indol-2-one from example 33a 5-bromo-2-methyl-pyrimidine in analogy to example 36 to give the title compound (31%) (29%) as a light yellow solid. MS (m/z): 361.9 [$(M+H)^+$].

**Example 39**

**6-(4-Cyclopropylimidazol-1-yl)-3,3-dimethyl-1-(1-methylimidazol-4-yl)indolin-2-one**

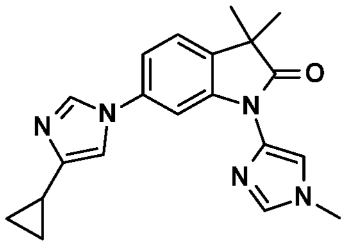

Example 39 was prepared from 6-(4-cyclopropyl-imidazol-1-yl)-3,3-dimethyl-1,3-dihydro-indol-2-one from example 21a and 4-bromo-1-methyl-1H-imidazole in analogy to example 36 to give the title compound (31%) as a light yellow solid. MS (m/z): 347.9 [$(M+H)^+$].

**Example 40**

**6-(4-Cyclopropylimidazol-1-yl)-3,3-dimethyl-1-(1-methylpyrazol-3-yl)indolin-2-one**

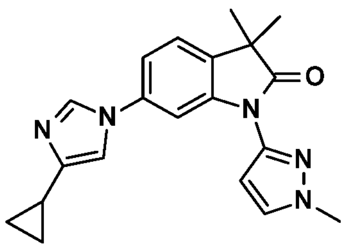

Example 40 was prepared from 6-(4-cyclopropyl-imidazol-1-yl)-3,3-dimethyl-1,3-dihydro-indol-2-one from example 21a and 3-bromo-1-methyl-pyrazole in analogy to example 36 to give the title compound (33%) as a grey solid. MS (m/z): 347.8 [$(M+H)^+$].

**Example 41**

**6-(4-Cyclopropylimidazol-1-yl)-3,3-dimethyl-1-(2-methylpyrimidin-5-yl)indolin-2-one**

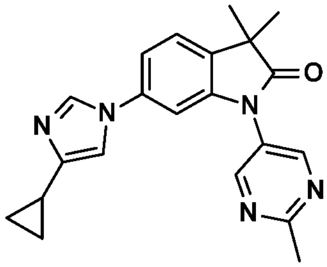

Example 41 was prepared from 6-(4-cyclopropyl-imidazol-1-yl)-3,3-dimethyl-1,3-dihydro-indol-2-one from example 21a and 5-bromo-2-methyl-pyrimidine in analogy to example 36 to give the title compound (28%) as a light yellow semi solid. MS (m/z): 359.9 [$(M+H)^+$].

**Example 42**

**3,3-Dimethyl-1-(1-methylimidazol-4-yl)-6-(2-methyloxazol-5-yl)indolin-2-one**

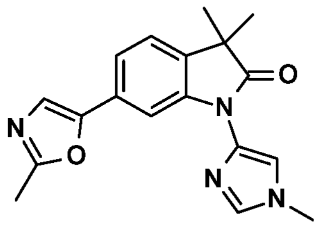

a) <u>1-(4-Methoxy-benzyl)-3,3-dimethyl-6-(2-methyl-oxazol-5-yl)-1,3-dihydro-indol-2-one</u>

A suspension of 6-bromo-1-(4-methoxybenzyl)-3,3-dimethyl-1,3-dihydro-indol-2-one (1.00 g), 2 methyloxazole (0.50 g) and potassium carbonate (1.15 g) in 1,4-dioxane (10 ml) was flushed with argon, then palladium diacetate (31 mg) and 2-(dicyclohexylphosphino)biphenyl (10 mg) were added and stirring was continued at 110 °C for 16 h. The mixture was partitioned between water and EtOAc, the organic layer was dried, evaporated and the residue purified by flash chromatography (silica gel, gradient, 0-50% EtOAc in n-heptane) to give the title compound (0.45 g, 45%) as a yellow liquid. MS (m/z): 363.0 [$(M+H)^+$].

b) <u>3,3-Dimethyl-6-(2-methyl-oxazol-5-yl)-1,3-dihydro-indol-2-one</u>

A solution of 1-(4-methoxy-benzyl)-3,3-dimethyl-6-(2-methyl-oxazol-5-yl)-1,3-dihydro-indol-2-one (450 mg) in TFA (20 ml) was heated at 110 °C for 72 h. The mixture was partitioned between saturated aqueous sodium bicarbonate solution and EtOAc, the organic layer was dried, evaporated and the residue purified by flash chromatography (silica gel, EtOAc/n-heptane, 4:1) to give the title compound (200 mg, 66%) as an off-white solid. MS (m/z): 243.3 [$(M+H)^+$].

c) <u>3,3-Dimethyl-1-(1-methylimidazol-4-yl)-6-(2-methyloxazol-5-yl)indolin-2-one (Example 42)</u>

Example 42 was prepared from 3,3-dimethyl-6-(2-methyl-oxazol-5-yl)-1,3-dihydro-indol-2-one and 4-bromo-1-methyl-1H-imidazole in analogy to example Id to give the title compound (30%) as a yellow solid. MS (m/z): 323.0 [$(M+H)^+$].

**Example 43**

**3,3-Dimethyl-6-(5-methyl-1,3,4-oxadiazol-2-yl)-1-(2-methylpyrimidin-5-yl)indolin-2-one**

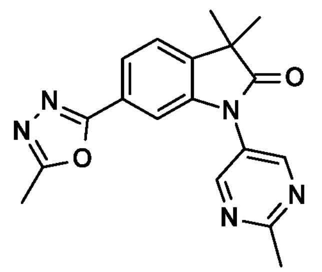

Example 43 was prepared from 3,3-dimethyl-6-(5-methyl-[1,3,4]oxadiazol-2-yl)-1,3-dihydroindol-2-one from example 34d and 5-bromo-2-methyl-pyrimidine in analogy to example Id to give the title compound (40%) as an off-white solid. MS (m/z): 336.6 [(M+H)$^+$].

**Example 44**

**3,3-Dimethyl-6-(2-methylpyrimidin-5-yl)-1-(pyridin-2-yl)indolin-2-one**

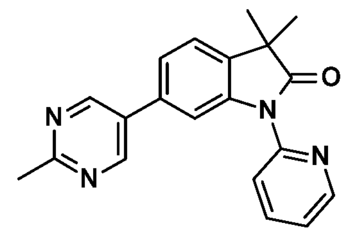

Example 44 was prepared in analogy to example Id using 2-iodopyridine to give the title compound (53%) as a white solid. MS (m/z): 331.2 [(M+H)$^+$].

**Example 45**

**1-(2-Fluoropyridin-4-yl)-3,3-dimethyl-6-(2-methylpyrimidin-5-yl)indolin-2-one**

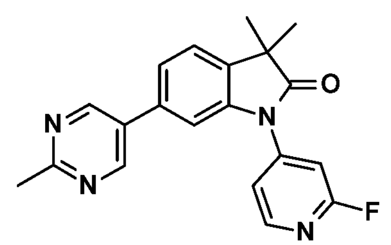

Example 45 was prepared in analogy to example 1d using 2-fluoro-4-iodopyridine to give the title compound (70%) as a white foam. MS (m/z): 349.1 [(M+H)$^+$].

**Example 46**

**1-(3-Fluoropyridin-4-yl)-3,3-dimethyl-6-(2-methylpyrimidin-5-yl)indolin-2-one**

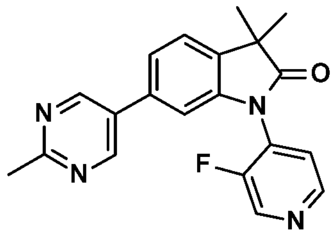

Example 46 was prepared in analogy to example 1d using 3-fluoro-4-iodopyridine to give the title compound (23%) as a white foam. MS (m/z): 349.2 [$(M+H)^+$].

**Example 47**

**1-(2-Fluoro-5-methylpyridin-4-yl)-3,3-dimethyl-6-(2-methylpyrimidin-5-yl)indolin-2-one**

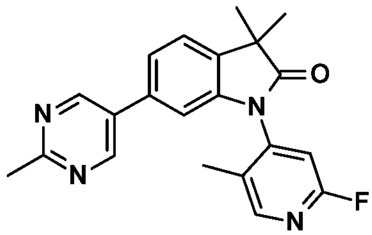

Example 47 was prepared in analogy to example 1d using 2-fluoro-4-iodo-5-methylpyridine to give the title compound (6%) as a white foam. MS (m/z): 363.2 [$(M+H)^+$].

**Example 48**

**3,3-Dimethyl-1-(6-methylpyridazin-3-yl)-6-(2-methylpyrimidin-5-yl)indolin-2-one**

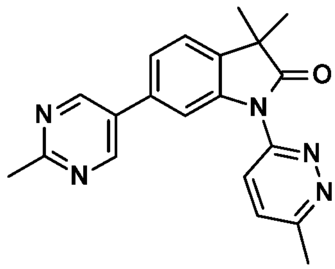

Example 48 was prepared in analogy to example 1d using 3-iodo-6-methylpyridazine to give the title compound (67%) as a light yellow solid. MS (m/z): 346.2 [$(M+H)^+$].

**Example 49**

**3,3-Dimethyl-6-(2-methyloxazol-5-yl)-1-(1-methylpyrazol-3-yl)indolin-2-one**

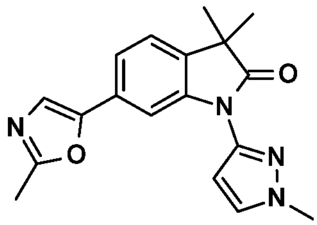

Example 49 was prepared from 3,3-dimethyl-6-(2-methyl-oxazol-5-yl)-1,3-dihydro-indol-2-one and 3-bromo-1-methyl-1H-pyrazole in analogy to example Id to give the title compound (22%) as an off-white solid. MS (m/z): 323.1 [(M+H)$^+$].

**Example 50**

**1-(Benzo[b]thiophen-4-yl)-3,3-dimethyl-6-(2-methylpyrimidin-5-yl)indolin-2-one**

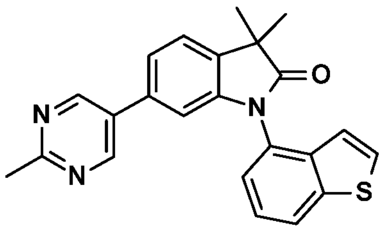

Example 50 was prepared in analogy to example 1d using 4-bromobenzo[b]thiophene to give the title compound (5%) as a white solid. MS (m/z): 386.2 [(M+H)$^+$].

**Example 51**

**3,3-Dimethyl-1-(5-methyl-1,3,4-oxadiazol-2-yl)-6-(2-methylpyrimidin-5-yl)indolin-2-one**

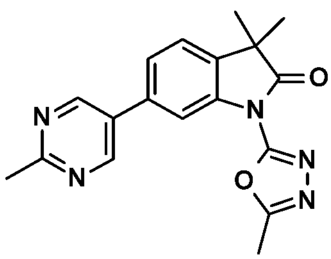

Example 51 was prepared in analogy to example 1d using 2-bromo-5-methyl-1,3,4-oxadiazole to give the title compound (31%) as a white solid. MS (m/z): 336.2 [(M+H)$^+$].

**Example 52**

**1-(3-Chloropyridin-4-yl)-3,3-dimethyl-6-(2-methylpyrimidin-5-yl)indolin-2-one**

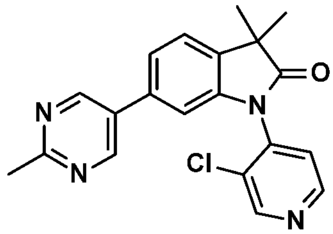

Example 52 was prepared in analogy to example 1d using 4-bromo-3-chloropyridine to give the title compound (21%) as a light yellow solid. MS (m/z): 365.1/367.1 [$(M+H)^+$].

**Example 53**

**3,3-Dimethyl-1-(5-methylpyrimidin-2-yl)-6-(2-methylpyrimidin-5-yl)indolin-2-one**

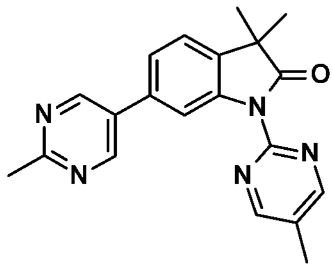

Example 53 was prepared in analogy to example 1d using 2-bromo-5-methylpyrimidine to give the title compound (60%) as an off-white solid. MS (m/z): 346.2 [$(M+H)^+$].

**Example 54**

**3,3-Dimethyl-1-(1-methylimidazol-4-yl)-6-(5-methyl-1,3,4-oxadiazol-2-yl)indolin-2-one**

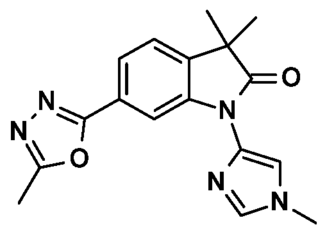

Example 54 was prepared from 3,3-dimethyl-6-(5-methyl-[1,3,4]oxadiazol-2-yl)-1,3-dihydroindol-2-one from example 34d and 4-bromo-1-methyl-1H-imidazole in analogy to example Id to give the title compound (25%) as an off-white solid. MS (m/z): 324.0 [$(M+H)^+$].

**Example 55**

**3,3-Dimethyl-6-(2-methylpyrimidin-5-yl)-1-(1H-pyrazol-3-yl)indolin-2-one**

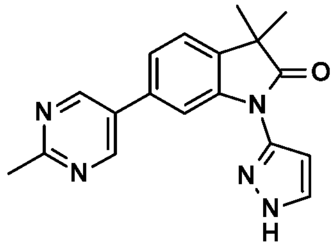

Example 55 was prepared in analogy to example 1d using tert-butyl 3-iodo-1H-pyrazole-1-carboxylate to give the title compound (41%) as a white solid. Under the reaction condition the Boc-group was cleaved. MS (m/z): 320.1 [(M+H)$^+$].

**Example 56**

**3,3-Dimethyl-1-(5-methylpyridin-2-yl)-6-(2-methylpyrimidin-5-yl)indolin-2-one**

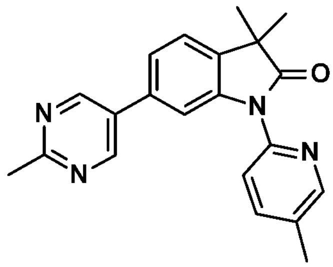

Example 56 was prepared in analogy to example 1d using 2-bromo-5-methylpyridine to give the title compound (74%) as a white solid. MS (m/z): 345.2 [(M+H)$^+$].

**Example 57**

**3,3-Dimethyl-1-(5-methylpyrazin-2-yl)-6-(2-methylpyrimidin-5-yl)indolin-2-one**

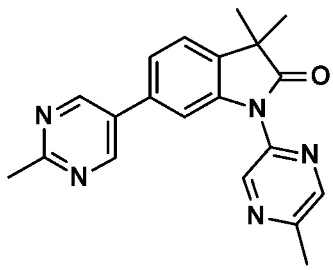

Example 57 was prepared in analogy to example 1d using 2-bromo-5-methylpyrazine to give the title compound (35%) as an off-white solid. MS (m/z): 346.2 [(M+H)$^+$].

**Example 58**

**3,3-Dimethyl-6-(4-methylimidazol-1-yl)-1-(2-methylpyrimidin-5-yl)indolin-2-one**

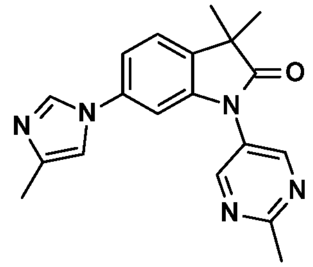

Example 58 was prepared from 3,3-dimethyl-6-(4-methyl-imidazol-1-yl)-1,3-dihydroindol-2-one from example 18c and 5-bromo-2-methyl-pyrimidine in analogy to example 36 to give the title compound (31%) as an off-white solid. MS (m/z): 333.8 [(M+H)$^+$].

**Example 59**

**3,3-Dimethyl-1-(5-methylpyridin-3-yl)-6-(2-methylpyrimidin-5-yl)indolin-2-one**

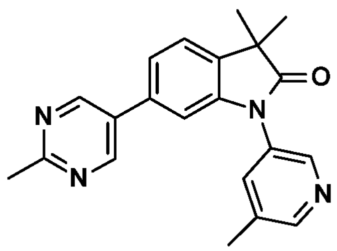

Example 59 was prepared in analogy to example 1d using 3-bromo-5-methyl-pyridine to give the title compound (49%) as a white solid. MS (m/z): 345.2 [(M+H)$^+$].

**Example 60**

**3,3-Dimethyl-1-(4-methylpyridin-2-yl)-6-(2-methylpyrimidin-5-yl)indolin-2-one**

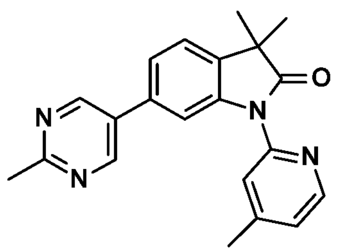

Example 60 was prepared in analogy to example 1d using 2-bromo-4-methyl-pyridine to give the title compound (72%) as a white solid. MS (m/z): 345.2 [(M+H)$^+$].

**Example 61**

**3,3-Dimethyl-1-(6-methylpyridin-2-yl)-6-(2-methylpyrimidin-5-yl)indolin-2-one**

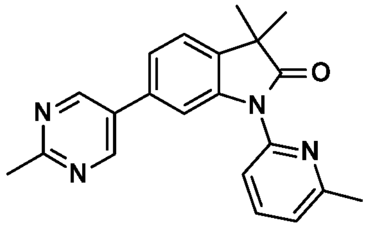

Example 61 was prepared in analogy to example 1d using 2-bromo-6-methyl-pyridine to give the title compound (67%) as a white solid. MS (m/z): 345.2 [(M+H)$^+$].

**Example 62**

**3,3-Dimethyl-1-(2-methylpyrimidin-4-yl)-6-(2-methylpyrimidin-5-yl)indolin-2-one**

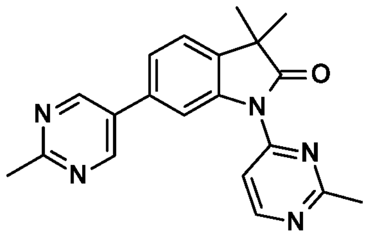

Example 62 was prepared in analogy to example 1d using 4-chloro-2-methyl-pyrimidine to give the title compound (6%) as a white solid. MS (m/z): 346.2 [(M+H)$^+$].

**Example 63**

**3,3-Dimethyl-1-(6-methylpyrazin-2-yl)-6-(2-methylpyrimidin-5-yl)indolin-2-one**

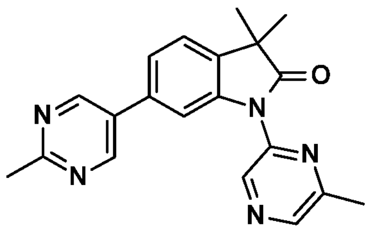

Example 63 was prepared in analogy to example 1d using 2-bromo-6-methylpyrazine to give the title compound (60%) as an off-white solid. MS (m/z): 346.2 [(M+H)$^+$].

**Example 64**

**3,3-Dimethyl-1-(4-methylpyrimidin-2-yl)-6-(2-methylpyrimidin-5-yl)indolin-2-one**

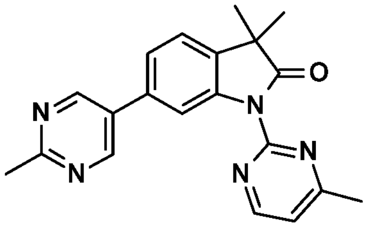

Example 64 was prepared in analogy to example 1d using 2-bromo-4-methylpyrimidine to give the title compound (50%) as a white solid. MS (m/z): 346.2 [(M+H)$^+$].

**Example 65**

**1-(1,2-Dimethyl-1H-imidazol-4-yl)-3,3-dimethyl-6-(2-methylpyrimidin-5-yl)indolin-2-one**

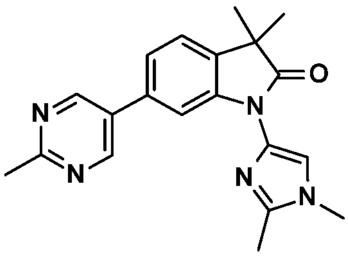

Example 65 was prepared in analogy to example 1d using 4-bromo-1,2-dimethyl-1H-imidazole to give the title compound (8%) as a white solid. MS (m/z): 348.2 [(M+H)$^+$].

**Example 66**

**1-(2,6-Dimethylpyridin-4-yl)-3,3-dimethyl-6-(2-methylpyrimidin-5-yl)indolin-2-one**

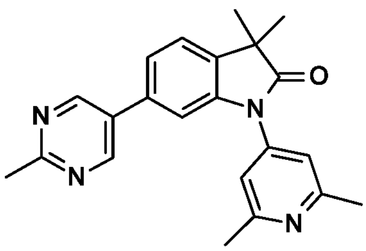

Example 66 was prepared in analogy to example 1d using 4-bromo-2,6-dimethylpyridine to give the title compound (52%) as an off-white solid. MS (m/z): 359.2 [(M+H)$^+$].

**Example 67**

**1-(4,6-Dimethylpyrimidin-2-yl)-3,3-dimethyl-6-(2-methylpyrimidin-5-yl)indolin-2-one**

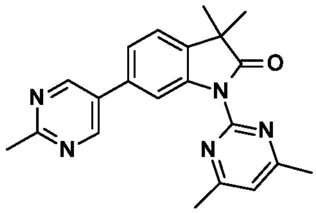

Example 67 was prepared in analogy to example 1d using 2-bromo-4,6-dimethylpyrimidine to give the title compound (50%) as a white solid. MS (m/z): 360.2 [(M+H)$^+$].

**Example 68**

**1-(2,6-Dimethylpyrimidin-4-yl)-3,3-dimethyl-6-(2-methylpyrimidin-5-yl)indolin-2-one**

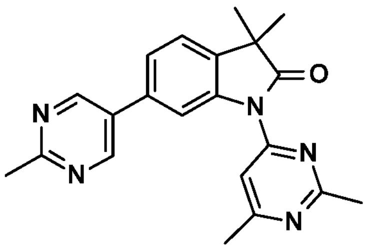

Example 68 was prepared in analogy to example 1d using 4-bromo-2,6-dimethylpyrimidine to give the title compound (46%) as a white solid. MS (m/z): 360.2 [(M+H)$^+$].

**Example 69**

**1-(4,5-Dimethylpyridin-2-yl)-3,3-dimethyl-6-(2-methylpyrimidin-5-yl)indolin-2-one**

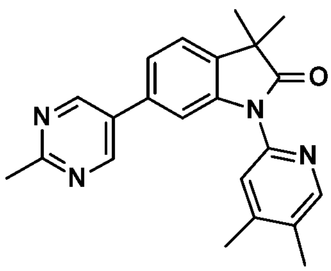

Example 69 was prepared in analogy to example 1d using 2-bromo-4,5-dimethylpyridine to give the title compound (50%) as a white solid. MS (m/z): 359.2 [(M+H)$^+$].

**Example 70**

**1-(5,6-dimethylpyridin-2-yl)-3,3-dimethyl-6-(2-methylpyrimidin-5-yl)indolin-2-one**

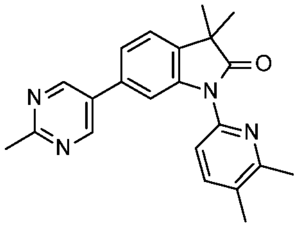

a) 6-Bromo-3,3-dimethyl-indolin-2-one (CAS [158326-84-2])

To a suspension of potassium tert-butylate (12.8 g) in dry tetrahydrofuran (80 ml) was added portion wise at 0 °C 6-bromoindolin-2-one (5.0 g, CAS [99365-40-9]) followed by copper (I) bromide-dimethylsulfide complex (470 mg). methyl iodide (6.82 g) was added drop wise within 45 min keeping the internal temperature below 8 °C, the mixture was warmed to 22 °C and stirring was continued for 16 h. hours. The mixture was quenched at 0 °C with saturated aqueous ammonium chloride solution and diluted with tert-butylmethylether and water. The organic layer was dried, evaporated and the residue purified by flash chromatography (silica gel, ethyl acetate/ n-heptane, 1:1) to give the title compound (5.17 g) as a brown solid (5.17 g, 91%). MS (m/z): 240.4/ 242.4 [$(M+H)^+$],

b) 3,3-Dimethyl-6-(4,4,5,5-tetramethyl-1,3,2-dioxaborolan-2-yl)indolin-2-one

A suspension of 6-bromo-3,3-dimethyl-indolin-2-one (1.00 g), bis(pinacolato)diboron (1.60 g), potassium acetate (0.83 g) in dimethylsulfoxide (14 ml) was flushed with argon, then treated with [1,1'-bis(diphenylphosphino)ferrocene] dichloropalladium(II) (152 mg) and stirring was continued at 110 °C for 16 h. The mixture was partitioned between aqueous hydrochloric acid (0.1 M) and EtOAc, the organic layer was dried, evaporated and the residue purified by flash chromatography (silica gel, gradient, 0% to 80% ethylacetate in n-heptane) to give the title compound (0.92 g, 77%) as a light yellow solid. MS (m/z): 288.2 [$(M+H)^+$],

c) 3,3-Dimethyl-6-(2-methylpyrimidin-5-yl)indolin-2-one

A suspension of 3,3-dimethyl-6-(4,4,5,5-tetramethyl-1,3,2-dioxaborolan-2-yl)indolin-2-one (200 mg) and 5-bromo-2-methylpyrimidine (181 mg) in 1,4-dioxane (2 ml) and aqueous sodium carbonate (2 M) was flushed with argon, then [1,1'-bis(diphenylphosphino)ferrocene] dichloropalladium(II) (26 mg) was added and stirring was continued at 115 °C for 3 h. The mixture was evaporated and the residue purified by flash chromatography (silica gel, gradient, 0% to 10% methanol in dichloromethane) to give the title compound (148 mg, 84%) as a brown solid. MS (m/z): 254.2 [$(M+H)^+$],

d) 1-(5,6-dimethylpyridin-2-yl)-3,3-dimethyl-6-(2-methylpyrimidin-5-yl)indolin-2-one

A degazed suspension of 3,3-dimethyl-6-(2-methylpyrimidin-5-yl)indolin-2-one (100 mg, 395 μmol, Eq: 1.00) and 6-bromo-2,3-dimethylpyridine (88.1 mg, 474 μmol, Eq: 1.20), potassium carbonate (120 mg, 869 μmol, Eq: 2.20), copper(I) iodide (7.52 mg, 39.5 μmol, Eq: 0.10), N,N'-Dimethylethylenediamine (6.96 mg, 8.5 μl, 79.0 μmol, Eq: 0.20) and acetonitrile (2 ml) was heated to 120°C for 18h.

The mixture was partitioned between water (10 mL) and dichloromethane (10 mL), then the aqueous layer was extracted with dichloromethane, the combined organic layers were dried, evaporated and the residue was purified by chromatography on silica gel to give the desired compound as a white solid (73mg, 52%). MS (m/z) = 359.2 [M + H]+.

**Example 71**

**3,3-dimethyl-6-(2-methyloxazol-5-yl)-1-(2-methylpyrimidin-5-yl)indolin-2-one**

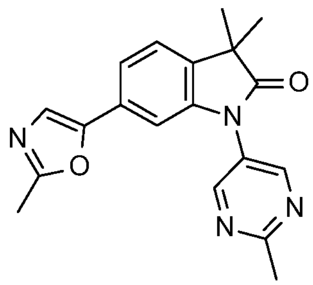

a) 6-bromo-1-(4-methoxybenzyl)-3,3-dimethylindolin-2-one

To a solution of 6-bromo-3,3-dimethylindolin-2-one (3 g, 12.5 mmol, Eq: 1.00, CAS [158326-84-2], example 70 step a) and 1-(chloromethyl)-4-methoxybenzene (1.96 g, 1.69 ml, 12.5 mmol, Eq: 1.00) in dimethylformamide (90 ml) was added at 22 °C cesium carbonate (4.07 g, 12.5 mmol, Eq: 1.00). The reaction mixture was heated at 80°C and stirred for 6 h. Volatiles were removed in vacuo and the residue was partionned between water and ethyl acetate then extracted with ethyl acetate (2 x 150 ml). Combined organic layers were washed with water, dried, evaporated and the residue was purified by chromatography on silica gel to give the desired compound as a light red oil (3.98g, 88%). MS (m/z) = 360.1/362.1 [M + H]+.

b) 1-(4-methoxybenzyl)-3,3-dimethyl-6-(oxazol-5-yl)indolin-2-one

A degazed mixture of 6-bromo-1-(4-methoxybenzyl)-3,3-dimethylindolin-2-one (720 mg, 2 mmol, Eq: 1.00, example 71 step a), palladium (II) acetate (22.5 mg, 100 μmol, Eq: 0.05), 2-di-tert-butylphosphino-3,4,5,6-tetramethyl-2',4',6'-tri-iso-propylbiphenyl (96.1 mg, 200 μmol, Eq: 0.1), pivalic acid (81.7 mg, 92.8 μl, 800 μmol, Eq: 0.4), potassium carbonate (829 mg, 6.00 mmol, Eq: 3), dimethylacetamide (7.5 ml) and oxazole (276 mg, 4.00 mmol, Eq: 2) was heated in an oil bath to 115 °C for 15 h. After cooling to 22°C the reaction mixture was directly purified by chromatography on silica gel to give the desired compound as a light yellow oil solid (500mg, 72%). MS (m/z) = 349.2 [M + H]+.

c) 1-(4-methoxybenzyl)-3,3-dimethyl-6-(2-methyloxazol-5-yl)indolin-2-one

To a solution of 1-(4-methoxybenzyl)-3,3-dimethyl-6-(oxazol-5-yl)indolin-2-one (200 mg, 574 μmol, Eq: 1.00, example 71 step b) in tetrahydrofuran (4 ml) was added at room temperature a solution of borane tetrahydrofuran complex in tetrahydrofuran, (1 M, 689 μl, 689 μmol, Eq: 1.2). After 30 minutes, the solution was cooled to -78 °C and a solution of n-butyllithium in hexane (1.6M, 431 μl, 689 μmol, Eq: 1.2) was added. After 15 minutes at -78 °C, iodomethane (97.8 mg, 43.0 μl, 689 μmol, Eq: 1.2) was added and the mixture was allowed to warm to -20 °C and stirred at this temperature for 4 h. Then a solution of acetic acid, in ethanol (5% v/v, 10.3 g, 9.86 ml, 8.61 mmol, Eq: 15) was added the reaction mixture was stirred at room temperature overnight. The mixture was partitionned between an aqueous saturated solution of sodium hydrogenocarbonate and diethylether then extracted with ether. The combined organic layers were washed brine, dried, evaporated and the residue was purified by chromatography on silica gel to give the desired compound as a light yellow solid (36mg, 17%). MS (m/z) = 363.2 [M + H]+.

d) 3,3-dimethyl-6-(2-methyloxazol-5-yl)indolin-2-one

A solution of 1-(4-methoxybenzyl)-3,3-dimethyl-6-(2-methyloxazol-5-yl)indolin-2-one (35 mg, 96.6 μmol, Eq: 1.00, example 71 step c) was dissolved in trifluoroacetic acid (661 mg, 446 μl, 5.79 mmol, Eq: 60) and the reaction mixture was reacted in microwave at 140 °C for 1 h. Volatiles were removed in vacuo and the green residue was purified by chromatography on silica gel to give the desired compound as a light brown solid (15.9mg, 68%). MS (m/z) = 243.1 [M + H]+.

e) 3,3-dimethyl-6-(2-methyloxazol-5-yl)-1-(2-methylpyrimidin-5-yl)indolin-2-one

The title compound was prepared in analogy to Example 70 step d, with 3,3-dimethyl-6-(2-methyloxazol-5-yl)indolin-2-one (example 71 step d) and 5-bromo-2-methyl-pyrimidine as starting materials. Off-white solid. Yield: 29%. MS (m/z)= 335.4 $(M+H)^+$

**Example 72**

**3,3-dimethyl-6-(4-methyl-1H-imidazol-1-yl)-1-(1-methyl-1H-imidazol-4-yl)indolin-2-one**

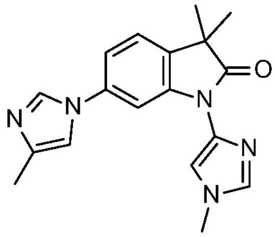

a) 3,3-dimethyl-6-(4-methylimidazol-1-yl)indolin-2-one

In a sealed tube, a degazed mixture of 6-bromo-3,3-dimethylindolin-2-one (150 mg, 625 μmol, Eq: 1.00, CAS [158326-84-2], example 70 step a), 4-methyl-1H-imidazole (256 mg, 3.12 mmol, Eq: 5.0), potassium carbonate (90.7 mg, 656 μmol, Eq: 1.05) and 2-acetylcyclohexanone (21.9 mg, 20.3 μl, 156 μmol, Eq: 0.25) in N-methylpyrrolidone (1.2 ml) and copper(I) chloride (6.18 mg, 62.5 μmol, Eq: 0.1) was stirred at 130 °C for 24 hours. The reaction mixture was partionned between an aqueous saturated solution of sodium hydrogenocarbonate and ethyl acetate then extracted with ethyl acetate. Combined organic layers were dried, evaporated and the residue was purified by chromatography on silica gel then Reversed-Phase-HPLC to give the desired compound as a white solid (39mg, 26%).
MS (m/z) = 242.2 [M + H]+.

b) 3,3-dimethyl-6-(4-methyl-1H-imidazol-1-yl)-1-(1-methyl-1H-imidazol-4-yl)indolin-2-one

The title compound was prepared in analogy to Example 70 step d, with 3,3-dimethyl-6-(4-methylimidazol-1-yl)indolin-2-one (example 72 step a) and 4-bromo-1-methyl-1H-imidazole as starting materials. yellow solid. Yield: 33%. MS (m/z) = 322.2 [M + H]+.

**Example 73**

**3,3-dimethyl-6-(3-methyl-1,2,4-oxadiazol-5-yl)-1-(2-methylpyrimidin-5-yl)indolin-2-one**

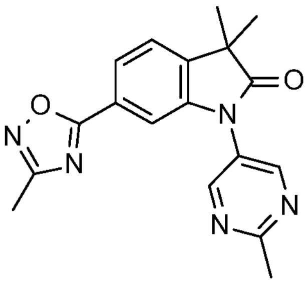

a) methyl 3,3-dimethyl-2-oxo-indoline-6-carboxylate

To a yellow solution of methyl 2-oxoindoline-6-carboxylate (6.24 g, 31.7 mmol, Eq: 1) and methyl iodide (9.08 g, 4 ml, 64 mmol, Eq: 2.02) in dry dimethylformamide (90.5 ml) was added portionwise a suspension of NaH in mineral oil (60% w/w, 2.54 g, 63.4 mmol, Eq: 2) over 1.5h while controlling the exothermicity with a water bath. The reaction mixture was carefully poured on an icecooled mixture of ~11 g sodium hydrogenocarbonate, water (150 mL) and ethyl acetate (150 mL). The resulting mixture was extracted with ethyl acetate and the organic layers were washed with brine. The combined organic layers were dried with sodium sulfate, filtered and concentrated in vacuo.

The residue was triturated with heptane/ ethyl acetate 1:1 and the precipitate was filtered and washed with heptane/ ethyl acetate 1:1. The solid was dried in vacuo to afford the desired product as a light brown solid (5.026 g, 72%).

MS (m/z) = 218.1 [M + H]+.

b) 3,3-dimethyl-2-oxo-2,3-dihydro-1H-indole-6-carboxylic acid

To a solution of methyl 3,3-dimethyl-2-oxo-indoline-6-carboxylate (6.0 g, 27.4 mmol, example 73 step a) in tetrahydrofuran (60 ml) were added lithium hydroxide (11.5 g, 273.9 mmol) and water (10 ml). The mixture was stirred at 25°C for 12 h. After completion of the reaction, the solvent was removed in vacuo. Ice-water (100 ml) was added to the reaction mixture and pH of the reaction mixture was adjusted to 5-6 by addition of hydrochloric acid (6 N, 40 ml). White precipitate was formed which was filtered and washed with water (2 x 25 ml) then dried under vacuum to afford the desired product (3.5 g, 62%) as dark brown solid.
MS (m/z): 204.1 (M-H)-.

c) N-[(E)-N-hydroxy-C-methyl-carbonimidoyl]-3,3-dimethyl-2-oxo-indoline-6-carboxamide

To a solution of 3,3-dimethyl-2-oxo-2,3-dihydro-1H-indole-6-carboxylic acid (1.5 g, 7.317 mmol, example 73 step b) and N-hydroxy-acetamidine (0.54 g, 7.317 mmol) in dry THF(10 ml) and dimethylformamide (1 ml) was added 2,4,6-Tripropyl-1,3,5,2,4,6-trioxatriphosphorinane-2,4,6-trioxide (3.45 g, 10.976 mmol) and triethylamine (3.0 ml, 21.9 mmol). The resulting mixture was stirred at 25°C over a period of 18 h. Solvent was removed under vacuum to afford desired product (1.5g, 78%). This product was used in next step without futher purification.
MS (m/z) = 262.2 [M + H]+.

d) 3,3-dimethyl-6-(3-methyl-[1,2,4]oxadiazol-5-yl)-1,3-dihydro-indol-2-one

A solution of N-[(E)-N-hydroxy-C-methyl-carbonimidoyl]-3,3-dimethyl-2-oxo-indoline-6-carboxamide (1.5g, 5.74mmol, example 73 step c) in dioxane (25 ml) was heated at 100°C for 16h. The reaction mixture was concentrated in vacuo and purified by chromatography on silica gel to give the desired compound as an off-white solid (300mg, 21%).
MS (m/z) = 242.2 [M - H]-.

e) 3,3-dimethyl-6-(3-methyl-1,2,4-oxadiazol-5-yl)-1-(2-methylpyrimidin-5-yl)indolin-2-one

The title compound was prepared in analogy to Example 70 step d, with 33,3-dimethyl-6-(3-methyl-[1,2,4]oxadiazol-5-yl)-1,3-dihydro-indol-2-one (example 73 step d) and 5-bromo-2-methyl-pyrimidine as starting materials. Light yellow solid. Yield: 24%.
MS (m/z) = 336.0 [M + H]+.

**Example 74**

**3,3-dimethyl-1-(1-methyl-1H-pyrazol-5-yl)-6-(2-methylpyrimidin-5-yl)indolin-2-one**

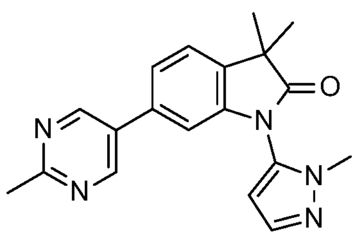

The title compound was prepared in analogy to Example 70 step d, with 3,3-dimethyl-6-(2-methylpyrimidin-5-yl)indolin-2-one (example 70 step c) and 5-iodo-1-methyl-1H-pyrazole as starting materials. Light brown solid. Yield: 9%. MS (m/z)= 334.2 (M+H)+

**Example 75**

**1-(5,6-dimethylpyridin-3-yl)-3,3-dimethyl-6-(2-methylpyrimidin-5-yl)indolin-2-one**

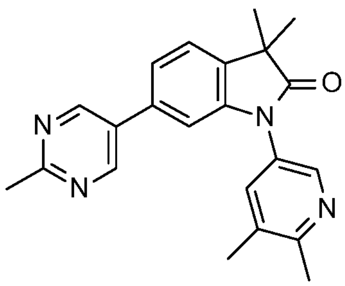

The title compound was prepared in analogy to Example 70 step d, with 3,3-dimethyl-6-(2-methylpyrimidin-5-yl)indolin-2-one (example 70 step c) and 5-bromo-2,3-dimethylpyridine as starting materials. White solid. Yield: 52%. MS (m/z)= 359.2 $(M+H)^+$

**Example 76**

**3,3-dimethyl-6-(2-methylpyrimidin-5-yl)-1-(pyridazin-3-yl)indolin-2-one**

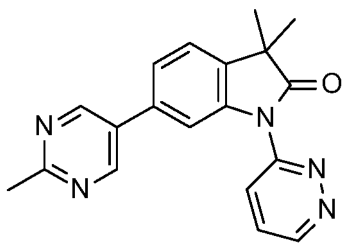

The title compound was prepared in analogy to Example 70 step d, with 3,3-dimethyl-6-(2-methylpyrimidin-5-yl)indolin-2-one (example 70 step c) and 3-bromopyridazine (CAS [88491-61-6] as starting materials. White solid. Yield: 71%.
MS (m/z)= 332.2 $(M+H)^+$

**Example 77**

**3,3-dimethyl-6-(2-methylpyrimidin-5-yl)-1-(pyrazin-2-yl)indolin-2-one**

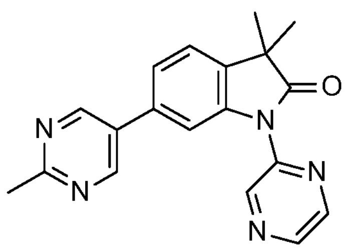

The title compound was prepared in analogy to Example 70 step d, with 3,3-dimethyl-6-(2-methylpyrimidin-5-yl)indolin-2-one (example 70 step c) and 2-iodopyrazine as starting materials. White solid. Yield: 55%. MS (m/z)= 332.3

(M+H)+

**Example 78**

**1-(1,5-dimethyl-1H-1,2,4-triazol-3-yl)-3,3-dimethyl-6-(2-methylpyrimidin-5-yl)indolin-2-one**

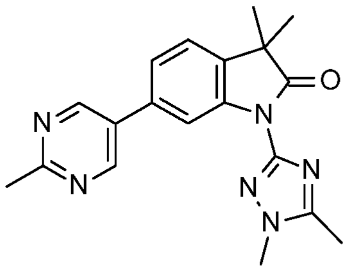

The title compound was prepared in analogy to Example 70 step d, with 3,3-dimethyl-6-(2-methylpyrimidin-5-yl)indolin-2-one (example 70 step c) and 3-bromo-1,5-dimethyl-1H-1,2,4-triazole (CAS [56616-93-4]) as starting materials. Off-white solid. Yield: 65%.
MS (m/z)= 349.2 (M+H)+

**Example 79**

**3,3-dimethyl-6-(2-methylpyrimidin-5-yl)-1-(pyrimidin-2-yl)indolin-2-one**

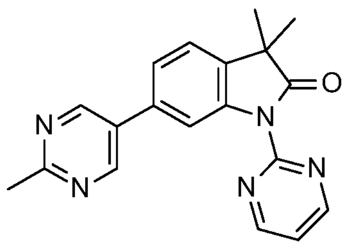

The title compound was prepared in analogy to Example 70 step d, with 3,3-dimethyl-6-(2-methylpyrimidin-5-yl)indolin-2-one (example 70 step c) and 2-bromopyrimidine (CAS [4595-60-2]) as starting materials. White solid. Yield: 32%. MS (m/z)= 332.1 (M+H)+

**Example 80**

**3,3-dimethyl-1-(1-methyl-1H-pyrazol-4-yl)-6-(2-methylpyrimidin-5-yl)indolin-2-one**

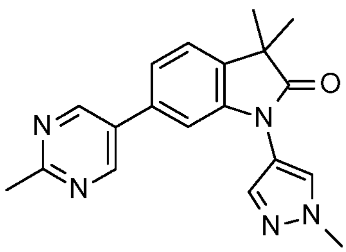

The title compound was prepared in analogy to Example 70 step d, with 3,3-dimethyl-6-(2-methylpyrimidin-5-yl)indolin-2-one (example 70 step c) and 4-bromo-1-methyl-1H-pyrazole (CAS [15803-02-8]) as starting materials. White solid. Yield: 30%. MS (m/z)= 334.2 $(M+H)^+$

**Example 81**

**3,3-dimethyl-1-(2-methyl-1H-imidazol-4-yl)-6-(2-methylpyrimidin-5-yl)indolin-2-one**

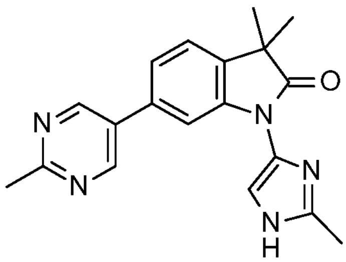

a) 2-[(4-iodo-2-methyl-imidazol-1-yl)methoxylethyl-trimethyl-silane

In a 50 ml three necked flask equipped with magnetic stirrer, septum, thermometer and an argon balloon were dissolved 3.34 g 4,5-diiodo-2-methyl imidazole ([73746-44-8]) in 30 ml of dry tetrahydrofuran. To this solution were added dropwise at -75°C to -65°C, 6.9 ml (1.10 equiv.) butyl lithium 1.6 M/ hexane. Upon initial addition of butyl lithium, the solution turn to a milky white suspension, which upon further addition of butyl lithium became a yellow solution. The reaction was stirred for 10 min at -75°C, and then 1.85 ml (1.75 g, 11.0 mmol, 1.05 equiv.) 2-(Trimethylsilyl)-ethoxymethylchloride was added dropwise at -75°C. The reaction was allowed to warm up to room temperature. The solution was cooled to -75°C and 6.2 ml (1.00 equiv.) butyl lithium 1.6 M/hexane were added dropwise, maintaining the temp. below -65°C. After 30 min at -75°C, 2 ml (5.0 equiv.) Methanol were added dropwise at -75°C. The mixture was stirred for 10 min at -75°C. A saturated auqueous solution of ammonium chloride (3 mL) was added and the mixture allowed to warm up room temparature then partitionned between water and ethyl acetate and the aqueous layer was extracted with ethyl acetate then dried in vacuo. The residue was purified by chromatography on silica gel to give the desired compound (1.27g, 37%).

b) 3,3-dimethyl-1-(2-methyl-1-((2-(trimethylsilyl)ethoxy)methyl)-1H-imidazol-4-yl)-6-(2-methylpyrimidin-5-yl)indolin-2-one:

The title compound was prepared in analogy to Example 70 step d, with 3,3-dimethyl-6-(2-methylpyrimidin-5-yl)indolin-2-one (example 70 step c) and 4-iodo-2-methyl-1-((2-(trimethylsilyl)ethoxy)methyl)-1H-imidazole (example 81 step a)
as starting materials. Yellow solid. Yield: 91%. MS (m/z)= 464.3 $(M+H)^+$

c) 3,3-dimethyl-1-(2-methyl-1H-imidazol-4-yl)-6-(2-methylpyrimidin-5-yl)indolin-2-one

To a solution of 3,3-dimethyl-1-(2-methyl-1-((2-(trimethylsilyl)ethoxy)methyl)-1H-imidazol-4-yl)-6-(2-methylpyrimidin-5-yl)indolin-2-one (164 mg, 354 μmol, Eq: 1.00) in dichloromethane (3 mL) and ethanol (244 mg, 310 μl, 5.31 mmol, Eq: 15) was added trifluoroacetic acid (1.61 g, 1.09 ml, 14.1 mmol, Eq: 40). The reaction mixture was stirred at 22 °C for 20 h. Ethanol (3 ml) was added and the reaction mixture mixture was stirred at 70°C for 20 h, leading to the evaporation of the dichloromethane. Volatiles were removed in vacuo. The residue was partitioned between a saturatzed aqueous solution of sodium carbonate and ethyl acetate. Aqueous layer was extracted twice with ethyl acetate. The combined organic layers were dried and evaporated.

The residue was purified by chromatography on silica gel to give the desired compound as a white solid (53 mg, 45%). MS (m/z) = 334.2 [M + H]+.

**Example 82**

**3,3-dimethyl-1-(1-methyl-1H-1,2,4-triazol-3-yl)-6-(2-methylpyrimidin-5-yl)indolin-2-one**

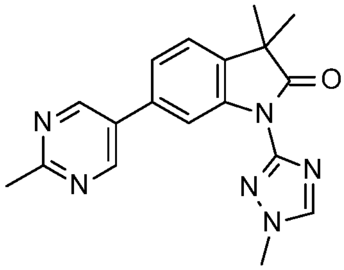

The title compound was prepared in analogy to Example 70 step d, with 3,3-dimethyl-6-(2-methylpyrimidin-5-yl)indolin-2-one (example 70 step c) and 3-bromo-1-methyl-1H-1,2,4-triazole as starting materials. Off-white solid. Yield: 80%. MS (m/z) = 335.2 [M + H]+.

**Example 83**

**3,3-dimethyl-6-(2-methylpyrimidin-5-yl)-1-(1H-pyrazol-4-yl)indolin-2-one**

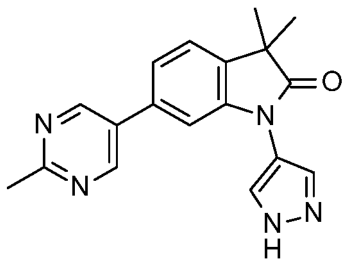

A suspension of 3,3-dimethyl-6-(2-methylpyrimidin-5-yl)indolin-2-one (100 mg, 395 μmol, Eq: 1.00, cf. Example 70 step c) and tert-butyl 4-iodo-1H-pyrazole-1-carboxylate (139 mg, 474 μmol, Eq: 1.20) were mixed in acetonitrile (2 ml). Potassium carbonate (120 mg, 869 μmol, Eq: 2.20) followed by copper(I) iodide (7.52 mg, 39.5 μmol, Eq: 0.10) and N,N'-dimethylethylenediamine (6.96 mg, 8.5 μl, 79.0 μmol, Eq: 0.20) were added and the mixture was heated to 120°C for 20 h in an oil bath and then to 160 °C for 2 h in microwave.

The mixture was partitioned between water and dichloromethane, the aqueous layer was extracted three times with dichloromethane and the combined organic layers were dried and evaporated.

The residue was purified by chromatography on silica gel to give the desired compound as a colorless foam (16mg, 13%). MS (m/z) = 320.2 [M + H]+.

**Example 84**

**3,3-dimethyl-6-(2-methylpyrimidin-5-yl)-1-(4-methylpyrimidin-5-yl)indolin-2-one**

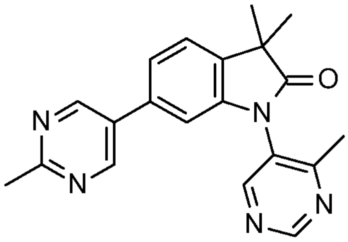

The title compound was prepared in analogy to Example 70 step d, with 3,3-dimethyl-6-(2-methylpyrimidin-5-yl)indolin-2-one (example 70 step c) and 5-iodo-4-methylpyrimidine (CAS [91749-26-7]) as starting materials. Off-white solid. Yield: 11%. MS (m/z) = 346.2 [M + H]+.

**Example 85**

**1-(1H-imidazol-4-yl)-3,3-dimethyl-6-(2-methylpyrimidin-5-yl)indolin-2-one**

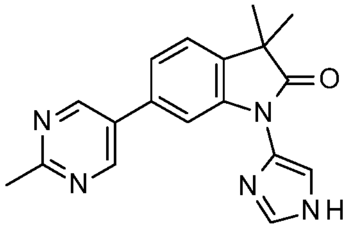

a) tert-butyl 4-bromo-1H-imidazole-1-carboxylate

4-bromo-1H-imidazole (2.59 g, 17.6 mmol, Eq: 1.00) and di-tert-butyl dicarbonate (4.04 g, 4.3 ml, 18.5 mmol, Eq: 1.05) were combined with tetrahydrofuran (19 ml). Dimethylaminopyridine (43.1 mg, 352 μmol, Eq: 0.02) was added and the reaction was stirred at 25°C for 1.5 h. The crude reaction mixture was concentrated in vacuo. The residue was taken up in ethyl acetate and washed with a solution 1M of hydrogen chloride, water, saturated sodium bicarbonate and brine. The organic layer was dried and concentrated in vacuo to afford the desired compound as an off-white solid (4.2 g, 95%).

b) 1-(1H-imidazol-4-yl)-3,3-dimethyl-6-(2-methylpyrimidin-5-yl)indolin-2-one

The title compound was prepared in analogy to Example 70 step d, with 3,3-dimethyl-6-(2-methylpyrimidin-5-yl)indolin-2-one (example 70 step c) and tert-butyl 4-bromo-1H-imidazole-1-carboxylate (example 85 step a) as starting materials. Light yellow solid. Yield: 12%.
MS (m/z) = 320.2 $[M + H]^+$.

**Example 86**

**3,3-dimethyl-1-(3-methyl-1H-pyrazol-5-yl)-6-(2-methylpyrimidin-5-yl)indolin-2-one hydrochloride**

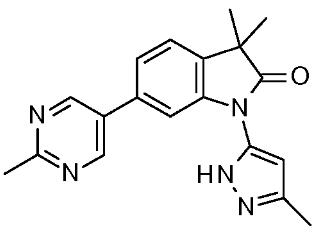

a) 3,3-dimethyl-1-(3-methyl-1-(tetrahydro-2H-pyran-2-yl)-1H-pyrazol-5-yl)-6-(2-methylpyrimidin-5-yl)indolin-2-one

The title compound was prepared in analogy to Example 70 step d, with 3,3-dimethyl-6-(2-methylpyrimidin-5-yl)indolin-2-one (example 70 step c) and 5-iodo-3-methyl-1-(tetrahydro-2H-pyran-2-yl)-1H-pyrazole as starting materials. Light yellow oil. Yield: 33%.
MS (m/z) = 418.3 $[M + H]^+$.

b) 3,3-dimethyl-1-(3-methyl-1H-pyrazol-5-yl)-6-(2-methylpyrimidin-5-yl)indolin-2-one hydrochloride

To a light yellow solution of 3,3-dimethyl-1-(3-methyl-1-(tetrahydro-2H-pyran-2-yl)-1H-pyrazol-5-yl)-6-(2-methylpyrimidin-5-yl)indolin-2-one (55 mg, 132 μmol, Eq: 1.00) in dichloromethane (1 ml) was added hydrogen chloride in dioxane (4 M) (329 μl, 1.32 mmol, Eq: 10) and the mixture was stirred at 22 °C for 4 h. Solvent was concentrated in vacuo and the residue was crystallized in ethyl acetate (5ml) to give the desired compound as an off-white solid (18 mg, 35%). MS (m/z) = 334.2 $[M + H]^+$.

**Example 87**

**3,3-dimethyl-1-(1-methyl-1H-1,2,3-triazol-4-yl)-6-(2-methylpyrimidin-5-yl)indolin-2-one**

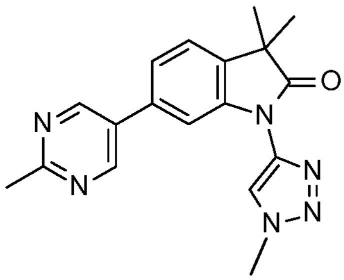

In a microwave tube to a mixture of 3,3-dimethyl-6-(2-methylpyrimidin-5-yl)indolin-2-one (100 mg, 395 μmol, Eq: 1.00, cf. Example 70 step c) and 4-bromo-1-methyl-1H-1,2,3-triazole (76.7 mg, 474 μmol, Eq: 1.2) was added acetonitrile (3 ml). The solvent was degassed by bubbling nitrogen through the suspension for 10 minutes. Then was added at 22 °C N,N'-dimethylethylenediamine (6.96 mg, 8.5 μl, 79.0 μmol, Eq: 0.2) followed by potassium carbonate (136 mg, 987 μmol, Eq: 2.5) and copper (I) iodide (7.52 mg, 39.5 μmol, Eq: 0.1). The tube was inerted, sealed and the mixture was heated in microwave at 170°C for 30 minutes.

The mixture was treated with 2 ml of water and extracted with ethyl acetate (2 x 2 ml). The organic layers were dried, filtered and concentrated in vacuo.

The residue was purified by chromatography on silica gel to give the desired compound as a white solid (8 mg, 6%). MS (m/z) = 335.2 $[M + H]^+$.

**Example 88**

**3,3-dimethyl-1-(4-methyl-1H-imidazol-2-yl)-6-(2-methylpyrimidin-5-yl)indolin-2-one**

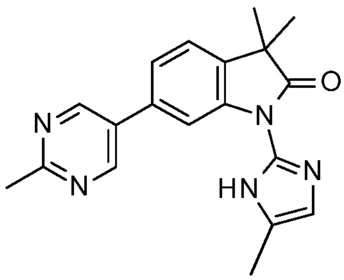

The title compound was prepared in analogy to Example 87, with 3,3-dimethyl-6-(2-methylpyrimidin-5-yl)indolin-2-one (example 70 step c) and tert-butyl 2-bromo-4-methyl-1H-imidazole-1-carboxylate as starting materials. White solid. Yield: 6%. MS (m/z) = 334.2 [M + H]+.

**Example 89**

**3,3-dimethyl-1-(3-methylisoxazol-5-yl)-6-(2-methylpyrimidin-5-yl)indolin-2-one**

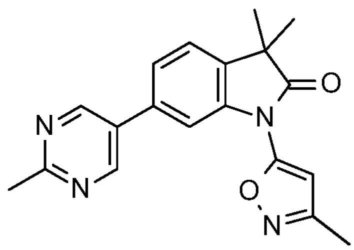

To a mixture of 3,3-dimethyl-6-(2-methylpyrimidin-5-yl)indolin-2-one (100 mg, 395 μmol, Eq: 1.00, cf. Example 70 step c), 5-iodo-3-methylisoxazole (99.0 mg, 474 μmol, Eq: 1.2) and cesium carbonate (322 mg, 987 μmol, Eq: 2.5) was added dioxane (1 ml). The solvent was degassed by bubbling nitrogen through the suspension for 10 minutes. Then was added at 22 °C 2-Dicyclohexylphosphino-2',4',6'-triisopropylbiphenyl (9.41 mg, 19.7 μmol, Eq: 0.05) followed by tris(dibenzylideneacetone)dipalladium(0) (3.62 mg, 3.95 μmol, Eq: 0.01). The tube was inerted, sealed and the mixture was heated to 120 °C for 2 h. The mixture was treated with 2 ml of water and extracted with ethyl acetate (3 x 2 ml). The organic layers were dried, filtered and concentrated in vacuo.

The residue was purified by chromatography on silica gel to give the desired compound as a light yellow solid (5 mg, 3%). MS (m/z) = 335.2 [M + H]+.

**Example 90**

**3,3-dimethyl-1,6-bis(5-methylpyrimidin-2-yl)indolin-2-one**

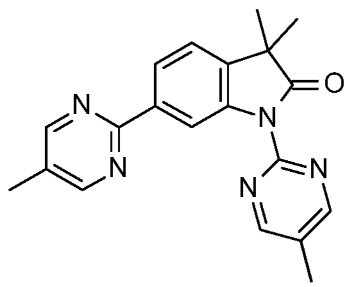

a) 3,3-dimethyl-6-(5-methylpyrimidin-2-yl)indolin-2-one

The title compound was prepared in analogy to Example 70 step c, with 3,3-dimethyl-6-(4,4,5,5-tetramethyl-1,3,2-dioxaborolan-2-yl)indolin-2-one (example 70 step b) and 2-bromo-5-methylpyrimidine as starting materials. Off-white solid. Yield: 43%. MS (m/z) = 254.2 [M + H]+

b) 3,3-dimethyl-1,6-bis(5-methylpyrimidin-2-yl)indolin-2-one

In a sealed tube argon was bubbled through a suspension of 3,3-dimethyl-6-(5-methylpyrimidin-2-yl)indolin-2-one (80 mg, 316 μmol, Eq: 1.00) and 2-bromo-5-methylpyrimidine (65.6 mg, 379 μmol, Eq: 1.20) in acetonitrile (2.5 ml) for 5 min. Potassium carbonate (96.0 mg, 695 μmol, Eq: 2.20) followed by copper(I) iodide (6.02 mg, 31.6 μmol, Eq: 0.10) and N,N'-dimethylethylenediamine (5.57 mg, 6.8 μl, 63.2 μmol, Eq: 0.20) were added and the mixture was heated

to 130°C in microwave for 1 h. The mixture was partitioned between water (10 ml) and dichloromethane (10 ml). The aqueous layer was extracted three times with dichloromethane (3x10 ml) and the combined organic layers were dried and evaporated.

The residue was purified by chromatography on silica gel to give the title compound as an off-white solid (83 mg, 76%). MS (m/z) = 346.2 [M + H]+.

**Example 91**

**3,3-dimethyl-1,6-bis(5-methylpyrazin-2-yl)indolin-2-one**

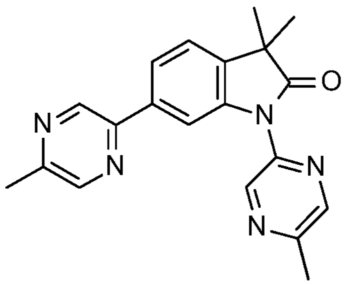

a) 3,3-dimethyl-6-(5-methylpyrazin-1-yl)indolin-2-one

The title compound was prepared in analogy to Example 70 step c, with 3,3-dimethyl-6-(4,4,5,5-tetramethyl-1,3,2-dioxaborolan-2-yl)indolin-2-one (example 70 step b) and 2-bromo-5-methylpyrazine as starting materials. White solid. Yield: 63%. MS (m/z) = 254.2 [M + H]+

b) 3,3-dimethyl-1,6-bis(5-methylpyrazin-2-yl)indolin-2-one

The title compound was prepared in analogy to Example 90 step b, with 3,3-dimethyl-6-(5-methylpyrazin-2-yl)indolin-2-one (example 91 step a) and 2-bromo-5-methylpyrazine as starting materials. Off-white solid. Yield: 68%. MS (m/z) = 346.2 [M + H]+.

**Example 92**

**1-(1H-imidazol-2-yl)-3,3-dimethyl-6-(2-methylpyrimidin-5-yl)indolin-2-one**

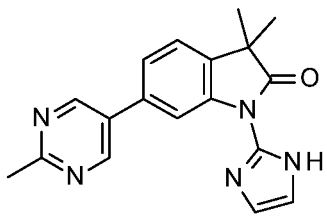

a) 2-(imidazol-1-ylmethoxy)ethyl-trimethyl-silane

Sodium hydride in mineral oil (60% w/w, 5.2g, 130 mmol, Eq: 1.30) was suspended in 240 ml of tetrahydrofuran and cooled to 0°C. A solution of imidazole (6.8g, 100 mmol, Eq: 1.00) dissolved in 100 ml of tetrahydrofuran was slowly dropped to the reaction mixture and the stirring was continuing at room temperature for 45 min. The reaction mixture was then cooled with an ice bath and 2-(trimethylsilyl)ethoxymethyl chloride (16.67g, 17.64 ml, 100 mmol, Eq: 1.00) was added and the suspension was allowed to stir at room temperature overnight. The reaction was quenched by addition of saturated sodium bicarbonate. The solvent was evaporated and the residue was extracted twice with ethyl acetate. The combined organic layer was washed with water, dried and concentrated in vacuo.

The residue was purified by Kugelrohr distillation to give the title compound as a colorless liquid (18.6 g, 93%).

b) 2-iodo-1-((2-(trimethylsilyl)ethoxy)methyl)-1H-imidazole

2-(imidazol-1-ylmethoxy)ethyl-trimethyl-silane (2 g, 10 mmol, Eq: 1.00, Example 92 step a) was dissolved in dry tetrahydrofuran (20 ml) and was cooled to -78°C. n-Buthylithium (1.6 M in hexane, 6.93 ml, 11 mmol, Eq: 1.10) was added dropwise for 20 min. The reaction solution was warmed to 0°C and stirred for 5 min at 0°C. Then it was cooled to -60°C and a solution of iodine (2.81g, 11 mmol, Eq: 1.10) in 10 ml of dry tetrahydrofuran was added for 10 min. The cold bath was removed and the solution was let warm to room temperature. The reaction was quenched with water and the aqueous layer was extracted two times with ethyl acetate. The combined organic layers were washed with sodium carbonate and brine, dried and concentrated in vacuo. The residue was purified by chromatography on silica gel to give the title compound as a light yellow oil (2.34 g, 71%).

c) 3,3-dimethyl-6-(1-methylpyrimidin-5-yl)-1-(1-((-2-trimethylsilyl)ethoxy)methyl)-1H-imidazol-2-yl)indolin-2-one

The title compound was prepared in analogy to Example 70 step d, with 3,3-dimethyl-6-(2-methylpyrimidin-5-yl)indolin-2-one (example 70 step c) and 2-iodo-1-((2-(trimethylsilyl)ethoxy)methyl)-1H-imidazole (example 92 step b) as starting materials. Yellow oil. Yield: 15%. MS (m/z)= 450.3 $(M+H)^+$

d) 1-(1H-imidazol-2-yl)-3,3-dimethyl-6-(2-methylpyrimidin-5-yl)indolin-2-one

To a solution of 3,3-dimethyl-6-(1-methylpyrimidin-5-yl)-1-(1-((1-(trimethylsilyl)ethoxy)methyl)-1H-imidazol-2-yl)indolin-2-one (35 mg, 77.8 μmol, Eq: 1.00, Example 92 step c) in tetrahydrofuran (1 ml) was added tetrabutylammonium floride (1M in THF) (311 μl, 311 μmol, Eq: 4.00) and the mixture was stirred at room temperature for 20h. The mixture was partitioned between water (10 ml) and dichloromethane (10 ml), the aqueous layer was extracted two times with dichloromethane (2x10 ml), and the combined organic layers were dried and concentrated in vacuo.

The residue was purified by preparative HPLC to give the desired compound as a white solid (3 mg, 14%). MS (m/z) = 320.2 $[M + H]^+$.

**Example 93**

**3,3-dimethyl-6-(2-methylpyrimidin-5-yl)-1-(pyrimidin-4-yl)indolin-2-one**

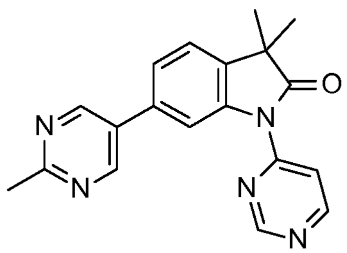

To a mixture of 3,3-dimethyl-6-(2-methylpyrimidin-5-yl)indolin-2-one (100 mg, 395 μmol, Eq: 1.00, Example 70 step c), 4-bromopyrimidine hydrochloride (154 mg, 790 μmol, Eq: 2) and cesium carbonate (450 mg, 1.38 mmol, Eq: 3.5) was added degassed dioxane (3 ml). Then palladium (II) acetate (17.7 mg, 79.0 μmol, Eq: 0.2) was added at room temperature followed by 4,5-Bis(diphenylphosphino)-9,9-dimethylxanthene (Xantphos) (68.5 mg, 118 μmol, Eq: 0.3). The tube was inerted, sealed and the mixture was heated to 115 °C for 2 h. The reaction was concentrated in vacuo.

The residue was purified by chromatography on silica gel, followed by precipitation in pentane to give the title compound as a light brown solid (60 mg, 47%). MS (m/z) = 332.2 $[M + H]^+$.

**Example 94**

**1-(1-ethyl-1H-pyrazol-3-yl)-3,3-dimethyl-6-(2-methylpyrimidin-5-yl)indolin-2-one**

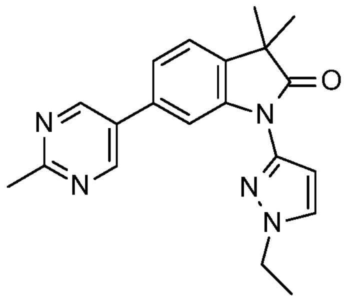

The title compound was prepared in analogy to Example 70 step d, with 3,3-dimethyl-6-(2-methylpyrimidin-5-yl)indolin-2-one (example 70 step c) and 1-ethyl-3-iodo-1H-pyrazole as starting materials. White solid. Yield: 8%. MS (m/z) = 348.2 $[M + H]^+$.

**Example 95**

**1-(5-cyclopropylpyrazin-2-yl)-3,3-dimethyl-6-(2-methylpyrimidin-5-yl)indolin-2-one**

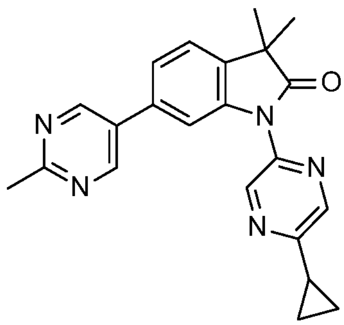

The title compound was prepared in analogy to Example 70 step d, with 3,3-dimethyl-6-(2-methylpyrimidin-5-yl)indolin-2-one (example 70 step c) and 2-bromo-5-cyclopropylpyrazine as starting materials. Light brown solid. Yield: 85%. MS (m/z) = 372.2 $[M + H]^+$.

**Example 96**

**5-(3,3-dimethyl-6-(2-methylpyrimidin-5-yl)-2-oxoindolin-1-yl)pyrazine-2-carbonitrile**

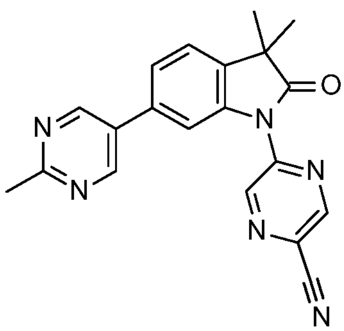

The title compound was prepared in analogy to Example 70 step d, with 3,3-dimethyl-6-(2-methylpyrimidin-5-yl)indolin-2-one (example 70 step c) and 5-bromopyrazine-2-carbonitrile as starting materials. White solid. Yield: 33%. MS (m/z) = 357.1 $[M + H]^+$.

**Example 97**

**1-(6-cyclopropylpyrazin-2-yl)-3,3-dimethyl-6-(2-methylpyrimidin-5-yl)indolin-2-one**

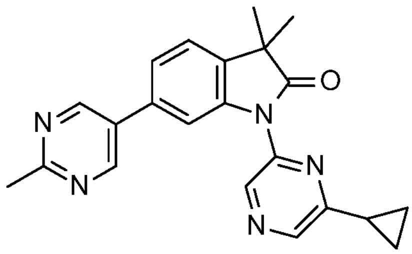

The title compound was prepared in analogy to Example 70 step d, with 3,3-dimethyl-6-(2-methylpyrimidin-5-yl)indolin-2-one (example 70 step c) and 2-bromo-6-cyclopropylpyrazine as starting materials. Light brown solid. Yield: 89%. MS (m/z) = 372.2 $[M + H]^+$.

**Example 98**

**1-(4,5-dimethylpyridin-3-yl)-3,3-dimethyl-6-(2-methylpyrimidin-5-yl)indolin-2-one**

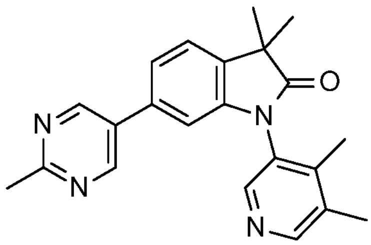

The title compound was prepared in analogy to Example 70 step d, with 3,3-dimethyl-6-(2-methylpyrimidin-5-yl)indolin-2-one (example 70 step c) and 3-bromo-4,5-dimethylpyridine as starting materials. White solid. Yield: 2%. MS (m/z) = 359.2 $[M + H]^+$.

**Example 99**

**3,3-dimethyl-1-(4-methylpyridin-3-yl)-6-(2-methylpyrimidin-5-yl)indolin-2-one**

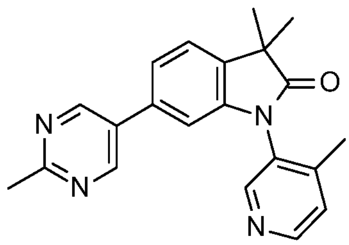

The title compound was prepared in analogy to Example 70 step d, with 3,3-dimethyl-6-(2-methylpyrimidin-5-yl)indolin-2-one (example 70 step c) and 3-bromo-4-methylpyridine as starting materials. White solid. Yield: 4%. MS (m/z) = 345.2 $[M + H]^+$.

**Example 100**

**1-(4,6-dimethylpyridin-3-yl)-3,3-dimethyl-6-(2-methylpyrimidin-5-yl)indolin-2-one**

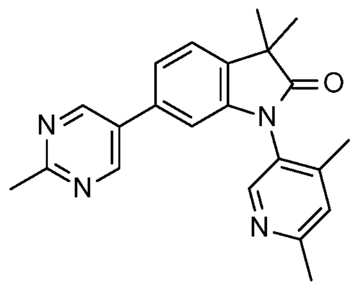

The title compound was prepared in analogy to Example 70 step d, with 3,3-dimethyl-6-(2-methylpyrimidin-5-yl)indolin-2-one (example 70 step c) and 5-bromo-2,4-dimethylpyridine as starting materials. White solid. Yield: 3%. MS (m/z) = 359.2 $[M + H]^+$.

**Example 101**

**5-(3,3-dimethyl-1-(5-methylpyrimidin-2-yl)-2-oxoindolin-6-yl)-2-methylpyrimidine 1-oxide**

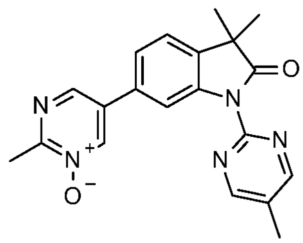

To a solution of 3,3-dimethyl-1-(5-methylpyrimidin-2-yl)-6-(2-methylpyrimidin-5-yl)indolin-2-one (20 mg, 57.9 μmol, Eq: 1.00, Example 53) in dichloromethane (0.6 ml) was added metachloroperbenzoic acid (19 mg, Eq: 1.3) at 22°C and mixture was stirred at 22 °C for 2 days. The mixture was partitioned between saturated aqueous solution of sodium carbonate (5 ml, 2N) and dichloromethane (5 ml). The aqueous layer was extracted with dichloromethane (3x5 ml) and the combined organic layers was dried, concentrated in vacuo and the residue was purified by chromatography on silica gel to give the desired compound as an off white solid (8 mg, 38%). MS (m/z) = 362.1 $[M + H]^+$.

**Example 102**

**1-(2-(hydroxymethyl)pyrimidin-5-yl)-3,3-dimethyl-6-(2-methylpyrimidin-5-yl)indolin-2-one**

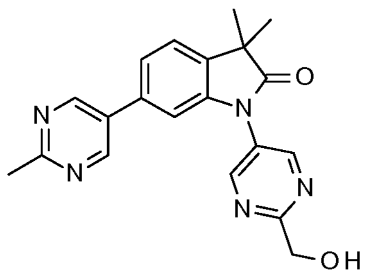

The title compound was prepared in analogy to Example 70 step d, with 3,3-dimethyl-6-(2-methylpyrimidin-5-yl)indolin-2-one (example 116 step a) and (5-bromopyrimidin-2-yl)methanol (prepared as previously described by e.g. Hasnik, Zbynek et al, Synlett, (4), 543-546; 2008)
as starting materials. Light brown solid. Yield: 31%. MS (m/z) = 362.2 $(M+H)^+$

**Example 103**

**1-[2-(aminomethyl)pyrimidin-5-yl]-3,3-dimethyl-6-(2-methylpyrimidin-5-yl)indol-2-one**

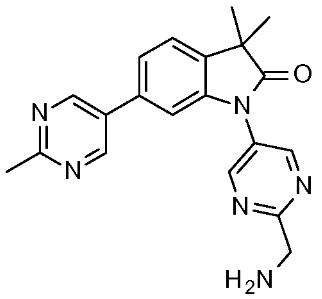

a) tert-butyl N-[[5-[3,3-dimethyl-6-(2-methylpyrimidin-5-yl)-2-oxo-indolin-1-yl]pyrimidin-2-yl]methyl]carbamate

The title compound was prepared in analogy to Example 70 step d, with 3,3-dimethyl-6-(2-methylpyrimidin-5-yl)indolin-2-one (example 116 step a) and tert-butyl (5-bromopyrimidin-2-yl)methylcarbamate. as starting materials. Off-white solid. Yield: 74%. MS (m/z) = 464.4.3 $(M+H)^+$

b) 1-[2-(aminomethyl)pyrimidin-5-yl]-3,3-dimethyl-6-(2-methylpyrimidin-5-yl)indol-2-one

To a solution of tert-butyl (5-(3,3-dimethyl-6-(2-methylpyrimidin-5-yl)-2-oxoindolin-1-yl)pyrimidin-2-yl)methylcarbamate (100 mg, 217 μmol, Eq: 1.00, example 103 step a) in dichloromethane (1 ml) was added trifluoroacetic acid (248 mg, 167 μl, 2.17 mmol, Eq: 10) at 0 °C and then mixture was stirred at 0 °C for 15min and at 22 °C for 16h.

The reaction mixture was basified by dropwise addition of an aqueous solution of sodium hydroxide (2N, pH ca. 14) then extracted with dichloromethane, dried and evaporated.

The combined organic layers was dried, concentrated in vacuo and the residue was purified by chromatography on silica gel to give the desired compound as an off-white solid (36 mg, 46%). MS (m/z) = 361.2 [M + H]+

**Example 104**

**3,3-dimethyl-6-(2-methylpyrimidin-5-yl)-1-(2H-tetrazol-5-yl)indolin-2-one**

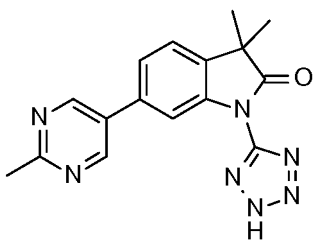

a) 3,3-dimethyl-6-(2-methylpyrimidin-5-yl)-2-oxo-indoline-1-carbonitrile

To a solution of 3,3-dimethyl-6-(2-methylpyrimidin-5-yl)indolin-2-one (300 mg, 1.18 mmol, Eq: 1.00, Example 70 step c) in dimethylformamide (4.5 ml) was added a suspension of sodium hydride in oil (60%, 62.0 mg, 1.42 mmol, Eq: 1.2) at 0°C. The mixture was warmed to 22°C and stirring was continued for 30 min. To the light yellow solution was

added at 0 °C cyanic bromide (163 mg, 1.54 mmol, Eq: 1.3) and stirring was continued at 22 °C for 1.5 h.
The reaction mixture was concentrated in vacuo and the residue was partitioned between water (10 ml) and ethyl acetate (10 ml). The aqueous layer was extracted three times with ethyl acetate (3x10 ml).
The combined organic layers was dried, concentrated in vacuo and the residue was purified by chromatography on silica gel to give the desired compound as a white solid (230 mg, 70%). MS(m/z) = 279.2 $[M + H]^+$

b) 3,3-dimethyl-6-(2-methylpyrimidin-5-yl)-1-(2H-tetrazol-5-yl)indolin-2-one

To a solution of 3,3-dimethyl-6-(2-methylpyrimidin-5-yl)-2-oxoindoline-1-carbonitrile (100 mg, 359 μmol, Eq: 1.00, example 104 step a) in a mixture of dimethylformamide (3 ml), water (2 ml) and isopropyl alcohol (1 ml) were added at 0 °C sodium azide (70.1 mg, 1.08 mmol, Eq: 3.00) and zinc bromide (68.8 mg, 305 μmol, Eq: 0.85). The reaction mixture was stirred at 0 °C for 16 h.
Volatiles were removed in vacuo and the residue was stirred in dichloromethane (15 ml). The suspension was filtered and the filtrate was concentrated in vacuo to give a colorless oil.
This oil was crystallized from boiling EtOH (5 ml) to give the title compound (61 mg, 53%) as a white solid. MS(m/z) = 322.1 $[M + H]^+$.

**Example 105**

**3,3-dimethyl-1-(2-methyl-2H-tetrazol-5-yl)-6-(2-methylpyrimidin-5-yl)indolin-2-one**

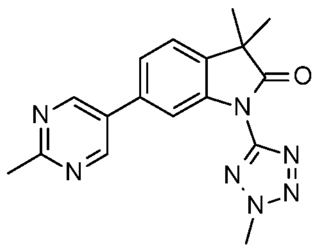

To a suspension of 3,3-dimethyl-6-(2-methylpyrimidin-5-yl)-1-(2H-tetrazol-5-yl)indolin-2-one (60 mg, 187 μmol, Eq: 1, example 110) in acetone (2.5 ml) were added potassium carbonate (51.6 mg, 373 μmol, Eq: 2.00) and methyl iodide (53 mg, 23.4 μl, 373 μmol, Eq: 2.00) at 0 °C. The reaction mixture was stirredat 22 °C for 20h. Further methyl iodide (26.5 mg, 11.7 μl, 187 μmol, Eq: 1) was added and mixture stirred at 50 °C for 16h. The mixture was partitioned between water (10 ml) and dichloromethane (10 ml), the aqueous layer was extracted three times with dichloromethane (3x10 ml).
The combined organic layers was dried, concentrated in vacuo and the residue was purified by chromatography on silica gel to give the desired compound as an off- white solid (10 mg, 16%).
MS(m/z) = 336.2 [M + H]+

**Example 106 and Example 107**

**3-(3,3-dimethyl-6-(2-methylpyrimidin-5-yl)-2-oxoindolin-1-yl)-6-methylpyridazine 1-oxide and 3-(3,3-dimethyl-6-(2-methyl-1-oxidopyrimidin-5-yl)-2-oxoindolin-1-yl)-6-methylpyridazine 1-oxide**

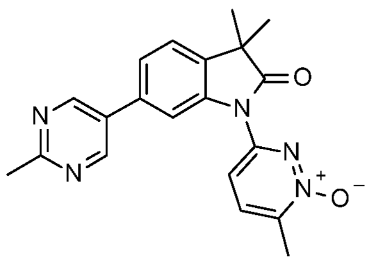

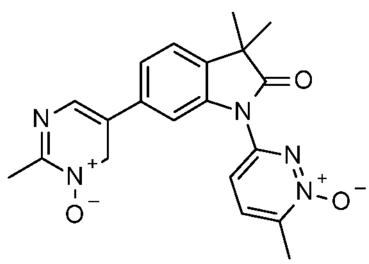

To a solution of 3,3-dimethyl-1-(6-methylpyridazin-3-yl)-6-(2-methylpyrimidin-5-yl)indolin-2-one (100 mg, 290 μmol, Eq: 1, example 48) in dichloromethane (3 ml) was added at 22 °C m-CPBA (100 mg, Eq: 1.25). The reaction mixture was stirred at 22 °C for 20 h.

The reaction was partitioned between an aqueous solution of sodium carbonate (10% m/m, 10 ml) and dichloromethane (10 ml), the aqueous layer was extracted with dichloromethane (3 x 10 ml).

The combined organic layers was dried, concentrated in vacuo and the residue was purified by chromatography on silica gel to give 2 fractions:

Fraction 1: 3-(3,3-dimethyl-6-(2-methylpyrimidin-5-yl)-2-oxoindolin-1-yl)-6-methylpyridazine 1-oxide, (44 mg, 42%) as a light yellow solid
Rf (dichloromethane/methanol 15:1)= 0.25 (UV) LC-MS: m/z = 362.3 [M + H]+
Fraction 2: 3-(3,3-dimethyl-6-(2-methyl-1-oxidopyrimidin-5-yl)-2-oxoindolin-1-yl)-6-methylpyridazine 1-oxide (15 mg, 14%) as white solid, Rf (dichloromethane/methanol 15:1)= 0.20 (UV), MS(m/z) = 378.3 [M + H]+

**Example 108**

**1-(2-(fluoromethyl)pyrimidin-5-yl)-3,3-dimethyl-6-(2-methylpyrimidin-5-yl)indolin-2-one**

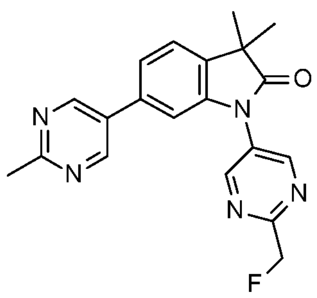

a) 5-bromo-2-(fluoromethyl)pyrimidine

To a solution of (5-bromopyrimidin-2-yl)methanol (400 mg, 2.12 mmol, Eq: 1) in dichloromethane (11 mlwas added diethylaminosulfur trifluoride (409 mg, 336 μl, 2.54 mmol, Eq: 1.20). The reaction mixture was stirred at room temperature for 4h then partitionned between an aqueous saturated solution of sodium hydrogenocarbonate and dichloromethane. The aquesous layer was extracted with dichloromethane.

The combined organic layers was dried, concentrated in vacuo and the residue was purified by chromatography on silica gel to give the desired compound as an off- white solid (110 mg, 27%). MS (m/z) = 191.0 /193.0 [M + H]+

b) 1-(2-(fluoromethyl)pyrimidin-5-yl)-3,3-dimethyl-6-(2-methylpyrimidin-5-yl)indolin-2-one

The title compound was prepared in analogy to Example 70 step d, with 3,3-dimethyl-6-(2-methylpyrimidin-5-yl)indolin-2-one (example 70 step c) and 5-bromo-2-(fluoromethyl)pyrimidine (Example 108 step a) as starting materials. White solid. Yield: 90%. MS (m/z)= 364.2 $(M+H)^+$

**Example 109**

**3,3-dimethyl-1-(6-methylpyridazin-3-yl)-6-(2-methylpyridin-4-yl)-1H-pyrrolo[3,2-c]pyridin-2(3H)-one**

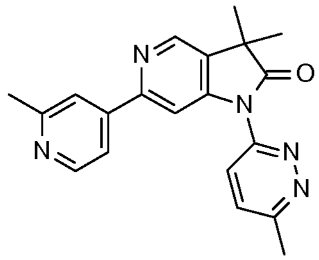

a) 3,3-dimethyl-6-(2-methyl-4-pyridyl)-1H-pyrrolo[3,2-c]pyridin-2-one

A degazed suspension of 6-chloro-3,3-dimethyl-1H-pyrrolo[3,2-c]pyridin-2(3H)-one (400 mg, 2.03 mmol, Eq: 1, prepared according to Woolford et al., WO 2012143726), (2-methylpyridin-4-yl)boronic acid (418 mg, 3.05 mmol, Eq: 1.5) in dioxane (8.14 ml), an aqueous solution of sodium carbonate (2M, 2.03 ml) and [1,1'-bis(diphenylphosphino)ferrocene]dichloropalladium(II) (74.4 mg, 102 μmol, Eq: 0.05) was heated at 110°C for 6h in a sealed vial. Volatiles were removed in vacuo and the residue was purified by chromatography on silica gel to give the desired compound as a light brown solid (415 mg, 77%). MS (m/z) = 254.2 $(M+H)^+$

b) 3,3-dimethyl-1-(6-methylpyridazin-3-yl)-6-(2-methylpyridin-4-yl)-1H-pyrrolo[3,2-c]pyridin-2(3H)-one

The title compound was prepared in analogy to Example 70 step d, with 3,3-dimethyl-6-(2-methylpyrimidin-5-yl)indolin-2-one (example 109 step a) and 3-iodo-6-methylpyridazine (CAS [1618-47-9]) as starting materials. White solid. Yield: 80%. MS (m/z) = 346.2 $(M+H)^+$

**Example 110**

**3,3-dimethyl-1-(1-methyl-1H-pyrazol-4-yl)-6-(2-methylpyridin-4-yl)-1H-pyrrolo[3,2-c]pyridin-2(3H)-one**

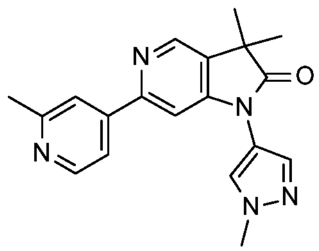

The title compound was prepared in analogy to Example 70 step d, with 3,3-dimethyl-6-(2-methylpyrimidin-5-yl)indolin-2-one (example 116 step a) and 4-iodo-1-methyl-1H-pyrazole as starting materials. White foam. Yield: 97%. MS (m/z) = 334.2 $(M+H)^+$

**Example 111**

**3,3-dimethyl-1-(1-methyl-1H-imidazol-4-yl)-6-(2-methylpyridin-4-yl)-1H-pyrrolo[3,2-c]pyridin-2(3H)-one**

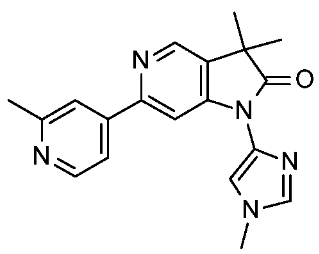

The title compound was prepared in analogy to Example 70 step d, with 3,3-dimethyl-6-(2-methylpyrimidin-5-yl)indolin-2-one (example 116 step a) and 4-iodo-1-methyl-1H-imidazole as starting materials. White solid. Yield: 81%. MS (m/z) = 334.2 $(M+H)^+$

**Example 112**

**6-(4-fluorophenyl)-3,3-dimethyl-1-(2-methylpyrimidin-5-yl)-1H-pyrrolo[3,2-c]pyridin-2(3H)-one**

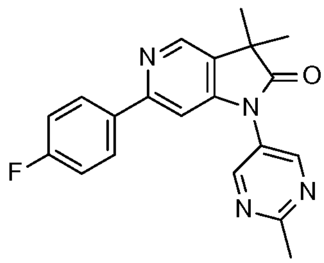

a) 6-(4-fluorophenyl)-3,3-dimethyl-1H-pyrrolo[3,2-c]pyridin-2-one

The title compound was prepared in analogy to Example 109 step a, with 6-chloro-3,3-dimethyl-1H-pyrrolo[3,2-c]pyridin-2(3H)-one (prepared according to Woolford et al., WO 2012143726) and (4-fluorophenyl)boronic acid as starting materials. White solid. Yield: 47%.
MS (m/z) = 257.2 $(M+H)^+$

b) 6-(4-fluorophenyl)-3,3-dimethyl-1-(2-methylpyrimidin-5-yl)-1H-pyrrolo[3,2-c]pyridin-2(3H)-one

The title compound was prepared in analogy to Example 70 step d, with 6-(4-fluorophenyl)-3,3-dimethyl-1H-pyrrolo[3,2-c]pyridin-2-one (example 112 step a) and 5-bromo-2-methylpyrimidine hydrochloride as starting materials. White solid. Yield: 88%. MS (m/z) = 349.3 $(M+H)^+$

**Example 113**

**1-(5-chloropyrimidin-2-yl)-3,3-dimethyl-6-(2-methylpyrimidin-5-yl)indolin-2-one**

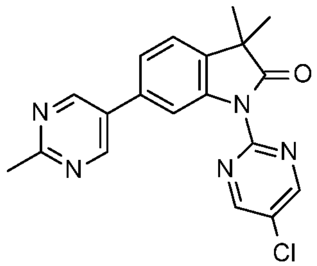

The title compound was prepared in analogy to Example 70 step d, with 3,3-Dimethyl-6-(2-methylpyrimidin-5-yl)indolin-2-one (example 70 step c) and 5-chloro-2-iodopyrimidine as starting materials. White solid. Yield: 84%. MS (m/z) = 366.2 $(M+H)^+$

**Example 114**

**1-(2-chloropyrimidin-5-yl)-3,3-dimethyl-6-(2-methylpyrimidin-5-yl)indolin-2-one**

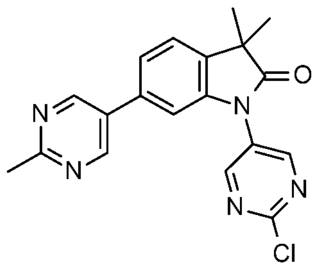

The title compound was prepared in analogy to Example 70 step d, with 3,3-Dimethyl-6-(2-methylpyrimidin-5-yl)indolin-2-one (example 70 step c) and 2-chloro-5-iodopyrimidine as starting materials. White solid. Yield: 20%. MS (m/z) = 366.2 $(M+H)^+$

### Example 115

**1-(2,6-dichloropyridin-4-yl)-3,3-dimethyl-6-(2-methylpyrimidin-5-yl)indolin-2-one**

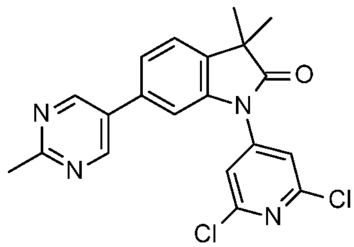

The title compound was prepared in analogy to Example 70 step d, with 3,3-Dimethyl-6-(2-methylpyrimidin-5-yl)indolin-2-one (example 70 step c) and 2,6-dichloro-4-iodopyridine as starting materials. White solid. Yield: 63%. MS (m/z) = 399.2 $(M+H)^+$

### Example 116

**1-(2-cyclopropylpyrimidin-5-yl)-3,3-dimethyl-6-(2-methylpyrimidin-5-yl)indolin-2-one**

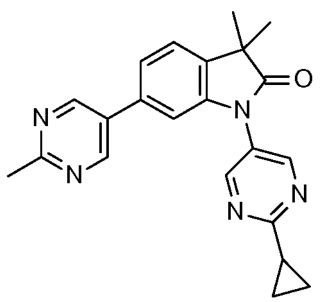

The title compound was prepared in analogy to Example 70 step d, with 3,3-Dimethyl-6-(2-methylpyrimidin-5-yl)indolin-2-one (example 70 step c) and 5-bromo-2-cyclopropylpyrimidine as starting materials. Off-white solid. Yield: 70%. MS (m/z) = 372.3 $(M+H)^+$

**Example 117**

**1-(5-chloropyrazin-2-yl)-3,3-dimethyl-6-(2-methylpyrimidin-5-yl)indol-2-one**

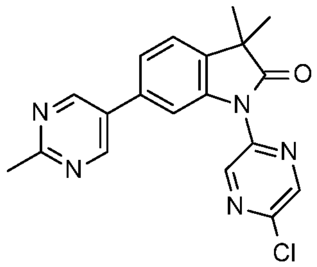

The title compound was prepared in analogy to Example 70 step d, with 3,3-Dimethyl-6-(2-methylpyrimidin-5-yl)indolin-2-one (example 70 step c) and 2,5-dichloropyrazine as starting materials. White solid. Yield: 6%. MS (m/z) = 366.2 $(M+H)^+$

**Example 118**

**1-(6-chloropyridazin-3-yl)-3,3-dimethyl-6-(2-methylpyrimidin-5-yl)indolin-2-one**

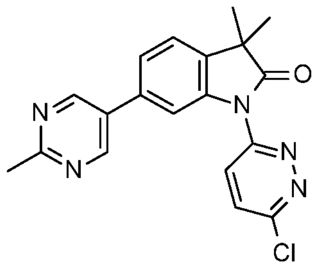

The title compound was prepared in analogy to Example 70 step d, with 3,3-Dimethyl-6-(2-methylpyrimidin-5-yl)indolin-2-one (example 70 step c) and 3,6-dichloropyridazine as starting materials. White solid. Yield: 2%. MS (m/z) = 366.2 $(M+H)^+$

**Example 119**

**1-(2-chloro-6-methylpyridin-4-yl)-3,3-dimethyl-6-(2-methylpyrimidin-5-yl)indolin-2-one**

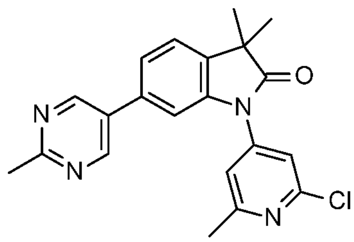

The title compound was prepared in analogy to Example 70 step d, with 3,3-Dimethyl-6-(2-methylpyrimidin-5-yl)indolin-2-one (example 70 step c) and 4-bromo-2-chloro-6-methylpyridine as starting materials. Light yellow solid. Yield: 42%. MS (m/z) = 379.2 $(M+H)^+$

**Example 120**

**3,3-dimethyl-6-(2-methylpyrimidin-5-yl)-1-(pyridazin-4-yl)indolin-2-one**

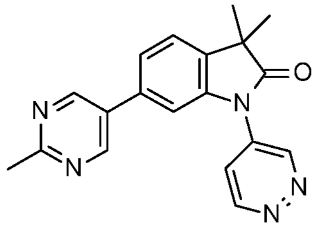

The title compound was prepared in analogy to Example 70 step d, with 3,3-Dimethyl-6-(2-methylpyrimidin-5-yl)indolin-2-one (example 70 step c) and 4-bromopyridazine as starting materials. Off-white solid. Yield: 42%. MS (m/z) = 332.2 $(M+H)^+$

**Example 121**

**1-(6-chloro-2-methylpyrimidin-4-yl)-3,3-dimethyl-6-(2-methylpyrimidin-5-yl)indolin-2-one**

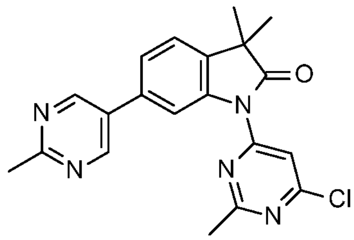

The title compound was prepared in analogy to Example 70 step d, with 3,3-Dimethyl-6-(2-methylpyrimidin-5-yl)indolin-2-one (example 70 step c) and 4,6-dichloro-2-methylpyrimidine as starting materials. Off-white solid. Yield: 10%. MS (m/z) = 380.2 $(M+H)^+$

**Example 122**

**3,3-dimethyl-1-(5-methylpyridazin-3-yl)-6-(2-methylpyrimidin-5-yl)indolin-2-one**

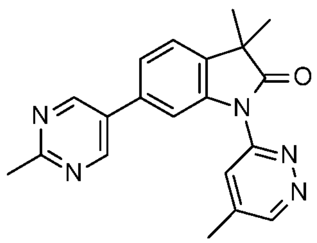

The title compound was prepared in analogy to Example 70 step d, with 3,3-Dimethyl-6-(2-methylpyrimidin-5-yl)indolin-2-one (example 70 step c) and 3-bromo-5-methylpyridazine as starting materials. White solid. Yield: 7%. MS (m/z) = 346.2 $(M+H)^+$

**Example 123**

**3,3-dimethyl-1-(3-methyl-1,2,4-thiadiazol-5-yl)-6-(2-methylpyrimidin-5-yl)indolin-2-one**

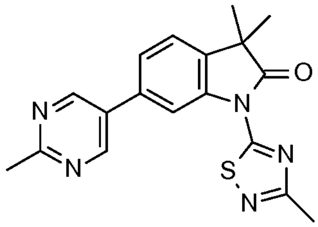

The title compound was prepared in analogy to Example 70 step d, with 3,3-Dimethyl-6-(2-methylpyrimidin-5-yl)indolin-2-one (example 70 step c) and 5-bromo-3-methyl-1,2,4-thiadiazole as starting materials. Off-white solid. Yield: 21%. MS (m/z) = 352.2 $(M+H)^+$

**Example 124**

**3,3-dimethyl-1-(3-methylisothiazol-5-yl)-6-(2-methylpyrimidin-5-yl)indolin-2-one**

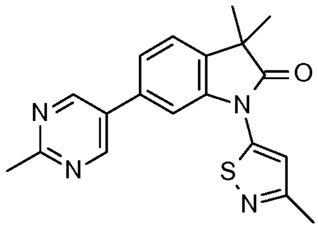

The title compound was prepared in analogy to Example 70 step d, with 3,3-Dimethyl-6-(2-methylpyrimidin-5-yl)indolin-2-one (example 70 step c) and 5-bromo-3-methylisothiazole as starting materials. Off-white solid. Yield: 94%. MS (m/z) = 351.2 $(M+H)^+$

**Example 125**

**3,3-dimethyl-6-(2-methylpyrimidin-5-yl)-1-(5-methylthiazol-2-yl)indolin-2-one**

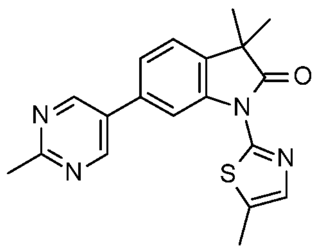

The title compound was prepared in analogy to Example 70 step d, with 3,3-Dimethyl-6-(2-methylpyrimidin-5-yl)indolin-2-one (example 70 step c) and 2-bromo-5-methylthiazole as starting materials. Light brown solid. Yield: 74%. MS (m/z) = 351.2 $(M+H)^+$

**Example 126**

**1-(6-chloropyridazin-4-yl)-3,3-dimethyl-6-(2-methylpyrimidin-5-yl)indolin-2-one**

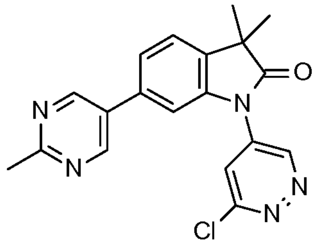

The title compound was prepared in analogy to Example 70 step d, with 3,3-Dimethyl-6-(2-methylpyrimidin-5-yl)indolin-2-one (example 70 step c) and 5-bromo-3-chloropyridazine as starting materials. Off-white solid. Yield: 66%. MS (m/z) = 366.1 $(M+H)^+$

**Example 127**

**3,3-dimethyl-1-(3-methylpyrazin-2-yl)-6-(2-methylpyrimidin-5-yl)indolin-2-one**

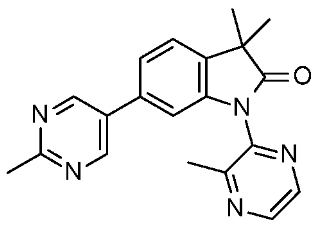

The title compound was prepared in analogy to Example 70 step d, with 3,3-Dimethyl-6-(2-methylpyrimidin-5-yl)indolin-2-one (example 70 step c) and 2-bromo-3-methylpyrazine as starting materials. Off-white solid. Yield: 88%. MS (m/z) = 346.1 $(M+H)^+$

**Example 128**

**1-(4-chloropyrimidin-2-yl)-3,3-dimethyl-6-(2-methylpyrimidin-5-yl)indolin-2-one**

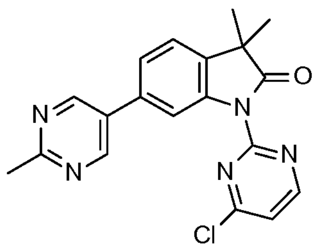

The title compound was prepared in analogy to Example 70 step d, with 3,3-Dimethyl-6-(2-methylpyrimidin-5-yl)indolin-2-one (example 70 step c) and 2-bromo-4-chloropyrimidine as starting materials. White solid. Yield: 7%. MS (m/z) = 366.2 $(M+H)^+$

**Example 129**

**1-(6-(methoxymethyl)pyridin-3-yl)-3,3-dimethyl-6-(2-methylpyrimidin-5-yl)indolin-2-one**

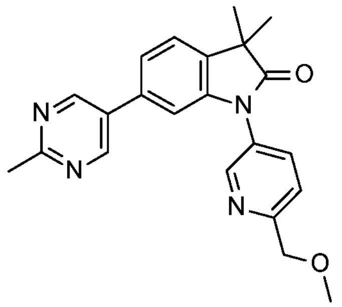

The title compound was prepared in analogy to Example 70 step d, with 3,3-Dimethyl-6-(2-methylpyrimidin-5-yl)indolin-2-one (example 70 step c) and 5-bromo-2-(methoxymethyl)pyridine as starting materials. White foam. Yield: quantitative
MS (m/z) = 375.2 $(M+H)^+$

**Example 130**

**1-(5-cyclopropylpyridazin-3-yl)-3,3-dimethyl-6-(2-methylpyrimidin-5-yl)indolin-2-one**

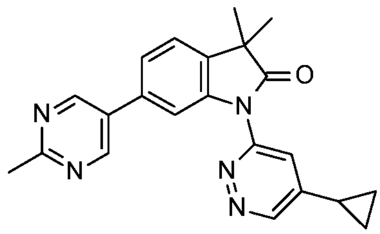

a) 3-chloro-5-cyclopropylpyridazine

A degazed mixture of 5-bromo-3-chloropyridazine (50 mg, 258 μmol, Eq: 1) and cyclopropylboronic acid (33.3 mg, 388 μmol, Eq: 1.5) in Dioxane (923 μl) and sodium carbonate in water (2M, 369 μl), [1,1'-bis(diphenylphosphino)ferrocene]dichloropalladium(II) (18.9 mg, 25.8 μmol, Eq: 0.1) was heated to 100°C overnight. The mixture was diluted with dichloromethane, adsorbed unto silica gel, dried in vacuo and the residue purified by flash chromatography on silica gel to give the title compound (27 mg, 68%) as a yellow viscous oil. MS (m/z): 155.0 $[(M+H)^+]$.

b) 1-(5-cyclopropylpyridazin-3-yl)-3,3-dimethyl-6-(2-methylpyrimidin-5-yl)indolin-2-one

The title compound was prepared in analogy to Example 70 step d, with 3,3-Dimethyl-6-(2-methylpyrimidin-5-yl)indolin-2-one (example 70 step c) and 3-chloro-5-cyclopropylpyridazine (example 130 step a) as starting materials. White solid. Yield: 43% MS (m/z) = 372.2 $(M+H)^+$

**Example 131**

**1-(1-isopropyl-1H-pyrazol-3-yl)-3,3-dimethyl-6-(2-methylpyrimidin-5-yl)indolin-2-one**

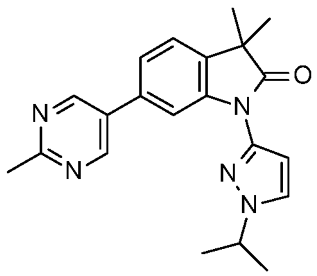

a) tert-butyl 3-(3,3-dimethyl-6-(2-methylpyrimidin-5-yl)-2-oxoindolin-1-yl)-1H-pyrazole-1-carboxylate

The title compound was prepared in analogy to Example 70 step d, with 3,3-Dimethyl-6-(2-methylpyrimidin-5-yl)indolin-2-one (example 70 step c) and tert-butyl 3-iodo-1H-pyrazole-1-carboxylate as starting materials. Light yellow solid. Yield: 40%. MS (m/z) = 420.2 $(M+H)^+$

b) 3,3-dimethyl-6-(2-methylpyrimidin-5-yl)-1-(1H-pyrazol-3-yl)indolin-2-one

A mixture of tert-butyl 3-(3,3-dimethyl-6-(2-methylpyrimidin-5-yl)-2-oxoindolin-1-yl)-1H-pyrazole-1-carboxylate (80 mg, 191 μmol, Eq: 1, example 131 step a) dissolved in dioxane (3 ml) and a solution of hydrochloric acid in dioxane (95.4 μl, 381 μmol, Eq: 2) was stirred 16h at room temperature. An additional solution of hydrochloric acid in dioxane (95.4 μl, 381 μmol, Eq: 2) was added and the reaction mixture was heated to 50°C for an additional 6 h.The reaction mixture was concentrated in vacuo and purified by flash chromatography then HPLC to afford 22 mg (36%) of the desired product as a white solid. MS (m/z) = 320.1$(M+H)^+$

c) 1-(1-isopropyl-1H-pyrazol-3-yl)-3,3-dimethyl-6-(2-methylpyrimidin-5-yl)indolin-2-one

A mixture of 3,3-dimethyl-6-(2-methylpyrimidin-5-yl)-1-(1H-pyrazol-3-yl)indolin-2-one (25 mg, 78.3 μmol, Eq: 1, example 131 step b) dissolved in dimethylformamide (833 μl), cesium carbonate (51 mg, 157 μmol, Eq: 2.00) and 2-iodopropane (20.4 mg, 12 μl, 117 μmol, Eq: 1.5) was stirred at room temperature for 20 h. The crude reaction mixture was concentrated in vacuo and purified by flash chromatography and then HPLC to afford 12 mg (42%) of the desired product as a white solid. MS (m/z) = 362.2 $(M+H)^+$

**Example 132**

**3,3-dimethyl-6-(5-methylpyrazin-2-yl)-1-(6-methylpyrazin-2-yl)indolin-2-one**

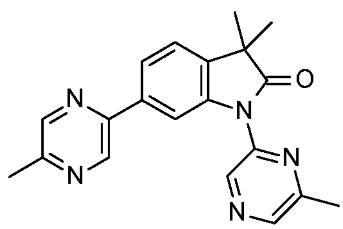

a) 3,3-dimethyl-6-(5-methylpyrazin-2-yl)indolin-2-one

The title compound was prepared in analogy to Example 70 step c, with 3,3-dimethyl-6-(4,4,5,5-tetramethyl-1,3,2-dioxaborolan-2-yl)indolin-2-o (example 70 step b) and 2-bromo-5-methylpyrazine as starting materials. White solid. Yield: 63%. MS (m/z) = 254.2 $(M+H)^+$

b) 3,3-dimethyl-6-(5-methylpyrazin-2-yl)-1-(6-methylpyrazin-2-yl)indolin-2-one

The title compound was prepared in analogy to Example 70 step d, with 3,3-dimethyl-6-(5-methylpyrazin-2-yl)indolin-2-one (example 132 step a) and 2-bromo-6-methylpyrazine as starting materials. White solid. Yield: 93%. MS

(m/z) = 346.2 $(M+H)^+$

**Example 133**

**3,3-dimethyl-6-(5-methylpyrazin-2-yl)-1-(2-methylpyrimidin-5-yl)indolin-2-one**

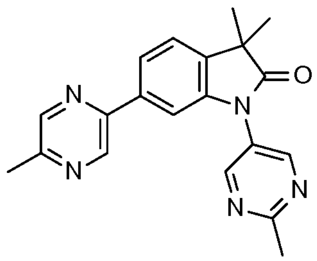

The title compound was prepared in analogy to Example 70 step d, with 3,3-dimethyl-6-(5-methylpyrazin-2-yl)indolin-2-one (example 132 step a) and 5-bromo-2-methylpyrimidine hydrochloride as starting materials. White solid. Yield: 86%. MS (m/z) = 346.2 $(M+H)^+$

**Example 134**

**1-(6-cyclopropylpyridazin-3-yl)-3,3-dimethyl-6-(2-methylpyrimidin-5-yl)indolin-2-one**

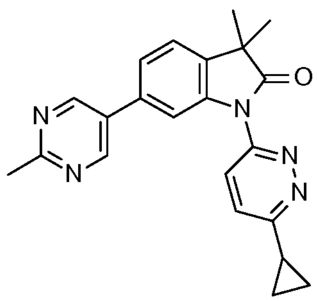

The title compound was prepared in analogy to Example 70 step d, with 3,3-Dimethyl-6-(2-methylpyrimidin-5-yl)indolin-2-one (example 70 step c) and 3-bromo-6-cyclopropylpyridazine as starting materials. White solid. Yield: 87%. MS (m/z) = 372.1 $(M+H)^+$

**Example 135**

**3,3-dimethyl-1-(6-methylpyrazin-2-yl)-6-(6-methyl-2-pyridyl)indolin-2-one**

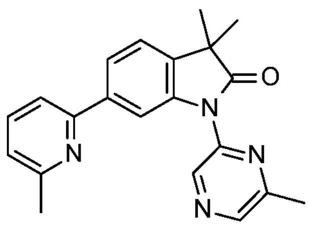

a) 6-bromo-3,3-dimethyl-1-(6-methylpyrazin-2-yl)indolin-2-one

The title compound was prepared in analogy to Example 70 step d, with 6-bromo-3,3-dimethyl-2,3-dihydro-1H-indol-2-one (example 70 step a) and 2-bromo-6-methyl-pyrazine as starting materials. Off-white solid. Yield: 84%. MS (m/z) = 331.8 $(M+H)^+$

b) 3,3-Dimethyl-1-(6-methylpyrazin-2-yl)-6-(tetramethyl-1,3,2-dioxaborolan-2-yl)-2,3-dihydro-1H-indol-2-one

The title compound was prepared in analogy to Example 136 step b, with 6-bromo-3,3-dimethyl-1-(6-methylpyrazin-2-yl)indolin-2-one (example 135 step a) as starting materials. Off-white solid. Yield: 96%. MS (m/z) = 379.8 $(M+H)^+$

c) 3,3-dimethyl-1-(6-methylpyrazin-2-yl)-6-(6-methyl-2-pyridyl)indolin-2-one

The title compound was prepared in analogy to Example 70 step c, with 3,3-Dimethyl-1-(6-methylpyrazin-2-yl)-6-(tetramethyl-1,3,2-dioxaborolan-2-yl)-2,3-dihydro-1H-indol-2-one (example 135 step b) and 2-bromo-6-methyl-pyridine as starting materials. Off-white solid. Yield: 33%. MS (m/z)= 344.8 $(M+H)^+$

**Example 136**

**3,3-dimethyl-1-(5-methylpyrazin-2-yl)-6-(2-methylpyrimidin-4-yl)indolin-2-one**

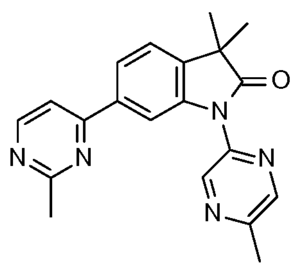

a) 6-Bromo-3,3-dimethyl-1-(5-methylpyrazin-2-yl)-2,3-dihydro-1H-indol-2-one

The title compound was prepared in analogy to Example 70 step d, with 6-bromo-3,3-dimethyl-2,3-dihydro-1H-indol-2-one (example 70 step a) and 2-bromo-5-methyl-pyrazine as starting materials. Off-white solid. Yield: 62%. MS (m/z) = 333.8 $(M+H)^+$

b) 3,3-Dimethyl-1-(5-methylpyrazin-2-yl)-6-(tetramethyl-1,3,2-dioxaborolan-2-yl)-2,3-dihydro-1H-indol-2-one

A degazed suspension of 6-bromo-3,3-dimethyl-1-(5-methylpyrazin-2-yl)-2,3-dihydro-1H-indol-2-one (1.7g, 5.12mmol, example 136 step a), potassium acetate (1.004g, 10.24mmol), bis(pinacolato)diboron (1.559g, 6.14mmol), [1,1'-bis(diphenylphosphino)ferrocene] dichloropalladium(II) dichloromethame complex (0.418g, 0.51 mmol) in dioxane (20mL) was stirred at 110°C for 6h. The reaction mixture was filtered and concentrated under vacuum and purified by chromatography on silica gel to afford the desired product as a white solid (33%). MS (m/) z= 379.9 $(M+H)^+$

c) 3,3-dimethyl-1-(5-methylpyrazin-2-yl)-6-(2-methylpyrimidin-4-yl)indolin-2-one

The title compound was prepared in analogy to Example 70 step c, with 3,3-Dimethyl-1-(5-methylpyrazin-2-yl)-6-(tetramethyl-1,3,2-dioxaborolan-2-yl)-2,3-dihydro-1H-indol-2-one (example 136 step b) and 4-bromo-2-methyl-pyrimidineas starting materials. White solid. Yield: 33%. MS (m/z) = 346.0 $(M+H)^+$

**Example 137**

**3,3-dimethyl-6-(5-methylpyrazin-2-yl)-1-(6-methylpyridazin-3-yl)indolin-2-one**

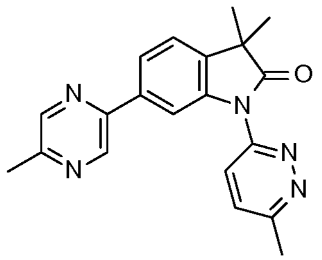

The title compound was prepared in analogy to Example 70 step d, with 3,3-dimethyl-6-(5-methylpyrazin-2-yl)indolin-2-one (example 132 step a) and 3-iodo-6-methyl-pyridazine as starting materials. Off-white solid. Yield: 73%. MS (m/z) = 346.1 (M+H)+

**Example 138**

**3,3-dimethyl-1-(1-methyl-1H-pyrazol-3-yl)-6-(5-methylpyrazin-2-yl)indolin-2-one**

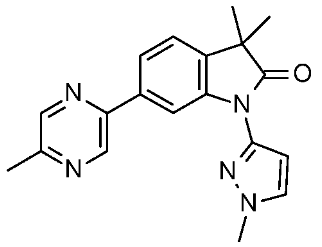

The title compound was prepared in analogy to Example 70 step d, with 3,3-dimethyl-6-(5-methylpyrazin-2-yl)indolin-2-one (example 132 step a) and 3-iodo-1-methyl-1H-pyrazole as starting materials. Light yellow solid. Yield: 65%. MS (m/z) = 334.2 (M+H)+

**Example 139**

**3,3-dimethyl-6-(2-methylpyrimidin-5-yl)-1-(1-(2,2,2-trifluoroethyl)-1H-pyrazol-3-yl)indolin-2-one**

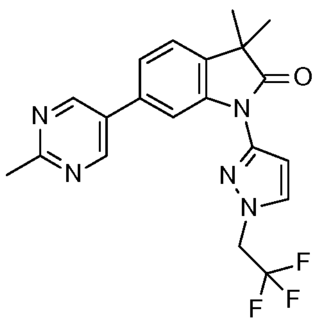

The title compound was prepared in analogy to Example 131 step c, with 3,3-dimethyl-6-(2-methylpyrimidin-5-yl)-1-(1H-pyrazol-3-yl)indolin-2-one (example 131 step b) and 2,2,2-trifluoroethyl iodide as starting materials. White solid. Yield: 15%. MS (m/z) = 402.2 (M+H)+

**Example 140**

**1-(1-(2-methoxyethyl)-1H-pyrazol-3-yl)-3,3-dimethyl-6-(2-methylpyrimidin-5-yl)indolin-2-one**

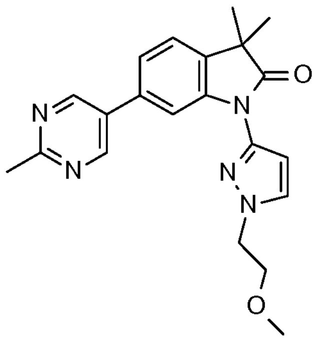

The title compound was prepared in analogy to Example 131 step c, with 3,3-dimethyl-6-(2-methylpyrimidin-5-yl)-1-(1H-pyrazol-3-yl)indolin-2-one (example 131 step b) and 2-Bromoethyl methylether as starting materials. White solid. Yield: 27%.
MS (m/z) = 378.2 $(M+H)^+$

**Example 141**

**2-(3-(3,3-dimethyl-6-(2-methylpyrimidin-5-yl)-2-oxoindolin-1-yl)-1H-pyrazol-1-yl)-N,N-dimethylacetamide**

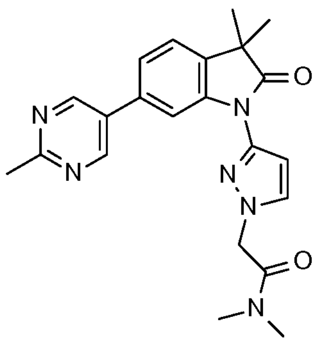

The title compound was prepared in analogy to Example 131 step c, with 3,3-dimethyl-6-(2-methylpyrimidin-5-yl)-1-(1H-pyrazol-3-yl)indolin-2-one (example 131 step b) and 2-Chloro-N,N-dimethylacetamide as starting materials. White solid. Yield: 40%. MS (m/z) = 405.2 $(M+H)^+$

**Example 142**

**1-(5-fluoropyridin-3-yl)-3,3-dimethyl-6-(2-methylpyrimidin-5-yl)indolin-2-one**

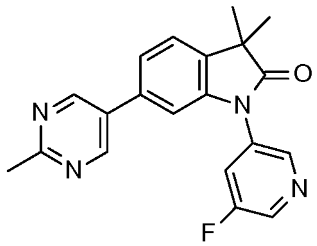

The title compound was prepared in analogy to Example 70 step d, with 3,3-Dimethyl-6-(2-methylpyrimidin-5-yl)indolin-2-one (example 70 step c) and 3-bromo-5-fluoropyridine as starting materials. White solid. Yield: quantitative. MS (m/z) = 349.1 $(M+H)^+$

**Example 143**

**5-(3,3-dimethyl-6-(2-methylpyrimidin-5-yl)-2-oxoindolin-1-yl)nicotinonitrile**

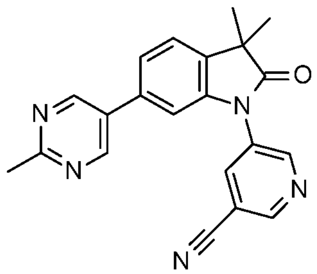

The title compound was prepared in analogy to Example 70 step d, with 3,3-Dimethyl-6-(2-methylpyrimidin-5-yl)indolin-2-one (example 70 step c) and 5-bromonicotinonitrile as starting materials. Off-white solid. Yield: 98%. MS (m/z) = 356.1 $(M+H)^+$

**Example 144**

**3,3-dimethyl-1-(6-methylpyrazin-2-yl)-6-(2-methyl-4-pyridyl)indolin-2-one**

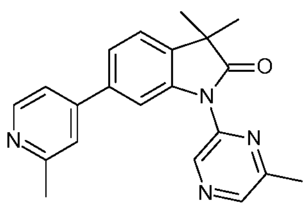

The title compound was prepared in analogy to Example 70 step c, with 3,3-Dimethyl-1-(6-methylpyrazin-2-yl)-6-(tetramethyl-1,3,2-dioxaborolan-2-yl)-2,3-dihydro-1H-indol-2-one(example 135 step b) and 4-bromo-2-methyl-pyridine as starting materials. White solid. Yield: 38%. MS (m/z) = 344.9 $(M+H)^+$

**Example 145**

**3,3-dimethyl-1-(6-methylpyrazin-2-yl)-6-(5-methyl-3-pyridyl)indolin-2-one**

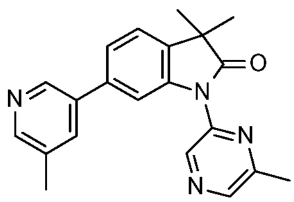

The title compound was prepared in analogy to Example 70 step c, with 3,3-Dimethyl-1-(6-methylpyrazin-2-yl)-6-(tetramethyl-1,3,2-dioxaborolan-2-yl)-2,3-dihydro-1H-indol-2-one(example 135 step b) and 3-bromo-5-methyl-pyridine as starting materials. Off-white solid. Yield: 34%. MS (m/z) = 344.9 $(M+H)^+$

**Example 146**

**6-(5-fluoro-3-pyridyl)-3,3-dimethyl-1-(6-methylpyrazin-2-yl)indolin-2-one**

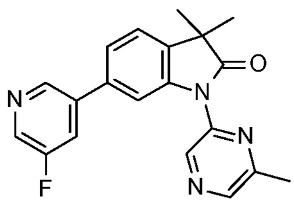

The title compound was prepared in analogy to Example 70 step c, with 3,3-Dimethyl-1-(6-methylpyrazin-2-yl)-6-(tetramethyl-1,3,2-dioxaborolan-2-yl)-2,3-dihydro-1H-indol-2-one(example 135 step b) and 3-bromo-5-fluoro-pyridine as starting materials. Off-white solid. Yield: 37%. MS (m/z) = 348.8 $(M+H)^+$

**Example 147**

**3,3-dimethyl-1-(5-methylpyrazin-2-yl)-6-(2-methyl-4-pyridyl)indolin-2-one**

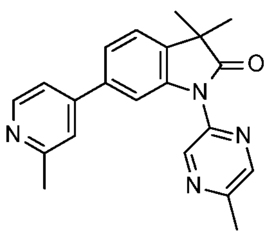

The title compound was prepared in analogy to Example 70 step c, with 3,3-Dimethyl-1-(5-methylpyrazin-2-yl)-6-(tetramethyl-1,3,2-dioxaborolan-2-yl)-2,3-dihydro-1H-indol-2-one (example 136 step b) and 4-bromo-2-methyl-pyridineas starting materials. White solid. Yield: 46%. MS (m/z) = 344.9 $(M+H)^+$

**Example 148**

**3,3-dimethyl-1-(5-methylpyrazin-2-yl)-6-(6-methyl-3-pyridyl)indolin-2-one**

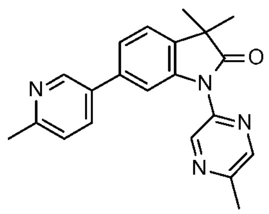

The title compound was prepared in analogy to Example 70 step c, with 3,3-Dimethyl-1-(5-methylpyrazin-2-yl)-6-(tetramethyl-1,3,2-dioxaborolan-2-yl)-2,3-dihydro-1H-indol-2-one (example 136 step b) and 5-bromo-2-methyl-pyridineas starting materials. White solid. Yield: 23%. MS (m/z) = 344.8 $(M+H)^+$

**Example 149**

**3,3-dimethyl-1-(5-methylpyrazin-2-yl)-6-(5-methyl-3-pyridyl)indolin-2-one**

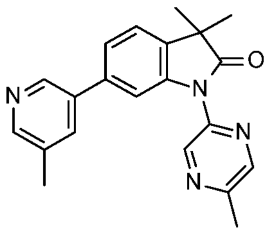

The title compound was prepared in analogy to Example 70 step c, with 3,3-Dimethyl-1-(5-methylpyrazin-2-yl)-6-(tetramethyl-1,3,2-dioxaborolan-2-yl)-2,3-dihydro-1H-indol-2-one (example 136 step b) and 3-bromo-5-methyl-pyridine.
as starting materials. White solid. Yield: 25%. MS (m/z) = 344.9 $(M+H)^+$

**Example 150**

**6-(5-fluoro-3-pyridyl)-3,3-dimethyl-1-(5-methylpyrazin-2-yl)indolin-2-one**

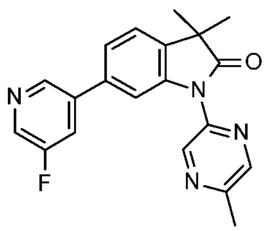

The title compound was prepared in analogy to Example 70 step c, with 3,3-Dimethyl-1-(5-methylpyrazin-2-yl)-6-(tetramethyl-1,3,2-dioxaborolan-2-yl)-2,3-dihydro-1H-indol-2-one (example 136 step b) and 3-bromo-5-fluoro-pyridineas starting materials. White solid. Yield: 37%. MS (m/z) = 349.2 $(M+H)^+$

**Example 151**

**6-(5-fluoro-6-methyl-3-pyridyl)-3,3-dimethyl-1-(5-methylpyrazin-2-yl)indolin-2-one**

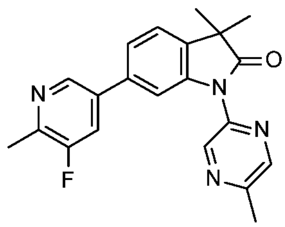

The title compound was prepared in analogy to Example 70 step c, with 3,3-Dimethyl-1-(5-methylpyrazin-2-yl)-6-(tetramethyl-1,3,2-dioxaborolan-2-yl)-2,3-dihydro-1H-indol-2-one (example 136 step b) and 5-bromo-3-fluoro-2-methyl-pyridineas starting materials. White solid. Yield: 53%. MS (m/z) = 363.0 $(M+H)^+$

**Example 152**

**3,3-dimethyl-6-(2-methylpyrimidin-5-yl)-1-(5-(trifluoromethyl)pyridin-3-yl)indolin-2-one**

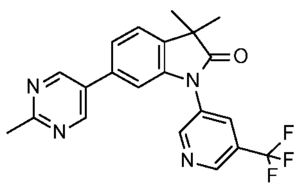

The title compound was prepared in analogy to Example 70 step d, with 3,3-dimethyl-6-(2-methylpyrimidin-5-yl)indolin-2-one (example 70 step c) and 3-bromo-5-(trifluoromethyl)pyridine as starting materials. White solid. Yield: quantitative.
MS (m/z)= 399.1 $(M+H)^+$

**Example 153**

**1-(5-(hydroxymethyl)pyridin-3-yl)-3,3-dimethyl-6-(2-methylpyrimidin-5-yl)indolin-2-one**

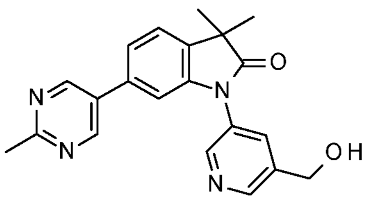

The title compound was prepared in analogy to Example 70 step d, with 3,3-dimethyl-6-(2-methylpyrimidin-5-yl)indolin-2-one (example 70 step c) and (5-bromopyridin-3-yl)methanol as starting materials. White solid. Yield: 75%. MS (m/z)= 361.2 $(M+H)^+$

**Example 154**

**3,3-dimethyl-1-(6-methylpyrazin-2-yl)-6-(2-methylpyrimidin-4-yl)indolin-2-one**

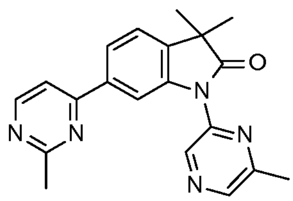

The title compound was prepared in analogy to Example 70 step c, with 3,3-Dimethyl-1-(6-methylpyrazin-2-yl)-6-(tetramethyl-1,3,2-dioxaborolan-2-yl)-2,3-dihydro-1H-indol-2-one (example 135 step b) and 4-bromo-2-methyl-pyrimidine as starting materials. Off-white solid. Yield: 46%. MS (m/z) = 346.1 $(M+H)^+$

**Example 155**

**5-[3,3-dimethyl-1-(6-methylpyrazin-2-yl)-2-oxo-indolin-6-yl]pyrimidine-2-carbonitrile**

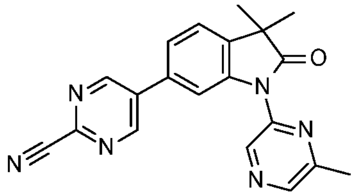

The title compound was prepared in analogy to Example 70 step c, with 3,3-dimethyl-1-(6-methylpyrazin-2-yl)-6-(tetramethyl-1,3,2-dioxaborolan-2-yl)-2,3-dihydro-1H-indol-2-one (example 135 step b) and 5-bromopyrimidine-2-carbonitrile as starting materials. Off-white solid. Yield: 16%. MS (m/z) = 357.3 $(M+H)^+$

**Example 156**

**1-(5-ethylpyridin-3-yl)-3,3-dimethyl-6-(2-methylpyrimidin-5-yl)indolin-2-one**

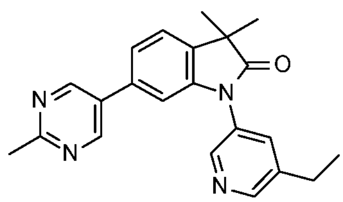

The title compound was prepared in analogy to Example 70 step d, with 3,3-dimethyl-6-(2-methylpyrimidin-5-yl)indolin-2-one (example 70 step c) and 3-bromo-5-ethylpyridine as starting materials. White solid. Yield: 96%. MS (m/z)= 359.2 $(M+H)^+$

**Example 157**

**6-(2-cyclopropylpyrimidin-5-yl)-3,3-dimethyl-1-(2-methylpyrimidin-5-yl)indolin-2-one**

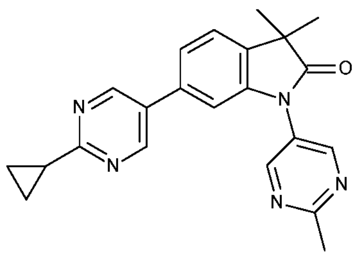

a) 6-bromo-3,3-dimethyl-1-(2-methylpyrimidin-5-yl)indolin-2-one

The title compound was prepared in analogy to Example 70 step d, with 6-bromo-3,3-dimethyl-2,3-dihydro-1H-indol-2-one (example 70 step a) and 5-bromo-2-methyl-pyrimidine as starting materials. Off-white solid. Yield: 39%.
MS m/z= 334.2 $(M+H)^+$

b) 3,3-dimethyl-1-(2-methylpyrimidin-5-yl)-6-(4,4,5,5-tetramethyl-1,3,2-dioxaborolan-2-yl)indolin-2-one

The title compound was prepared in analogy to Example 136 step b, with -bromo-3,3-dimethyl-1-(2-methylpyrimidin-5-yl)indolin-2-one (example 157 step a) as starting materials. Off-white solid. Used in the next step without further purification.
MS m/z= 380.0 $(M+H)^+$

c) 6-(2-cyclopropylpyrimidin-5-yl)-3,3-dimethyl-1-(2-methylpyrimidin-5-yl)indolin-2-one

The title compound was prepared in analogy to Example 70 step c, with 3,3-dimethyl-1-(2-methylpyrimidin-5-yl)-6-(4,4,5,5-tetramethyl-1,3,2-dioxaborolan-2-yl)indolin-2-one (example 157 step b) and 5-bromo-2-cyclopropyl-pyrimidine as starting materials. Off-white solid. Yield: 14% over 2 steps.
MS m/z= 372.1 $(M+H)^+$

Biological Assays and Data

Now it has been found that the compounds of formula I may be used for the treatment of CNS diseases. The described compounds of formula I reduce L-687,414-induced hyperlocomotion. This was assessed by using a computerized Digiscan 16 Animal Activity Monitoring System (Omnitech Electronics, Columbus, Ohio) to quantify locomotor activity. Animals were kept under a 12 h light/dark cycle and experiments were performed during the light period. Each activity monitoring chamber consisted of a Plexiglas box (41×41×28 cm; W×L×H) with sawdust bedding on the floor surrounded by invisible horizontal and vertical infrared sensor beams. The test boxes were divided by a Plexiglas cross providing each mouse with 20×20 cm of moving space. Cages were connected to a Digiscan Analyzer linked to a computer that constantly collected the beam status information. Records of photocell beam interruptions for individual animals were taken every 5 min over the duration of the experimental session and the sum of the first 6 periods was used as the final parameter. At least 8 mice were used in each treatment group. Compounds were administered either p.o. 15 min before a s.c. injection of 50 mg/kg of L-687,414, or i.p. at the same time as a s.c. injection of 50 mg/kg of L-687,414. Mice were then transferred from their home cage to the recording chambers for a 15-min habituation phase allowing free exploration of the new environment. Horizontal activity was then recorded for a 30-min time period. The % inhibition of L-687,414-induced hyperlocomotion was calculated according to the equation:

$$((\text{Veh+L-687,414 horizontal activity} - \text{drug+L-687,414 horizontal activity})/\text{Veh+L-687,414 horizontal activity}) \times 100$$

$ID_{50}$ values, defined as doses of each compound producing 50% inhibition of L-687,414-induced hyperlocomotion, were calculated by linear regression analysis of a dose-response data using an Excel-based computer-fitting program.
As data was not presupposed to be normally distributed, groups treated with test compounds were statistically compared with the control (vehicle-treated) group using one-tailed Mann Whitney U tests. In statistics, the Mann-Whitney U test

(also called the Mann-Whitney-Wilcoxon (MWW) or Wilcoxon rank-sum test) is a non-parametric statistical hypothesis test for assessing whether one of two samples of independent observations tends to have larger values than the other. It is one of the most well-known non-parametric significance tests. A p value gives the probability that two groups are significantly different from each other and the value of < 0.05 is generally accepted as a criterion, it implies that there is > 95% chance that two groups are really different from each other. P values given in table 1 are one-tailed since only decreases in locomotion were expected and tested for (Mann, H. B., Whitney, D. R. (1947), "On a Test of Whether one of Two Random Variables is Stochastically Larger than the Other", Annals of Mathematical Statistics, 18 (1), 50-60).

Measure of adenosine transport activity

To measure adenosine transport activity of ENT-1 mammalian cells, stable cells expressing the mouse ENT-1 transporter were plated on day 1 in 96-well culture plates at the density of 60,000 cells/well, in complete DMEM/F12 medium supplemented with glutamax, 10% FBS and 10 μg/ml puromycin. On day 2, the medium was aspirated and the cells were washed twice with uptake buffer (10 mM Hepes-Tris, pH 7.4 containing 150 mM NaCl, 1 mM

$CaCl_2$, 2.5 mM KCl, 2.5 mM $MgSO_4$, 10 mM D-glucose) (UB). For inhibition experiments, cells were then incubated at RT with various concentrations of compounds with 1 % DMSO final. Non-specific uptake was defined in the presence of 10 μM S-(4-Nitrobenzyl)-6-thioinosine (NBTI, Sigma Cat #N2255).

A solution containing [2,8-$^3$H]-adenosine 6 nM (40 Ci/mmol, American Radiolabeled chemicals Inc, Cat #ART 0287A) was then immediately added to the wells. The plates were then incubated for 20 min with gentle shaking and the reaction was stopped by aspiration of the mixture and washing (three times) with ice-cold UB. The cells were lysed by the addition of scintillation liquid, shaken 3 hours and the radioactivity in the cells was estimated using a microplates scintillation counter (TopCount NXT, Packard).

Table 1

| Effects of compounds of formula I on ENT1 inhibition and on L-687,414-induced hyperlocomotion | | | | | |
|---|---|---|---|---|---|
| **Expl.** | **structure** | **ENT1, adenosine uptake, $IC_{50}$ (UM)** | **L-687,414-induced hyperlocomotion** | | |
| | | | **Dose ip [mg/kg]** | **Inhibition, ip [%]** | **P value** |
| 1 | 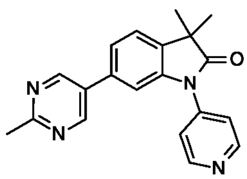 | 0.431 | 30 | 95.4 | 0.00008 |
| 2 | 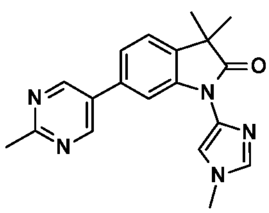 | 0.613 | 30 | 95.7 | 0.00008 |
| 3 | 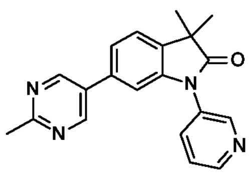 | 1.78 | | | |
| 4 | 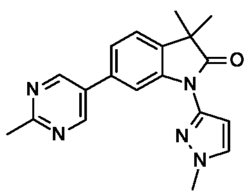 | 0.951 | 30 | 93.4 | 0.00008 |

(continued)

| Effects of compounds of formula I on ENT1 inhibition and on L-687,414-induced hyperlocomotion | | | | | |
|---|---|---|---|---|---|
| **Expl.** | **structure** | **ENT1, adenosine uptake, $IC_{50}$ (UM)** | **L-687,414-induced hyperlocomotion** | | |
| | | | **Dose ip [mg/kg]** | **Inhibition, ip [%]** | **P value** |
| 5 | 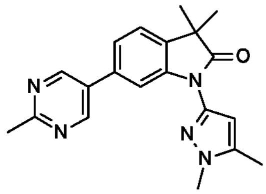 | 1.05 | | | |
| 6 | 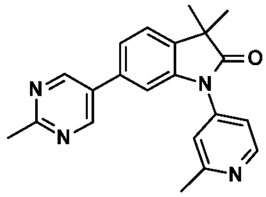 | 2.13 | | | |
| 7 | 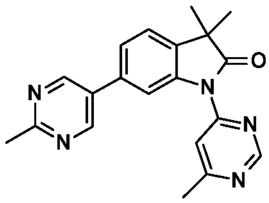 | 1.54 | | | |
| 8 | 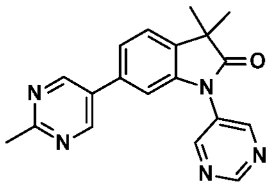 | 3.89 | | | |
| 9 | 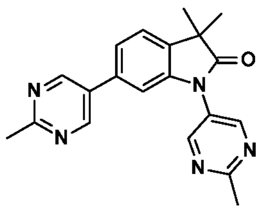 | 0.174 | 30 | 93.2 | 0.00016 |
| 10 | 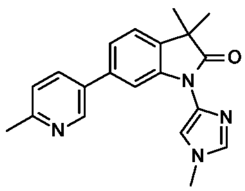 | 5.88 | | | |
| 11 | 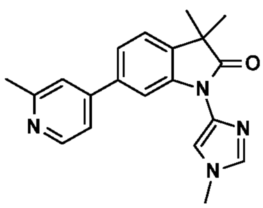 | 7.03 | | | |

(continued)

Effects of compounds of formula I on ENT1 inhibition and on L-687,414-induced hyperlocomotion

| Expl. | structure | ENT1, adenosine uptake, $IC_{50}$ (UM) | L-687,414-induced hyperlocomotion | | |
|---|---|---|---|---|---|
| | | | Dose ip [mg/kg] | Inhibition, ip [%] | P value |
| 12 | 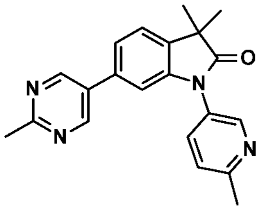 | 0.148 | | | |
| 13 | 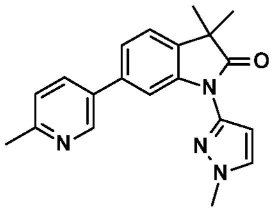 | 1.15 | | | |
| 16 | 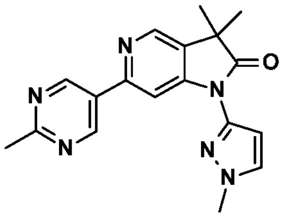 | | 30 | 78.3 | 0.00148 |
| 19 | 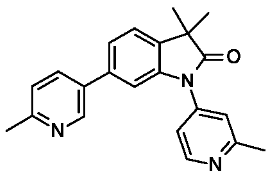 | 3.45 | | | |
| 20 | 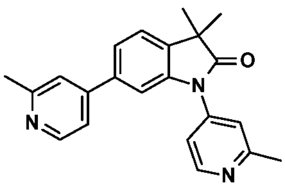 | 5.67 | | | |
| 21 | 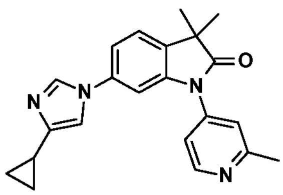 | 4.02 | | | |
| 22 | 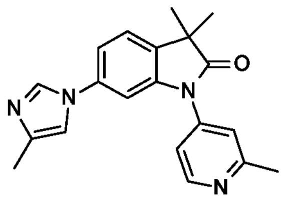 | | 30 | 63.8 | 0.01896 |

(continued)

| Effects of compounds of formula I on ENT1 inhibition and on L-687,414-induced hyperlocomotion | | | | | |
|---|---|---|---|---|---|
| Expl. | structure | ENT1, adenosine uptake, $IC_{50}$ (UM) | L-687,414-induced hyperlocomotion | | |
| | | | Dose ip [mg/kg] | Inhibition, ip [%] | P value |
| 24 | 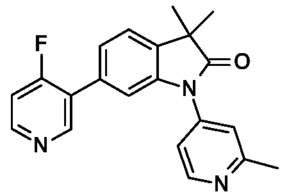 | 8.79 | | | |
| 27 | 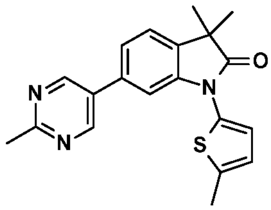 | 0.355 | | | |
| 30 | 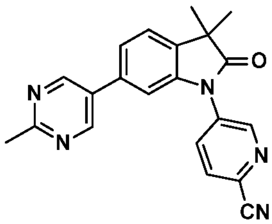 | 2,14 | | | |
| 31 | 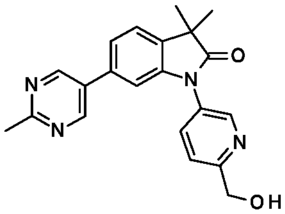 | 0.168 | | | |
| 32 | 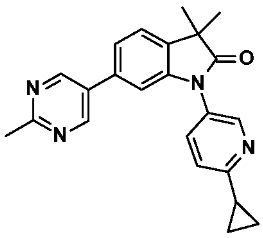 | 0.059 | | | |
| 33 | 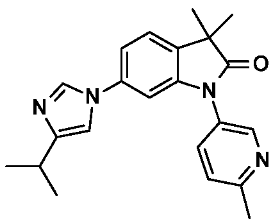 | 1.22 | | | |

(continued)

| Effects of compounds of formula I on ENT1 inhibition and on L-687,414-induced hyperlocomotion | | | | | |
|---|---|---|---|---|---|
| **Expl.** | **structure** | **ENT1, adenosine uptake, $IC_{50}$ (UM)** | **L-687,414-induced hyperlocomotion** | | |
| | | | **Dose ip [mg/kg]** | **Inhibition, ip [%]** | **P value** |
| 34 | 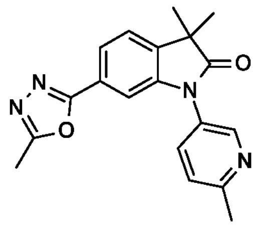 | | 30 | 59.7 | 0.03248 |
| 36 | 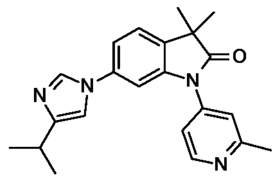 | 2.46 | | | |
| 37 | 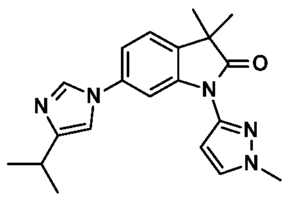 | 6.75 | 30 | 73.8 | 0.00093 |
| 38 | 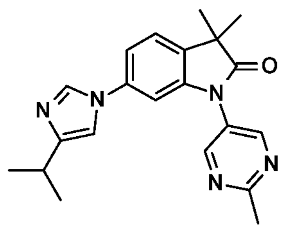 | 0.426 | | | |
| 44 | 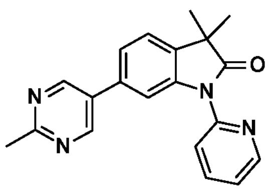 | 1.4 | | | |
| 45 | 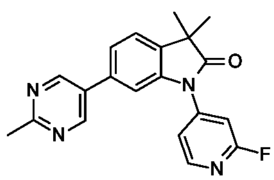 | 1 | 30 | 80.5 | 0.00016 |
| 46 | 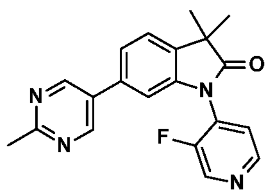 | 3.53 | | | |

(continued)

| Effects of compounds of formula I on ENT1 inhibition and on L-687,414-induced hyperlocomotion | | | | | |
|---|---|---|---|---|---|
| **Expl.** | **structure** | **ENT1, adenosine uptake, $IC_{50}$ (UM)** | **L-687,414-induced hyperlocomotion** | | |
| | | | **Dose ip [mg/kg]** | **Inhibition, ip [%]** | **P value** |
| 47 | 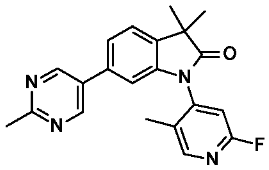 | 7.19 | | | |
| 48 | 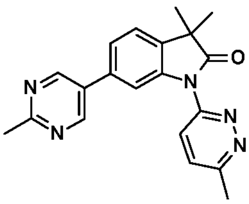 | 0.055 | | | |
| 49 | 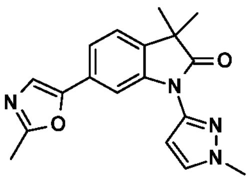 | | 30 | 47.1 | 0.01896 |
| 50 | 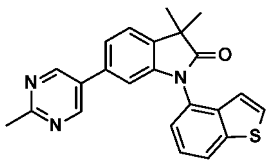 | 0.844 | | | |
| 51 | 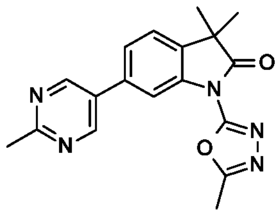 | 1.85 | | | |
| 52 | 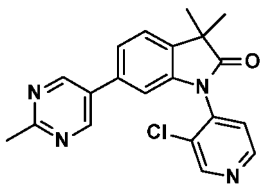 | 6.96 | | | |
| 53 | 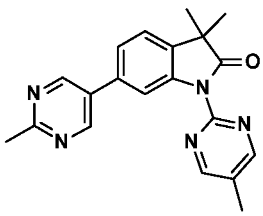 | 1.52 | | | |

(continued)

| Effects of compounds of formula I on ENT1 inhibition and on L-687,414-induced hyperlocomotion | | | | | |
|---|---|---|---|---|---|
| **Expl.** | **structure** | **ENT1, adenosine uptake, $IC_{50}$ (UM)** | **L-687,414-induced hyperlocomotion** | | |
| | | | **Dose ip [mg/kg]** | **Inhibition, ip [%]** | **P value** |
| 55 | 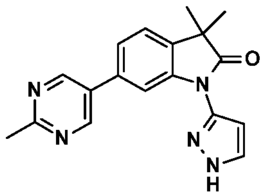 | 2.41 | | | |
| 56 | 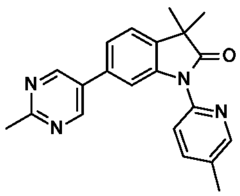 | 0.316 | | | |
| 57 | 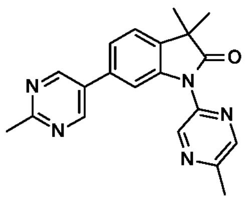 | 0.027 | | | |
| 59 | 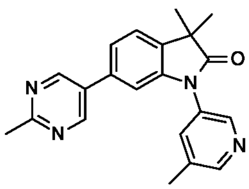 | 0.657 | | | |
| 60 | 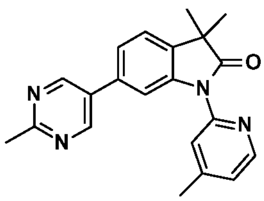 | 3.78 | | | |
| 61 | 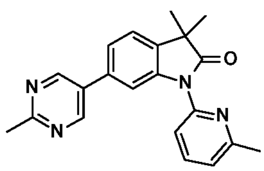 | 0.946 | | | |
| 63 | 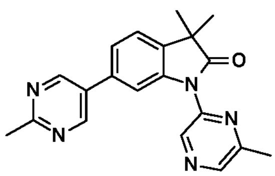 | 0.67 | | | |

(continued)

| Effects of compounds of formula I on ENT1 inhibition and on L-687,414-induced hyperlocomotion | | | | | |
|---|---|---|---|---|---|
| Expl. | structure | ENT1, adenosine uptake, $IC_{50}$ (UM) | L-687,414-induced hyperlocomotion | | |
| | | | Dose ip [mg/kg] | Inhibition, ip [%] | P value |
| 64 | 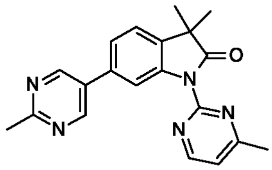 | 5.09 | | | |
| 65 | 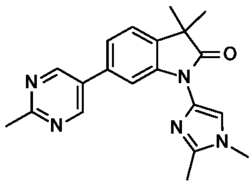 | 6.74 | | | |
| 66 | 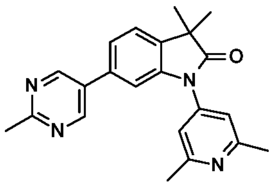 | 1.14 | | | |
| 67 | 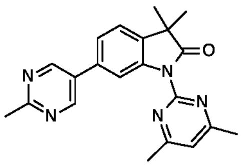 | 5.63 | | | |
| 68 | 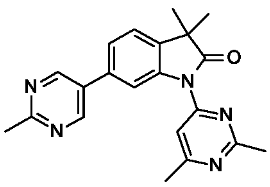 | 0.502 | | | |
| 69 | 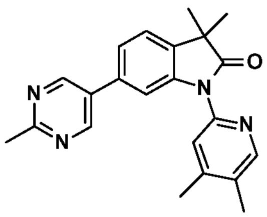 | 1.28 | | | |
| 70 | 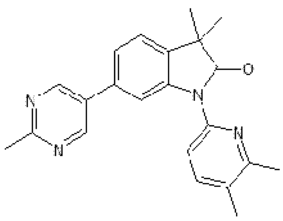 | 0.340 | 30 | 93.5 | 0.00097 |

(continued)

| Effects of compounds of formula I on ENT1 inhibition and on L-687,414-induced hyperlocomotion | | | | | |
|---|---|---|---|---|---|
| **Expl.** | **structure** | **ENT1, adenosine uptake, $IC_{50}$ (UM)** | **L-687,414-induced hyperlocomotion** | | |
| | | | **Dose ip [mg/kg]** | **Inhibition, ip [%]** | **P value** |
| 75 | 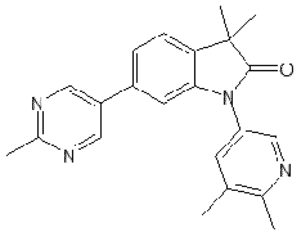 | 0.827 | | | |
| 76 | 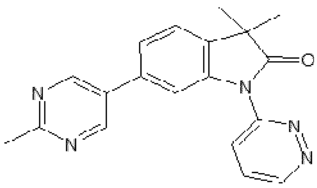 | 2.102 | | | |
| 77 | 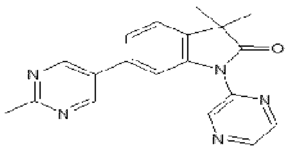 | 2.410 | 30 | 92.6 | 0.00138 |
| 78 | 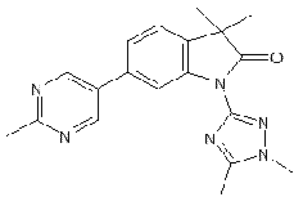 | | 30 | 89.9 | 0.00679 |
| 79 | 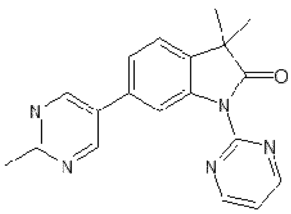 | 3.880 | | | |
| 80 | 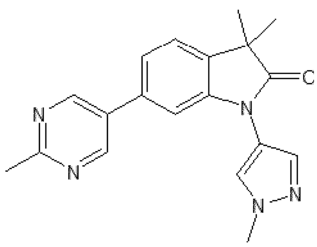 | 1.497 | | | |

(continued)

| Effects of compounds of formula I on ENT1 inhibition and on L-687,414-induced hyperlocomotion | | | | | |
|---|---|---|---|---|---|
| Expl. | structure | ENT1, adenosine uptake, $IC_{50}$ (UM) | L-687,414-induced hyperlocomotion | | |
| | | | Dose ip [mg/kg] | Inhibition, ip [%] | P value |
| 82 | 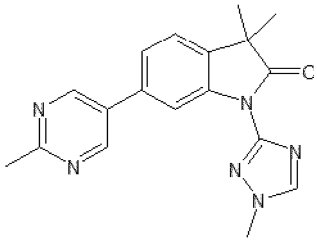 | | 30 | 89.6 | 0.00906 |
| 83 | 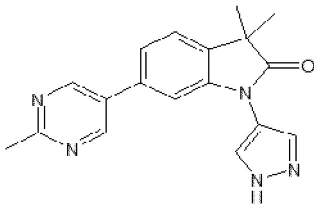 | 4.660 | | | |
| 84 | 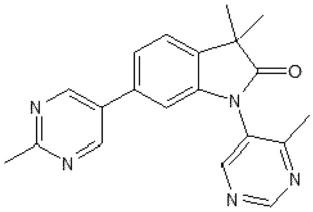 | 15.628 | | | |
| 85 | 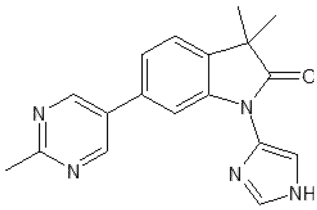 | 5.244 | | | |
| 86 | 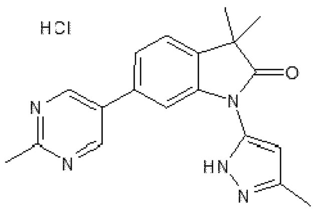 | 0.423 | | | |
| 87 | 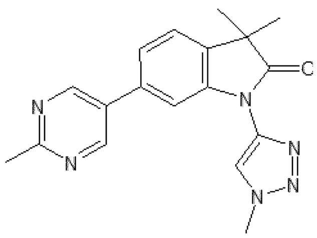 | 2.073 | | | |

(continued)

| Effects of compounds of formula I on ENT1 inhibition and on L-687,414-induced hyperlocomotion | | | | | |
|---|---|---|---|---|---|
| **Expl.** | **structure** | **ENT1, adenosine uptake, $IC_{50}$ (UM)** | **L-687,414-induced hyperlocomotion** | | |
| | | | **Dose ip [mg/kg]** | **Inhibition, ip [%]** | **P value** |
| 88 | 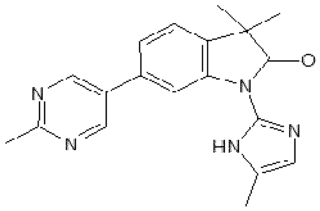 | 7.660 | | | |
| 91 | 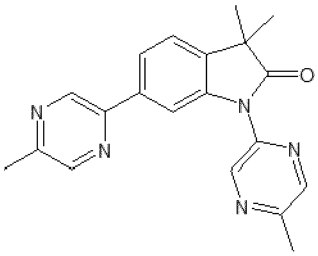 | 1.816 | 30 | 73.7 | 0.00137 |
| 92 | 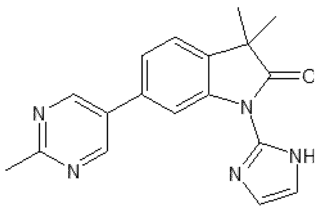 | 1.291 | | | |
| 93 | 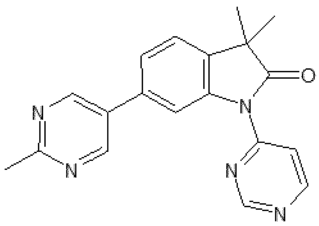 | | 30 | 92.4 | 0.00138 |
| 94 | 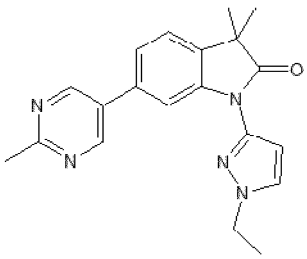 | 1.164 | | | |
| 95 | 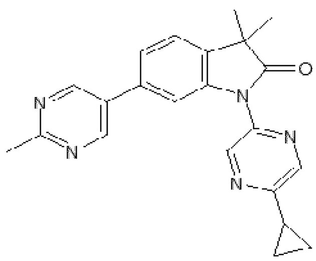 | 0.047 | 30 | 76.92 | 0.00194 |

(continued)

Effects of compounds of formula I on ENT1 inhibition and on L-687,414-induced hyperlocomotion

| Expl. | structure | ENT1, adenosine uptake, $IC_{50}$ (UM) | L-687,414-induced hyperlocomotion | | |
|---|---|---|---|---|---|
| | | | Dose ip [mg/kg] | Inhibition, ip [%] | P value |
| 96 | 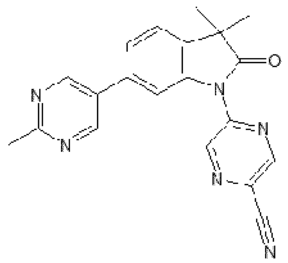 | 0.154 | | | |
| 97 | 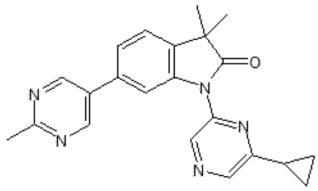 | 0.102 | 30 | 78.55 | 0.00073 |
| 99 | 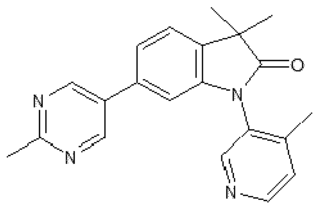 | 4.329 | | | |
| 101 | 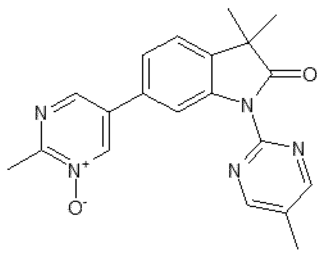 | 2.401 | | | |
| 102 | 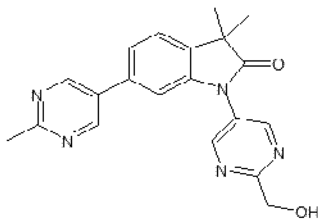 | 10.177 | | | |
| 103 | 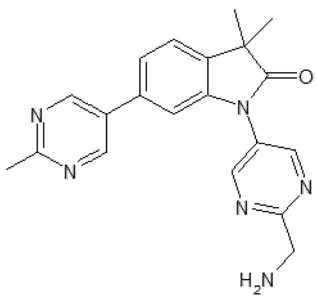 | 18.351 | | | |

(continued)

| Effects of compounds of formula I on ENT1 inhibition and on L-687,414-induced hyperlocomotion | | | | | |
|---|---|---|---|---|---|
| **Expl.** | **structure** | **ENT1, adenosine uptake, $IC_{50}$ (UM)** | **L-687,414-induced hyperlocomotion** | | |
| | | | **Dose ip [mg/kg]** | **Inhibition, ip [%]** | **P value** |
| 105 | 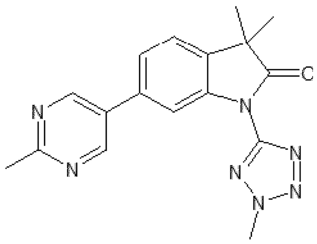 | 6.996 | | | |
| 106 | 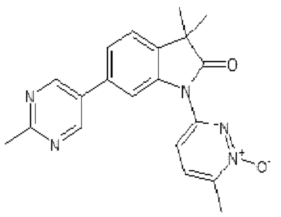 | 0.481 | | | |
| 107 | 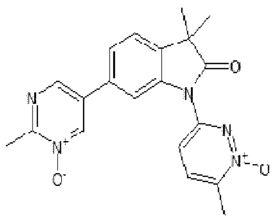 | 2.188 | | | |
| 108 | 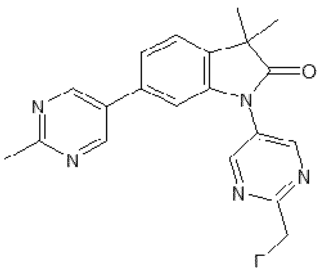 | 1.900 | | | |
| 109 | 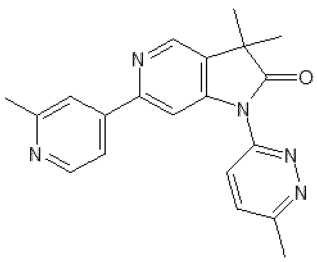 | | 30 | 51.3 | 0.01247 |
| 110 | 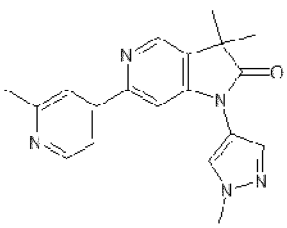 | | 30 | 58.5 | 0.04156 |

(continued)

Effects of compounds of formula I on ENT1 inhibition and on L-687,414-induced hyperlocomotion

| Expl. | structure | ENT1, adenosine uptake, $IC_{50}$ (UM) | L-687,414-induced hyperlocomotion | | |
|---|---|---|---|---|---|
| | | | Dose ip [mg/kg] | Inhibition, ip [%] | P value |
| 111 | 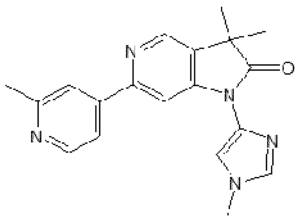 | | 30 | 51 | 0.02613 |
| 113 | 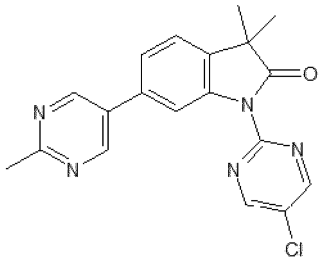 | 0.203 | 30 | 91.1 | 0.00097 |
| 114 | 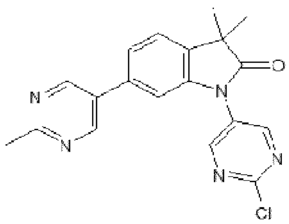 | 0.456 | | | |
| 115 | 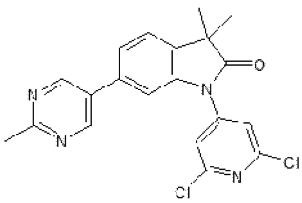 | 0.144 | | | |
| 116 | 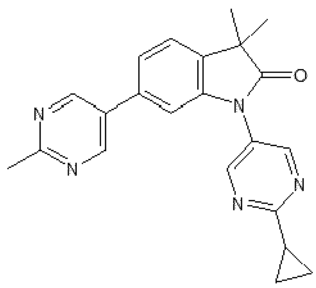 | 0.230 | 30 | 92.8 | 0.00068 |
| 117 | 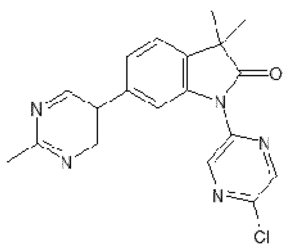 | 0.035 | | | |

(continued)

| Effects of compounds of formula I on ENT1 inhibition and on L-687,414-induced hyperlocomotion | | | | | |
|---|---|---|---|---|---|
| **Expl.** | **structure** | **ENT1, adenosine uptake, $IC_{50}$ (UM)** | **L-687,414-induced hyperlocomotion** | | |
| | | | **Dose ip [mg/kg]** | **Inhibition, ip [%]** | **P value** |
| 119 | 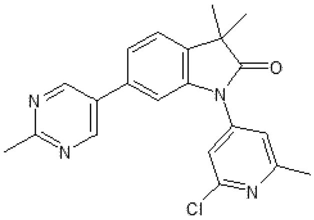 | 0.335 | | | |
| 120 | 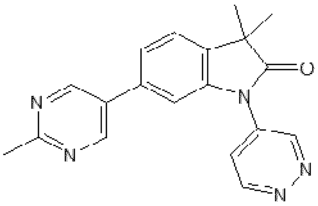 | 3.598 | 30 | 90.8 | 0.00097 |
| 121 | 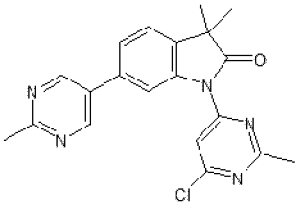 | 0.669 | | | |
| 122 | 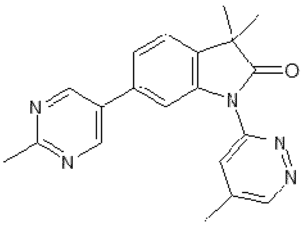 | 0.626 | | | |
| 123 | 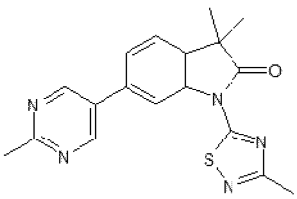 | 0.694 | | | |
| 124 | 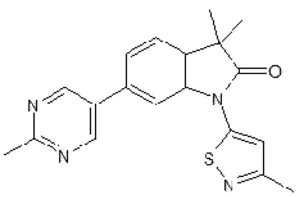 | 0.226 | 30 | 53.3 | 0.00753 |

(continued)

| Effects of compounds of formula I on ENT1 inhibition and on L-687,414-induced hyperlocomotion | | | | | |
|---|---|---|---|---|---|
| Expl. | structure | ENT1, adenosine uptake, $IC_{50}$ (UM) | L-687,414-induced hyperlocomotion | | |
| | | | Dose ip [mg/kg] | Inhibition, ip [%] | P value |
| 125 | 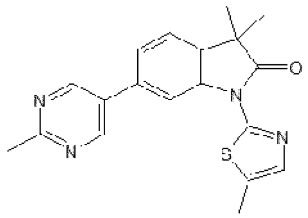 | 0.132 | 30 | 52 | 0.01511 |
| 126 | 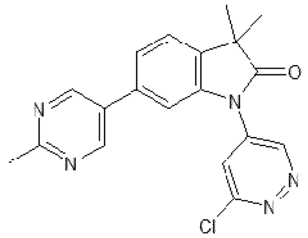 | 2.206 | 30 | 74.7 | 0.00504 |
| 127 | 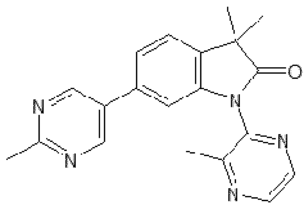 | 7.728 | 30 | 72.8 | 0.00906 |
| 128 | 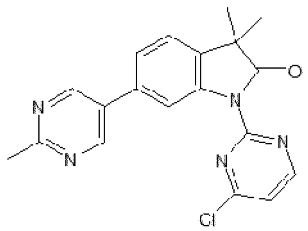 | 0.851 | | | |
| 129 | 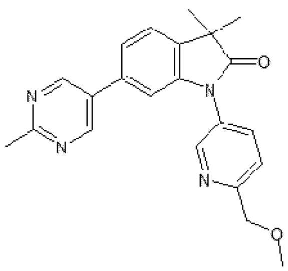 | 0.887 | | | |
| 130 | 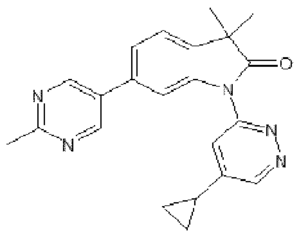 | 2.368 | | | |

(continued)

| Effects of compounds of formula I on ENT1 inhibition and on L-687,414-induced hyperlocomotion | | | | | |
|---|---|---|---|---|---|
| **Expl.** | **structure** | **ENT1, adenosine uptake, $IC_{50}$ (UM)** | **L-687,414-induced hyperlocomotion** | | |
| | | | **Dose ip [mg/kg]** | **Inhibition, ip [%]** | **P value** |
| 132 | 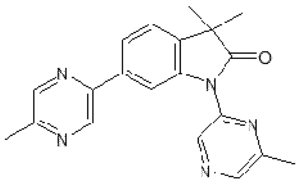 | 2.454 | | | |
| 133 | 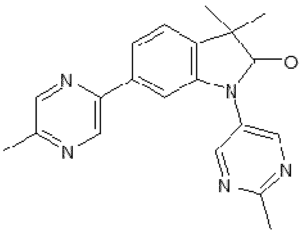 | 4.785 | | | |
| 134 | 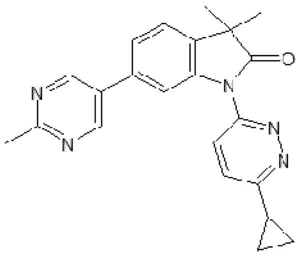 | 0.036 | | | |
| 135 | 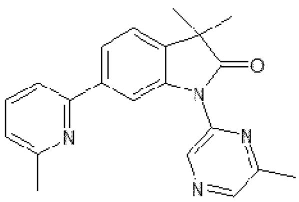 | 2.281 | | | |
| 137 | 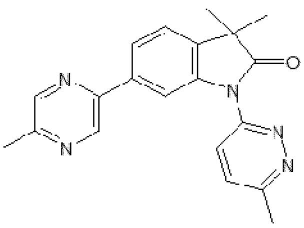 | 4.073 | | | |
| 138 | 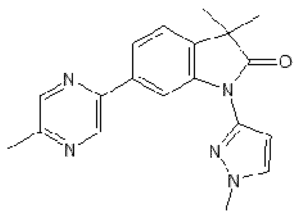 | 5.788 | | | |

(continued)

| Effects of compounds of formula I on ENT1 inhibition and on L-687,414-induced hyperlocomotion | | | | | |
|---|---|---|---|---|---|
| **Expl.** | **structure** | **ENT1, adenosine uptake, $IC_{50}$ (UM)** | **L-687,414-induced hyperlocomotion** | | |
| | | | **Dose ip [mg/kg]** | **Inhibition, ip [%]** | **P value** |
| 139 | 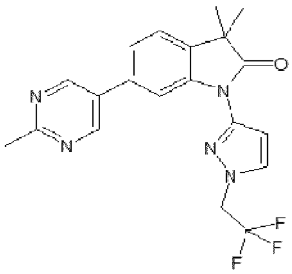 | 0.140 | | | |
| 141 | 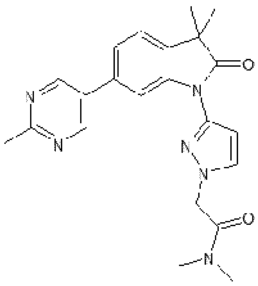 | 1.115 | | | |
| 142 | 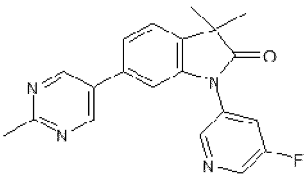 | 0.765 | | | |
| 143 | 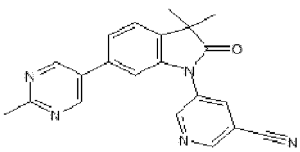 | 0.070 | | | |
| 144 | 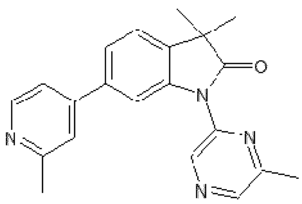 | 3.509 | | | |
| 145 | 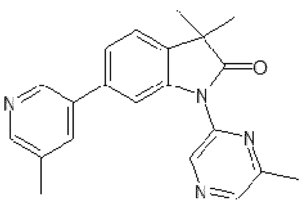 | 0.337 | | | |

(continued)

| Effects of compounds of formula I on ENT1 inhibition and on L-687,414-induced hyperlocomotion | | | | | |
|---|---|---|---|---|---|
| Expl. | structure | ENT1, adenosine uptake, $IC_{50}$ (UM) | L-687,414-induced hyperlocomotion | | |
| | | | Dose ip [mg/kg] | Inhibition, ip [%] | P value |
| 146 | 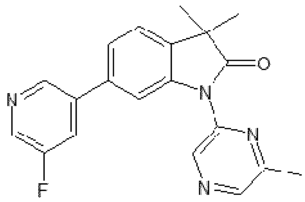 | 2.133 | | | |
| 147 | 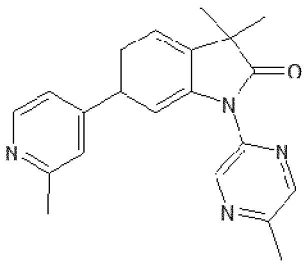 | 9.003 | | | |
| 148 | 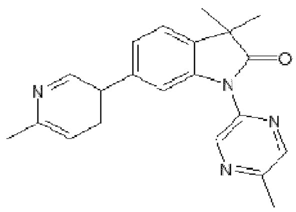 | 0.402 | | | |
| 149 | 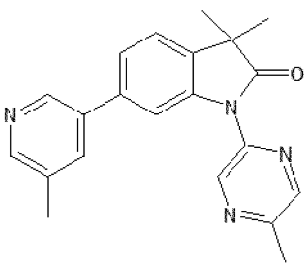 | 0.207 | | | |
| 150 | 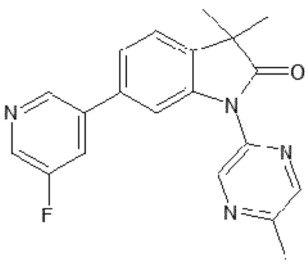 | 1.484 | | | |
| 151 | 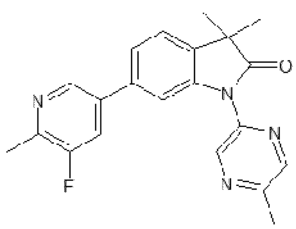 | 0.037 | | | |

(continued)

| Effects of compounds of formula I on ENT1 inhibition and on L-687,414-induced hyperlocomotion | | | | | |
|---|---|---|---|---|---|
| Expl. | structure | ENT1, adenosine uptake, $IC_{50}$ (UM) | L-687,414-induced hyperlocomotion | | |
| | | | Dose ip [mg/kg] | Inhibition, ip [%] | P value |
| 152 | 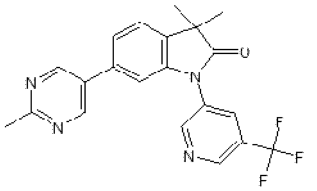 | 0.317 | | | |
| 153 | 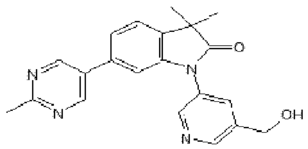 | 0.291 | | | |
| 154 | 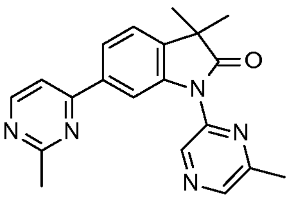 | 4.47 | | | |
| 156 | 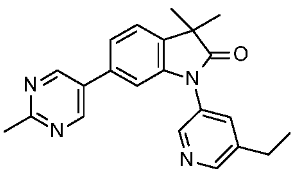 | 0.30 | | | |

As mentioned above, some compounds have been tested in SmartCube®, an analytical system developed by PsychoGenics Inc.

SmartCube® was used to compare the behavioral signature of a test compound to a database of behavioral signatures obtained from a large set of clinically approved reference drugs, grouped per indications. In this way, the neuro-pharmacological effects of a test compound can be predicted by similarity to major classes of compounds, such as antipsychotics, anxiolytics and antidepressants. This approach is ideally suited to screen collections of existing drugs or drug candidates with previously unknown neuropharmacology, which could expedite the development of new and unexpected treatments for psychiatric disorders.

Some compounds of the present invention were injected i.p. at different doses 15 minutes before the test. At least 8 mice were used in each treatment group. Digital videos of the subjects were processed with computer vision algorithms to extract over 2000 dependent measures including frequency and duration of many different behavioral states. The results of the classifications are presented as bar charts for each compound and dose (mg/kg), the Y-axis indicates the relative probability that the test compound will show efficacy in the specific CNS indication.

The bar charts of example compounds 9, 25, 48 and 53 at a dose of 5 mg/kg are shown in Figure 1. For comparison, the behavioral signatures of the atypical antipsychotics clozapine, olanzapine and risperidone are shown in Figure 2. Compounds of the present invention show similar signatures to those of atypical antipsychotics. An independent analysis was performed on the unclassified data to determine the similarity of the example compounds to active doses of known atypical antipsychotics. For this analysis, we use discrimination rate as the measure of separability between the two drugs, i.e. one drug's "distinguishability" from another. A rate equal to 50% (or 0.5) corresponds to zero distinguishability. Empirical data has shown that a threshold rate for reliable separation lies above 70% i.e., two drugs showing a discrimination rate of 70% or lower are considered similar, whereas a discrimination rate higher than 70% indicates that two drugs are dissimilar. The table below shows the similarity analysis of selected compounds of the present invention to several atypical antipsychotics. In most cases, the example compounds show a similarity to risperidone, clozapine and olanzapine with a discrimination rate of ≤ 0.70.

Table 2: Similarity analysis of compounds of formula I (at 5 mg/kg) showing effects in SmartCube®

| | Clozapine | Olanzapine | Risperidone |
|---|---|---|---|
| Example 9 | **0.65** | **0.66** | **0.69** |
| Example 25 | **0.61** | **0.62** | 0.74 |
| Example 48 | **0.58** | **0.61** | **0.67** |
| Example 53 | **0.59** | **0.61** | **0.62** |

Therefore, it can be assumed that the present compounds have similar efficacies as known atypical antipsychotics.

Figure 1: SmartCube® signatures of compounds 9, 25, 49 and 53 (at 5 mg/kg) are similar to those of atypical antipsychotics.

Figure 2: SmartCube® signatures of atypical antipsychotics clozapine, olanzapine and risperidone at various doses.

The compounds of formula (I) and pharmaceutically acceptable salts thereof can be used as medicaments, e.g. in the form of pharmaceutical preparations. The pharmaceutical preparations can be administered orally, e.g. in the form of tablets, coated tablets, dragées, hard and soft gelatine capsules, solutions, emulsions or suspensions. However, the administration can also be effected rectally, e.g. in the form of suppositories, or parenterally, e.g. in the form of injection solutions.

The compounds of formula (I) and pharmaceutically acceptable salts thereof can be processed with pharmaceutically inert, inorganic or organic carriers for the production of pharmaceutical preparations. Lactose, corn starch or derivatives thereof, talc, stearic acid or its salts and the like can be used, for example, as such carriers for tablets, coated tablets, dragées and hard gelatine capsules. Suitable carriers for soft gelatine capsules are, for example, vegetable oils, waxes, fats, semi-solid and liquid polyols and the like; depending on the nature of the active substance no carriers are, however, usually required in the case of soft gelatine capsules. Suitable carriers for the production of solutions and syrups are, for example, water, polyols, sucrose, invert sugar, glucose and the like. Adjuvants, such as alcohols, polyols, glycerol, vegetable oils and the like, can be used for aqueous injection solutions of water-soluble salts of compounds of formula (I), but as a rule are not necessary. Suitable carriers for suppositories are, for example, natural or hardened oils, waxes, fats, semi-liquid or liquid polyols and the like.

In addition, the pharmaceutical preparations can contain preservatives, solubilizers, stabilizers, wetting agents, emulsifiers, sweeteners, colorants, flavorants, salts for varying the osmotic pressure, buffers, masking agents or antioxidants. They can also contain still other therapeutically valuable substances.

As mentioned earlier, medicaments containing a compound of formula (I) or pharmaceutically acceptable salts thereof and a therapeutically inert excipient are also an object of the present invention, as is a process for the production of such medicaments which comprises bringing one or more compounds of formula I or pharmaceutically acceptable salts thereof and, if desired, one or more other therapeutically valuable substances into a galenical dosage form together with one or more therapeutically inert carriers. The active compounds may also be used in form of their prodrugs.

As further mentioned earlier, the use of the compounds of formula (I) for the preparation of medicaments useful in the prevention and/or the treatment of the above recited diseases is also an object of the present invention.

The dosage can vary within wide limits and will, of course, be fitted to the individual requirements in each particular case. In general, the effective dosage for oral or parenteral administration is between 0.01-20 mg/kg/day, with a dosage of 0.1-10 mg/kg/day being preferred for all of the indications described. The daily dosage for an adult person weighing 70 kg accordingly lies between 0.7-1400 mg per day, preferably between 7 and 700 mg per day.

**Preparation of pharmaceutical compositions comprising compounds of the invention:**

Tablets of the following composition are manufactured in the usual manner:

| **ingredient** | **mg/tablet** | | | |
|---|---|---|---|---|
| | 5 | 25 | 100 | 500 |
| Compound of formula I | 5 | 25 | 100 | 500 |
| Lactose Anhydrous DTG | 125 | 105 | 30 | 150 |

(continued)

| ingredient | mg/tablet | | | |
|---|---|---|---|---|
| | 5 | 25 | 100 | 500 |
| Sta-Rx 1500 | 6 | 6 | 6 | 60 |
| Microcrystalline Cellulose | 30 | 30 | 30 | 450 |
| Magnesium Stearate | 1 | 1 | 1 | 1 |
| Total | 167 | 167 | 167 | 831 |

Manufacturing Procedure

1. Mix ingredients 1, 2, 3 and 4 and granulate with purified water.
2. Dry the granules at 50°C.
3. Pass the granules through suitable milling equipment.
4. Add ingredient 5 and mix for three minutes; compress on a suitable press.

Capsules of the following composition are manufactured:

| ingredient | mg/capsule | | | |
|---|---|---|---|---|
| | 5 | 25 | 100 | 500 |
| Compound of formula I | 5 | 25 | 100 | 500 |
| Hydrous Lactose | 159 | 123 | 148 | - |
| Corn Starch | 25 | 35 | 40 | 70 |
| Talk | 10 | 15 | 10 | 25 |
| Magnesium Stearate | 1 | 2 | 2 | 5 |
| Total | 200 | 200 | 300 | 600 |

Manufacturing Procedure

1. Mix ingredients 1, 2 and 3 in a suitable mixer for 30 minutes.

2. Add ingredients 4 and 5 and mix for 3 minutes.

3. Fill into a suitable capsule.

A compound of formula I lactose and corn starch are firstly mixed in a mixer and then in a comminuting machine. The mixture is returned to the mixer; the talc is added thereto and mixed thoroughly. The mixture is filled by machine into suitable capsules, e.g. hard gelatin capsules.

Injection solutions of the following composition are manufactured:

| ingredient | mg/injection solution. |
|---|---|
| Compound of formula I | 3 |
| Polyethylene Glycol 400 | 150 |
| acetic acid | q.s. ad pH 5.0 |
| water for injection solutions | ad 1.0 ml |

Manufacturing Procedure

A compound of formula I is dissolved in a mixture of Polyethylene Glycol 400 and water for injection (part). The pH is adjusted to 5.0 by acetic acid. The volume is adjusted to 1.0 ml by addition of the residual amount of water. The solution is filtered, filled into vials using an appropriate overage and sterilized.

## Claims

1. A compound of formula

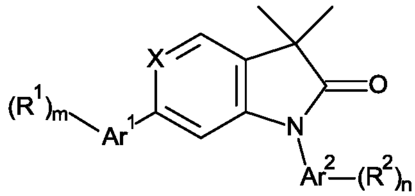

I

wherein

$Ar^1$ is phenyl or a five or six membered heteroaryl group, containing one, two or three heteroatoms, selected from N, S or O, wherein the N-heteroatom in the heteroaryl group may be oxidized to $N^+$-($O^-$);
$R^1$ is $C_{1-7}$-alkyl, halogen, cyano or cycloalkyl;
$Ar^2$ is a five or six membered heteroaryl group, containing one, two, three or four heteroatoms, selected from N, S or O, wherein the N-heteroatom in the heteroaryl group may be oxidized to $N^+$-($O^-$), or is benzo[b]thiophenyl;
$R^2$ is hydrogen, $C_{1-7}$-alkyl, halogen, cyano, $C_{1-7}$-substituted by hydroxyl, $C_{1-7}$-alkyl substituted by halogen, $C_{1-7}$-alkyl substituted by amino, $C_{1-7}$-alkyl substituted by $C_{1-7}$-alkoxy, $C_{1-7}$-alkyl substituted by amide, or is cycloalkyl;
X is CH or N;
n is 1 or 2;
m is 1 or 2;

as well as with a pharmaceutically acceptable salt thereof, with a racemic mixture, or with its corresponding enantiomer and/or optical isomer and/or stereoisomer thereof.

2. A compound of formula I according to claim 1, wherein X is CH.

3. A compound of formula I according to claim 2, wherein $Ar^1$ and $Ar^2$ are both a six membered heteroaryl group, containing one, two or three heteroatoms, selected from N, S or O.

4. A compound of formula I according to claim 3, wherein the compounds are

3,3-Dimethyl-6-(2-methylpyrimidin-5-yl)-1-(pyridin-4-yl)indolin-2-one
3,3-Dimethyl-6-(2-methylpyrimidin-5-yl)-1-(pyridin-3-yl)indolin-2-one
3,3-Dimethyl-1-(2-methylpyridin-4-yl)-6-(2-methylpyrimidin-5-yl)indolin-2-one
3,3-Dimethyl-1-(6-methylpyrimidin-4-yl)-6-(2-methylpyrimidin-5-yl)indolin-2-one
3,3-Dimethyl-6-(2-methylpyrimidin-5-yl)-1-(pyrimidin-5-yl)indolin-2-one
3,3-Dimethyl-1,6-bis(2-methylpyrimidin-5-yl)indolin-2-one
3,3-Dimethyl-1-(6-methylpyridin-3-yl)-6-(2-methylpyrimidin-5-yl)indolin-2-one
3,3-Dimethyl-6-(6-methylpyridin-3-yl)-1-(2-methylpyridin-4-yl)indolin-2-one
3,3-Dimethyl-1,6-bis(2-methylpyridin-4-yl)indolin-2-one
6-(4-Fluoropyridin-3-yl)-3,3-dimethyl-1-(2-methylpyridin-4-yl)indolin-2-one
1-(5-Fluoro-2-methylpyridin-4-yl)-3,3-dimethyl-6-(2-methylpyrimidin-5-yl)indolin-2-one
5-(3,3-Dimethyl-6-(2-methylpyrimidin-5-yl)-2-oxoindolin-1-yl)picolinonitrile
1-(6-(Hydroxymethyl)pyridin-3-yl)-3,3-dimethyl-6-(2-methylpyrimidin-5-yl)indolin-2-one
1-(6-Cyclopropylpyridin-3-yl)-3,3-dimethyl-6-(2-methylpyrimidin-5-yl)indolin-2-one

3,3-Dimethyl-6-(2-methylpyrimidin-5-yl)-1-(pyridin-2-yl)indolin-2-one
1-(2-Fluoropyridin-4-yl)-3,3-dimethyl-6-(2-methylpyrimidin-5-yl)indolin-2-one
1-(3-Fluoropyridin-4-yl)-3,3-dimethyl-6-(2-methylpyrimidin-5-yl)indolin-2-one
1-(2-Fluoro-5-methylpyridin-4-yl)-3,3-dimethyl-6-(2-methylpyrimidin-5-yl)indolin-2-one
3,3-Dimethyl-1-(6-methylpyridazin-3-yl)-6-(2-methylpyrimidin-5-yl)indolin-2-one
1-(3-Chloropyridin-4-yl)-3,3-dimethyl-6-(2-methylpyrimidin-5-yl)indolin-2-one
3,3-Dimethyl-1-(5-methylpyrimidin-2-yl)-6-(2-methylpyrimidin-5-yl)indolin-2-one
3,3-Dimethyl-1-(5-methylpyridin-2-yl)-6-(2-methylpyrimidin-5-yl)indolin-2-one
3,3-Dimethyl-1-(5-methylpyrazin-2-yl)-6-(2-methylpyrimidin-5-yl)indolin-2-one
3,3-Dimethyl-1-(5-methylpyridin-3-yl)-6-(2-methylpyrimidin-5-yl)indolin-2-one
3,3-Dimethyl-1-(4-methylpyridin-2-yl)-6-(2-methylpyrimidin-5-yl)indolin-2-one
3,3-Dimethyl-1-(6-methylpyridin-2-yl)-6-(2-methylpyrimidin-5-yl)indolin-2-one
3,3-Dimethyl-1-(2-methylpyrimidin-4-yl)-6-(2-methylpyrimidin-5-yl)indolin-2-one
3,3-Dimethyl-1-(6-methylpyrazin-2-yl)-6-(2-methylpyrimidin-5-yl)indolin-2-one
3,3-Dimethyl-1-(4-methylpyrimidin-2-yl)-6-(2-methylpyrimidin-5-yl)indolin-2-one
1-(2,6-Dimethylpyridin-4-yl)-3,3-dimethyl-6-(2-methylpyrimidin-5-yl)indolin-2-one
-(4,6-Dimethylpyrimidin-2-yl)-3,3-dimethyl-6-(2-methylpyrimidin-5-yl)indolin-2-one
1-(2,6-Dimethylpyrimidin-4-yl)-3,3-dimethyl-6-(2-methylpyrimidin-5-yl)indolin-2-one
1-(4,5-Dimethylpyridin-2-yl)-3,3-dimethyl-6-(2-methylpyrimidin-5-yl)indolin-2-one
1-(5,6-dimethylpyridin-2-yl)-3,3-dimethyl-6-(2-methylpyrimidin-5-yl)indolin-2-one
1-(5,6-dimethylpyridin-3-yl)-3,3-dimethyl-6-(2-methylpyrimidin-5-yl)indolin-2-one
3,3-dimethyl-6-(2-methylpyrimidin-5-yl)-1-(pyridazin-3-yl)indolin-2-one
3,3-dimethyl-6-(2-methylpyrimidin-5-yl)-1-(pyrazin-2-yl)indolin-2-one
3,3-dimethyl-6-(2-methylpyrimidin-5-yl)-1-(pyrimidin-2-yl)indolin-2-one
3,3-dimethyl-6-(2-methylpyrimidin-5-yl)-1-(4-methylpyrimidin-5-yl)indolin-2-one
3,3-dimethyl-1,6-bis(5-methylpyrimidin-2-yl)indolin-2-one
3,3-dimethyl-1,6-bis(5-methylpyrazin-2-yl)indolin-2-one
3,3-dimethyl-6-(2-methylpyrimidin-5-yl)-1-(pyrimidin-4-yl)indolin-2-one
1-(5-cyclopropylpyrazin-2-yl)-3,3-dimethyl-6-(2-methylpyrimidin-5-yl)indolin-2-one
5-(3,3-dimethyl-6-(2-methylpyrimidin-5-yl)-2-oxoindolin-1-yl)pyrazine-2-carbonitrile
1-(6-cyclopropylpyrazin-2-yl)-3,3-dimethyl-6-(2-methylpyrimidin-5-yl)indolin-2-one
1-(4,5-dimethylpyridin-3-yl)-3,3-dimethyl-6-(2-methylpyrimidin-5-yl)indolin-2-one
1-(4,5-dimethylpyridin-3-yl)-3,3-dimethyl-6-(2-methylpyrimidin-5-yl)indolin-2-one
1-(4,6-dimethylpyridin-3-yl)-3,3-dimethyl-6-(2-methylpyrimidin-5-yl)indolin-2-one
5-(3,3-dimethyl-1-(5-methylpyrimidin-2-yl)-2-oxoindolin-6-yl)-2-methylpyrimidine 1-oxide
1-(2-(hydroxymethyl)pyrimidin-5-yl)-3,3-dimethyl-6-(2-methylpyrimidin-5-yl)indolin-2-one
1-[2-(aminomethyl)pyrimidin-5-yl]-3,3-dimethyl-6-(2-methylpyrimidin-5-yl)indol-2-one
3-(3,3-dimethyl-6-(2-methylpyrimidin-5-yl)-2-oxoindolin-1-yl)-6-methylpyridazine 1-oxide
3-(3,3-dimethyl-6-(2-methyl-1-oxidopyrimidin-5-yl)-2-oxoindolin-1-yl)-6-methylpyridazine 1-oxide
1-(2-(fluoromethyl)pyrimidin-5-yl)-3,3-dimethyl-6-(2-methylpyrimidin-5-yl)indolin-2-one
3,3-dimethyl-1-(6-methylpyridazin-3-yl)-6-(2-methylpyridin-4-yl)-1H-pyrrolo[3,2-c]pyridin-2(3H)-one
6-(4-fluorophenyl)-3,3-dimethyl-1-(2-methylpyrimidin-5-yl)-1H-pyrrolo[3,2-c]pyridin-2(3H)-one
1-(5-chloropyrimidin-2-yl)-3,3-dimethyl-6-(2-methylpyrimidin-5-yl)indolin-2-one
1-(2-chloropyrimidin-5-yl)-3,3-dimethyl-6-(2-methylpyrimidin-5-yl)indolin-2-one
1-(2,6-dichloropyridin-4-yl)-3,3-dimethyl-6-(2-methylpyrimidin-5-yl)indolin-2-one
1-(2-cyclopropylpyrimidin-5-yl)-3,3-dimethyl-6-(2-methylpyrimidin-5-yl)indolin-2-one
1-(5-chloropyrazin-2-yl)-3,3-dimethyl-6-(2-methylpyrimidin-5-yl)indol-2-one
1-(6-chloropyridazin-3-yl)-3,3-dimethyl-6-(2-methylpyrimidin-5-yl)indolin-2-one
1-(2-chloro-6-methylpyridin-4-yl)-3,3-dimethyl-6-(2-methylpyrimidin-5-yl)indolin-2-one
3,3-dimethyl-6-(2-methylpyrimidin-5-yl)-1-(pyridazin-4-yl)indolin-2-one
1-(6-chloro-2-methylpyrimidin-4-yl)-3,3-dimethyl-6-(2-methylpyrimidin-5-yl)indolin-2-one
3,3-dimethyl-1-(5-methylpyridazin-3-yl)-6-(2-methylpyrimidin-5-yl)indolin-2-one
1-(6-chloropyridazin-4-yl)-3,3-dimethyl-6-(2-methylpyrimidin-5-yl)indolin-2-one
3,3-dimethyl-1-(3-methylpyrazin-2-yl)-6-(2-methylpyrimidin-5-yl)indolin-2-one
1-(4-chloropyrimidin-2-yl)-3,3-dimethyl-6-(2-methylpyrimidin-5-yl)indolin-2-one
1-(6-(methoxymethyl)pyridin-3-yl)-3,3-dimethyl-6-(2-methylpyrimidin-5-yl)indolin-2-one
1-(5-cyclopropylpyridazin-3-yl)-3,3-dimethyl-6-(2-methylpyrimidin-5-yl)indolin-2-one
3,3-dimethyl-6-(5-methylpyrazin-2-yl)-1-(6-methylpyrazin-2-yl)indolin-2-one

3,3-dimethyl-6-(5-methylpyrazin-2-yl)-1-(2-methylpyrimidin-5-yl)indolin-2-one
1-(6-cyclopropylpyridazin-3-yl)-3,3-dimethyl-6-(2-methylpyrimidin-5-yl)indolin-2-one
3,3-dimethyl-1-(6-methylpyrazin-2-yl)-6-(6-methyl-2-pyridyl)indolin-2-one
3,3-dimethyl-1-(5-methylpyrazin-2-yl)-6-(2-methylpyrimidin-4-yl)indolin-2-one
3,3-dimethyl-6-(5-methylpyrazin-2-yl)-1-(6-methylpyridazin-3-yl)indolin-2-one
1-(5-fluoropyridin-3-yl)-3,3-dimethyl-6-(2-methylpyrimidin-5-yl)indolin-2-one
5-(3,3-dimethyl-6-(2-methylpyrimidin-5-yl)-2-oxoindolin-1-yl)nicotinonitrile
3,3-dimethyl-1-(6-methylpyrazin-2-yl)-6-(2-methyl-4-pyridyl)indolin-2-one
3,3-dimethyl-1-(6-methylpyrazin-2-yl)-6-(5-methyl-3-pyridyl)indolin-2-one
6-(5-fluoro-3-pyridyl)-3,3-dimethyl-1-(6-methylpyrazin-2-yl)indolin-2-one
3,3-dimethyl-1-(5-methylpyrazin-2-yl)-6-(2-methyl-4-pyridyl)indolin-2-one
3,3-dimethyl-1-(5-methylpyrazin-2-yl)-6-(6-methyl-3-pyridyl)indolin-2-one
3,3-dimethyl-1-(5-methylpyrazin-2-yl)-6-(5-methyl-3-pyridyl)indolin-2-one
6-(5-fluoro-3-pyridyl)-3,3-dimethyl-1-(5-methylpyrazin-2-yl)indolin-2-one
6-(5-fluoro-6-methyl-3-pyridyl)-3,3-dimethyl-1-(5-methylpyrazin-2-yl)indolin-2-one
3,3-dimethyl-6-(2-methylpyrimidin-5-yl)-1-(5-(trifluoromethyl)pyridin-3-yl)indolin-2-one
1-(5-(hydroxymethyl)pyridin-3-yl)-3,3-dimethyl-6-(2-methylpyrimidin-5-yl)indolin-2-one
3,3-dimethyl-1-(6-methylpyrazin-2-yl)-6-(2-methylpyrimidin-4-yl)indolin-2-one
5-[3,3-dimethyl-1-(6-methylpyrazin-2-yl)-2-oxo-indolin-6-yl]pyrimidine-2-carbonitrile or
1-(5-ethylpyridin-3-yl)-3,3-dimethyl-6-(2-methylpyrimidin-5-yl)indolin-2-one.

**5.** A compound of formula I according to claim 2, wherein $Ar^1$ is a six membered heteroaryl group, containing one, two or three heteroatoms, selected from N, S or O, and $Ar^2$ is five membered heteroaryl group, containing one, two or three heteroatoms, selected from N, S or O.

**6.** A compound of formula I according to claim 5, wherein the compounds are

3,3-Dimethyl-1-(1-methyl-1H-imidazol-4-yl)-6-(2-methylpyrimidin-5-yl)indolin-2-one
3,3-Dimethyl-1-(1-methyl-1H-pyrazol-3-yl)-6-(2-methylpyrimidin-5-yl)indolin-2-one
1-(1,5-Dimethyl-1H-pyrazol-3-yl)-3,3-dimethyl-6-(2-methylpyrimidin-5-yl)indolin-2-one
3,3-Dimethyl-1-(1-methyl-1H-imidazol-4-yl)-6-(6-methylpyridin-3-yl)indolin-2-one
3,3-Dimethyl-1-(1-methyl-1-methyl-1H-imidazol-4-yl)-6-(2-methylpyridin-4-yl)indolin-2-one
3,3-Dimethyl-1-(1-methyl-1H-pyrazol-3-yl)-6-(6-methylpyridin-3-yl)indolin-2-one
6-(4-Fluoropyridin-3-yl)-3,3-dimethyl-1-(1-methyl-1H-imidazol-4-yl)indolin-2-one
6-(4-Fluoropyridin-3-yl)-3,3-dimethyl-1-(1-methyl-1H-pyrazol-3-yl)indolin-2-one
3,3-Dimethyl-6-(2-methylpyrimidin-5-yl)-1-(5-methylthiophen-2-yl)indolin-2-one
3,3-Dimethyl-1-(1-methyl-1H-imidazol-5-yl)-6-(2-methylpyrimidin-5-yl)indolin-2-one
3,3-Dimethyl-1-(5-methyl-1,3,4-oxadiazol-2-yl)-6-(2-methylpyrimidin-5-yl)indolin-2-one
3,3-Dimethyl-6-(2-methylpyrimidin-5-yl)-1-(1H-pyrazol-3-yl)indolin-2-one
1-(1,2-Dimethyl-1H-imidazol-4-yl)-3,3-dimethyl-6-(2-methylpyrimidin-5-yl)indolin-2-one
1-(5-ethylpyridin-3-yl)-3,3-dimethyl-6-(2-methylpyrimidin-5-yl)indolin-2-one
1-(1,5-dimethyl-1H-1,2,4-triazol-3-yl)-3,3-dimethyl-6-(2-methylpyrimidin-5-yl)indolin-2-one
3,3-dimethyl-1-(1-methyl-1H-pyrazol-4-yl)-6-(2-methylpyrimidin-5-yl)indolin-2-one
3,3-dimethyl-1-(2-methyl-1H-imidazol-4-yl)-6-(2-methylpyrimidin-5-yl)indolin-2-one
3,3-dimethyl-1-(1-methyl-1H-1,2,4-triazol-3-yl)-6-(2-methylpyrimidin-5-yl)indolin-2-one
3,3-dimethyl-6-(2-methylpyrimidin-5-yl)-1-(1H-pyrazol-4-yl)indolin-2-one
1-(1H-imidazol-4-yl)-3,3-dimethyl-6-(2-methylpyrimidin-5-yl)indolin-2-one
3,3-dimethyl-1-(3-methyl-1H-pyrazol-5-yl)-6-(2-methylpyrimidin-5-yl)indolin-2-one
3,3-dimethyl-1-(1-methyl-1H-1,2,3-triazol-4-yl)-6-(2-methylpyrimidin-5-yl)indolin-2-one
3,3-dimethyl-1-(4-methyl-1H-imidazol-2-yl)-6-(2-methylpyrimidin-5-yl)indolin-2-one
3,3-dimethyl-1-(3-methylisoxazol-5-yl)-6-(2-methylpyrimidin-5-yl)indolin-2-one
1-(1H-imidazol-2-yl)-3,3-dimethyl-6-(2-methylpyrimidin-5-yl)indolin-2-one
1-(1-ethyl-1H-pyrazol-3-yl)-3,3-dimethyl-6-(2-methylpyrimidin-5-yl)indolin-2-one
3,3-dimethyl-6-(2-methylpyrimidin-5-yl)-1-(2H-tetrazol-5-yl)indolin-2-one
3,3-dimethyl-1-(2-methyl-2H-tetrazol-5-yl)-6-(2-methylpyrimidin-5-yl)indolin-2-one
3,3-dimethyl-1-(1-methyl-1H-pyrazol-4-yl)-6-(2-methylpyridin-4-yl)-1H-pyrrolo[3,2-c]pyridin-2(3H)-one
3,3-dimethyl-1-(1-methyl-1H-imidazol-4-yl)-6-(2-methylpyridin-4-yl)-1H-pyrrolo[3,2-c]pyridin-2(3H)-one
3,3-dimethyl-1-(3-methyl-1,2,4-thiadiazol-5-yl)-6-(2-methylpyrimidin-5-yl)indolin-2-one

3,3-dimethyl-1-(3-methylisothiazol-5-yl)-6-(2-methylpyrimidin-5-yl)indolin-2-one
3,3-dimethyl-6-(2-methylpyrimidin-5-yl)-1-(5-methylthiazol-2-yl)indolin-2-one
1-(1-isopropyl-1H-pyrazol-3-yl)-3,3-dimethyl-6-(2-methylpyrimidin-5-yl)indolin-2-one
3,3-dimethyl-1-(1-methyl-1H-pyrazol-3-yl)-6-(5-methylpyrazin-2-yl)indolin-2-one
3,3-dimethyl-6-(2-methylpyrimidin-5-yl)-1-(1-(2,2,2-trifluoroethyl)-1H-pyrazol-3-yl)indolin-2-one
1-(1-(2-methoxyethyl)-1H-pyrazol-3-yl)-3,3-dimethyl-6-(2-methylpyrimidin-5-yl)indolin-2-one or
2-(3-(3,3-dimethyl-6-(2-methylpyrimidin-5-yl)-2-oxoindolin-1-yl)-1H-pyrazol-1-yl)-N,N-dimethylacetamide.

**7.** A compound of formula I according to claim 2, wherein $Ar^1$ is a five membered heteroaryl group, containing one, two or three heteroatoms, selected from N, S or O, and $Ar^2$ is a six membered heteroaryl group, containing one, two or three heteroatoms, selected from N, S or O.

**8.** A compound of formula I according to claim 7, wherein the compounds are

3,3-Dimethyl-6-(4-methylimidazol-1-yl)-1-(6-methyl-3-pyridyl)indolin-2-one
6-(4-Cyclopropylimidazol-1-yl)-3,3-dimethyl-1-(2-methyl-4-pyridyl)indolin-2-one
3,3-Dimethyl-6-(4-methylimidazol-1-yl)-1-(2-methyl-4-pyridyl)indolin-2-one
3,3-Dimethyl-6-(1-methyl-1H-pyrazol-3-yl)-1-(2-methylpyrimidin-5-yl)indolin-2-one
3,3-Dimethyl-6-(1-methyl-1H-imidazol-4-yl)-1-(2-methylpyrimidin-5-yl)indolin-2-one
6-(4-Isopropylimidazol-1-yl)-3,3-dimethyl-1-(6-methyl-3-pyridyl)indolin-2-one
3,3-Dimethyl-6-(5-methyl-1,3,4-oxadiazol-2-yl)-1-(6-methyl-3-pyridyl)indolin-2-one
3,3-Dimethyl-6-(5-methyl-1,3,4-oxadiazol-2-yl)-1-(2-methyl-4-pyridyl)indolin-2-one
6-(4-Isopropylimidazol-1-yl)-3,3-dimethyl-1-(2-methyl-4-pyridyl)indolin-2-one
6-(4-Isopropylimidazol-1-yl)-3,3-dimethyl-1-(2-methylpyrimidin-5-yl)indolin-2-one
6-(4-Cyclopropylimidazol-1-yl)-3,3-dimethyl-1-(2-methylpyrimidin-5-yl)indolin-2-one
3,3-Dimethyl-6-(5-methyl-1,3,4-oxadiazol-2-yl)-1-(2-methylpyrimidin-5-yl)indolin-2-one
3,3-Dimethyl-6-(4-methylimidazol-1-yl)-1-(2-methylpyrimidin-5-yl)indolin-2-one
3,3-dimethyl-6-(2-methyloxazol-5-yl)-1-(2-methylpyrimidin-5-yl)indolin-2-one or
3,3-dimethyl-6-(3-methyl-1,2,4-oxadiazol-5-yl)-1-(2-methylpyrimidin-5-yl)indolin-2-one.

**9.** A compound of formula I according to claim 2, wherein $Ar^1$ and $Ar^2$ are both a five membered heteroaryl group, containing one, two or three heteroatoms, selected from N, S or O.

**10.** A compound of formula I according to claim 9, wherein the compounds are

6-(4-Isopropylimidazol-1-yl)-3,3-dimethyl-1-(1-methylpyrazol-3-yl)indolin-2-one
6-(4-Cyclopropylimidazol-1-yl)-3,3-dimethyl-1-(1-methylimidazol-4-yl)indolin-2-one
6-(4-Cyclopropylimidazol-1-yl)-3,3-dimethyl-1-(1-methylpyrazol-3-yl)indolin-2-one
3,3-Dimethyl-1-(1-methylimidazol-4-yl)-6-(2-methyloxazol-5-yl)indolin-2-one
3,3-Dimethyl-6-(2-methyloxazol-5-yl)-1-(1-methylpyrazol-3-yl)indolin-2-one
3,3-Dimethyl-1-(1-methylimidazol-4-yl)-6-(5-methyl-1,3,4-oxadiazol-2-yl)indolin-2-one or
3,3-dimethyl-6-(4-methyl-1H-imidazol-1-yl)-1-(1-methyl-1H-imidazol-4-yl)indolin-2-one.

**11.** A compound of formula I according to claim 2, wherein $Ar^2$ is benzo[b]thiophenyl, and the other substituents are as described in claim 1.

**12.** A compound of formula I according to claim 11, wherein the compound is 1-(Benzo[b]thiophen-4-yl)-3,3-dimethyl-6-(2-methylpyrimidin-5-yl)indolin-2-one.

**13.** A compound of formula I according to claim 1, wherein X is N and the other substituents are as described in claim 1.

**14.** A compound of formula I according to claim 13, wherein the compounds are

3,3-Dimethyl-1-(1-methyl-1H-imidazol-4-yl)-6-(2-methylpyrimidin-5-yl)-1H-pyrrolo[3,2-c]pyridin-2(3H)-one
3,3-Dimethyl-1-(1-methyl-1H-pyrazol-3-yl)-6-(2-methylpyrimidin-5-yl)-1H-pyrrolo[3,2-c]pyridin-2(3H)-one
3,3-Dimethyl-1-(2-methylpyridin-4-yl)-6-(2-methylpyrimidin-5-yl)-1H-pyrrolo[3,2-c]pyridin-2(3H)-one
3,3-dimethyl-1-(6-methylpyridazin-3-yl)-6-(2-methylpyridin-4-yl)-1H-pyrrolo[3,2-c]pyridin-2(3H)-one
3,3-dimethyl-1-(1-methyl-1H-pyrazol-4-yl)-6-(2-methylpyridin-4-yl)-1H-pyrrolo[3,2-c]pyridin-2(3H)-one

3,3-dimethyl-1-(1-methyl-1H-imidazol-4-yl)-6-(2-methylpyridin-4-yl)-1H-pyrrolo[3,2-c]pyridin-2(3H)-one or 6-(4-fluorophenyl)-3,3-dimethyl-1-(2-methylpyrimidin-5-yl)-1H-pyrrolo[3,2-c]pyridin-2(3H)-one.

**15.** A combination of a compound of formula I according to any one of claims 1 - 14 together with a known marketed antipsychotic, antidepressant, anxiolytic or mood stabilizer.

**16.** A combination according to claim 15, wherein the marketed antipsychotic drug is olanzapine (Zyprexa), clozapine (Clozaril), risperidone (Risperdal), aripiprazole (Abilify) or ziprasidone.

**17.** A combination according to claim 15, wherein the marketed anti-depressive drug is citalopram (Celexa), escitalopram (Lexapro, Cipralex), paroxetine (Paxil, Seroxat), fluoxetine (Prozac), sertraline (Zoloft, Lustral) duloxetine (Cymbalta), milnacipran (Ixel, Savella), venlafaxine (Effexor), or mirtazapine (Remeron).

**18.** A combination according to claim 15, wherein the marketed anxiolytic drug is alprazolam (Helex, Xanax, Xanor, Onax, Alprox, Restyl, Tafil, Paxal), chlordiazepoxide (Librium, Risolid, Elenium), clonazepam (Rivotril, Klonopin, Iktorivil, Paxam), diazepam (Antenex, Apaurin, Apzepam, Apozepam, Hexalid, Pax, Stesolid, Stedon, Valium, Vival, Valaxona), Estazolam (ProSom), eszopiclone (Lunesta), zaleplon (Sonata, Starnoc), zolpidem (Ambien, Nytamel, Stilnoct, Stilnox, Zoldem, Zolnod), pregabalin (Lyrica) or gabapentin (Fanatrex, Gabarone, Gralise, Neurontin, Nupentin).

**19.** A combination according to claim 15, wherein the marketed mood stabilizer is Carbamazepine (Tegretol), Lamotrigine (Lamictal), Lithium (Eskalith, Lithane, Lithobid), and Valproic Acid (Depakote).

**20.** A process for preparation of a compound of formula I as described in any one of claims 1 to 14
reacting a compound of formula

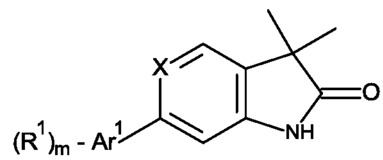

7

with a compound of formula

$(R^2)_n$-$Ar^2$-Y 8

to a compound of formula

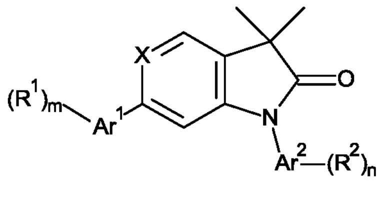

I

wherein Y is Cl, Br or I and the other groups have the meaning as described in claim 1 and,
if desired, converting the compounds obtained into pharmaceutically acceptable acid addition salts.

**21.** A compound according to any one of claims 1 - 14 for use as therapeutically active substance.

**22.** A pharmaceutical composition comprising a compound in accordance with any one of claims 1-14 and a therapeutically active carrier for use in the treatment of CNS diseases related to positive (psychosis) and negative symptoms of schizophrenia, substance abuse, alcohol and drug addiction, obsessive-compulsive disorders, cognitive impairment, bipolar disorders, mood disorders, major depression, treatment resistant depression, anxiety disorders, Alzheimer's disease, autism, Parkinson's disease, chronic pain, borderline personality disorder, neurodegenerative disease, sleep disturbances, chronic fatigue syndrome, stiffness, inflammatory disease, asthma, Huntington's disease,

ADHD, amyotrophic lateral sclerosis, epilepsy, effects in arthritis, autoimmune disease, viral and fungal infections, cardiovascular diseases, ophthalmology and inflammatory retinal diseases and balance problems.

**23.** The use of a compound of formula I as claimed in any one of claims 1-14 for the manufacture of a medicament for the treatment of CNS diseases related to positive (psychosis) and negative symptoms of schizophrenia, substance abuse, alcohol and drug addiction, obsessive-compulsive disorders, cognitive impairment, bipolar disorders, mood disorders, major depression, treatment resistant depression, anxiety disorders, Alzheimer's disease, autism, Parkinson's disease, chronic pain, borderline personality disorder, neurodegenerative disease, sleep disturbances, chronic fatigue syndrome, stiffness, inflammatory disease, asthma, Huntington's disease, ADHD, amyotrophic lateral sclerosis, epilepsy, effects in arthritis, autoimmune disease, viral and fungal infections, cardiovascular diseases, ophthalmology and inflammatory retinal diseases and balance problems.

**24.** A compound according to any one of claims 1 -14 for use in the treatment of CNS diseases related to positive (psychosis) and negative symptoms of schizophrenia, substance abuse, alcohol and drug addiction, obsessive-compulsive disorders, cognitive impairment, bipolar disorders, mood disorders, major depression, treatment resistant depression, anxiety disorders, Alzheimer's disease, autism, Parkinson's disease, chronic pain, borderline personality disorder, neurodegenerative disease, sleep disturbances, chronic fatigue syndrome, stiffness, inflammatory disease, asthma, Huntington's disease, ADHD, amyotrophic lateral sclerosis, epilepsy, effects in arthritis, autoimmune disease, viral and fungal infections, cardiovascular diseases, ophthalmology and inflammatory retinal diseases and balance problems.

**Patentansprüche**

**1.** Verbindung der Formel

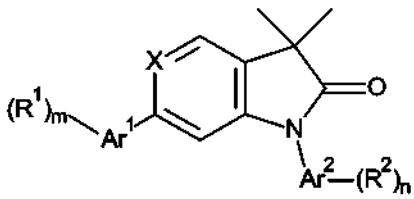

I

wobei

$Ar^1$ für Phenyl oder eine fünf- oder sechsgliedrige Heteroarylgruppe steht, die ein, zwei oder drei Heteroatome enthält, die aus N, S oder O ausgewählt sind, wobei das N-Heteroatom in der Heteroarylgruppe zu $N^+$-($O^-$) oxidiert sein kann;
$R^1$ für $C_{1-7}$-Alkyl, Halogen, Cyano oder Cycloalkyl steht;
$Ar^2$ für eine fünf- oder sechsgliedrige Heteroarylgruppe steht, die ein, zwei, drei oder vier Heteroatome enthält, die aus N, S oder O ausgewählt sind, wobei das N-Heteroatom in der Heteroarylgruppe zu $N^+$-($O^-$) oxidiert sein kann, oder für Benzo[b]thiophenyl steht;
$R^2$ für Wasserstoff, $C_{1-7}$-Alkyl, Halogen, Cyano, $C_{1-7}$- Alkyl substituiert durch Hydroxyl, $C_{1-7}$-Alkyl substituiert durch Halogen, $C_{1-7}$-Alkyl substituiert durch Amino, $C_{1-7}$-Alkyl substituiert durch $C_{1-7}$-Alkoxy, $C_{1-7}$-Alkyl substituiert durch Amid, oder für Cycloalkyl steht;
X für CH oder N steht;
n für 1 oder 2 steht;
m für 1 oder 2 steht;

sowie mit einem pharmazeutisch verträglichen Salz davon, mit einer racemischen Mischung oder mit ihrem entsprechenden Enantiomer und/oder optischen Isomer und/oder Stereoisomer davon.

**2.** Verbindung der Formel I nach Anspruch 1, wobei X für CH steht.

**3.** Verbindung der Formel I nach Anspruch 2, wobei sowohl $Ar^1$ als auch $Ar^2$ für eine sechsgliedrige Heteroarylgruppe steht, die ein, zwei oder drei Heteroatome enthält, die aus N, S oder O ausgewählt sind.

**4.** Verbindung der Formel I nach Anspruch 3, wobei die Verbindungen Folgende sind:

3,3-Dimethyl-6-(2-methylpyrimidin-5-yl)-1-(pyridin-4-yl)indolin-2-on
3,3-Dimethyl-6-(2-methylpyrimidin-5-yl)-1-(pyridin-3-yl)indolin-2-on
3,3-Dimethyl-1-(2-methylpyridin-4-yl)-6-(2-methylpyrimidin-5-yl)indolin-2-on
3,3-Dimethyl-1-(6-methylpyrimidin-4-yl)-6-(2-methylpyrimidin-5-yl)indolin-2-on
3,3-Dimethyl-6-(2-methylpyrimidin-5-yl)-1-(pyrimidin-5-yl)indolin-2-on
3,3-Dimethyl-1,6-bis(2-methylpyrimidin-5-yl)indolin-2-on
3,3-Dimethyl-1-(6-methylpyridin-3-yl)-6-(2-methylpyrimidin-5-yl)indolin-2-on
3,3-Dimethyl-6-(6-methylpyridin-3-yl)-1-(2-methylpyridin-4-yl)indolin-2-on
3,3-Dimethyl-1,6-bis(2-methylpyridin-4-yl)indolin-2-on
6-(4-Fluorpyridin-3-yl)-3,3-dimethyl-1-(2-methylpyridin-4-yl)indolin-2-on
1-(5-Fluor-2-methylpyridin-4-yl)-3,3-dimethyl-6-(2-methylpyrimidin-5-yl)indolin-2-on
5-(3,3-Dimethyl-6-(2-methylpyrimidin-5-yl)-2-oxoindolin-1-yl)picolinonitril
1-(6-(Hydroxymethyl)pyridin-3-yl)-3,3-dimethyl-6-(2-methylpyrimidin-5-yl)indolin-2-on
1-(6-Cyclopropylpyridin-3-yl)-3,3-dimethyl-6-(2-methylpyrimidin-5-yl)indolin-2-on
3,3-Dimethyl-6-(2-methylpyrimidin-5-yl)-1-(pyridin-2-yl)indolin-2-on
1-(2-Fluorpyridin-4-yl)-3,3-dimethyl-6-(2-methylpyrimidin-5-yl)indolin-2-on
1-(3-Fluorpyridin-4-yl)-3,3-dimethyl-6-(2-methylpyrimidin-5-yl)indolin-2-on
1-(2-Fluor-5-methylpyridin-4-yl)-3,3-dimethyl-6-(2-methylpyrimidin-5-yl)indolin-2-on
3,3-Dimethyl-1-(6-methylpyridazin-3-yl)-6-(2-methylpyrimidin-5-yl)indolin-2-on
1-(3-Chlorpyridin-4-yl)-3,3-dimethyl-6-(2-methylpyrimidin-5-yl)indolin-2-on
3,3-Dimethyl-1-(5-methylpyrimidin-2-yl)-6-(2-methylpyrimidin-5-yl)indolin-2-on
3,3-Dimethyl-1-(5-methylpyridin-2-yl)-6-(2-methylpyrimidin-5-yl)indolin-2-on
3,3-Dimethyl-1-(5-methylpyrazin-2-yl)-6-(2-methylpyrimidin-5-yl)indolin-2-on
3,3-Dimethyl-1-(5-methylpyridin-3-yl)-6-(2-methylpyrimidin-5-yl)indolin-2-on
3,3-Dimethyl-1-(4-methylpyridin-2-yl)-6-(2-methylpyrimidin-5-yl)indolin-2-on
3,3-Dimethyl-1-(6-methylpyridin-2-yl)-6-(2-methylpyrimidin-5-yl)indolin-2-on
3,3-Dimethyl-1-(2-methylpyrimidin-4-yl)-6-(2-methylpyrimidin-5-yl)indolin-2-on
3,3-Dimethyl-1-(6-methylpyrazin-2-yl)-6-(2-methylpyrimidin-5-yl)indolin-2-on
3,3-Dimethyl-1-(4-methylpyrimidin-2-yl)-6-(2-methylpyrimidin-5-yl)indolin-2-on
1-(2,6-Dimethylpyridin-4-yl)-3,3-dimethyl-6-(2-methylpyrimidin-5-yl)indolin-2-on
-(4,6-Dimethylpyrimidin-2-yl)-3,3-dimethyl-6-(2-methylpyrimidin-5-yl)indolin-2-on
1-(2,6-Dimethylpyrimidin-4-yl)-3,3-dimethyl-6-(2-methylpyrimidin-5-yl)indolin-2-on
1-(4,5-Dimethylpyridin-2-yl)-3,3-dimethyl-6-(2-methylpyrimidin-5-yl)indolin-2-on
1-(5,6-Dimethylpyridin-2-yl)-3,3-dimethyl-6-(2-methylpyrimidin-5-yl)indolin-2-on
1-(5,6-Dimethylpyridin-3-yl)-3,3-dimethyl-6-(2-methylpyrimidin-5-yl)indolin-2-on
3,3-Dimethyl-6-(2-methylpyrimidin-5-yl)-1-(pyridazin-3-yl)indolin-2-on
3,3-Dimethyl-6-(2-methylpyrimidin-5-yl)-1-(pyrazin-2-yl)indolin-2-on
3,3-Dimethyl-6-(2-methylpyrimidin-5-yl)-1-(pyrimidin-2-yl)indolin-2-on
3,3-Dimethyl-6-(2-methylpyrimidin-5-yl)-1-(4-methylpyrimidin-5-yl)indolin-2-on
3,3-Dimethyl-1,6-bis(5-methylpyrimidin-2-yl)indolin-2-on
3,3-Dimethyl-1,6-bis(5-methylpyrazin-2-yl)indolin-2-on
3,3-Dimethyl-6-(2-methylpyrimidin-5-yl)-1-(pyrimidin-4-yl)indolin-2-on
1-(5-Cyclopropylpyrazin-2-yl)-3,3-dimethyl-6-(2-methylpyrimidin-5-yl)indolin-2-on
5-(3,3-Dimethyl-6-(2-methylpyrimidin-5-yl)-2-oxoindolin-1-yl)pyrazin-2-carbonitril
1-(6-Cyclopropylpyrazin-2-yl)-3,3-dimethyl-6-(2-methylpyrimidin-5-yl)indolin-2-on
1-(4,5-Dimethylpyridin-3-yl)-3,3-dimethyl-6-(2-methylpyrimidin-5-yl)indolin-2-on
1-(4,5-Dimethylpyridin-3-yl)-3,3-dimethyl-6-(2-methylpyrimidin-5-yl)indolin-2-on
1-(4,6-Dimethylpyridin-3-yl)-3,3-dimethyl-6-(2-methylpyrimidin-5-yl)indolin-2-on
5-(3,3-Dimethyl-1-(5-methylpyrimidin-2-yl)-2-oxoindolin-6-yl)-2-methylpyrimidin-1-oxid
1-(2-(Hydroxymethyl)pyrimidin-5-yl)-3,3-dimethyl-6-(2-methylpyrimidin-5-yl)indolin-2-on
1-[2-(Aminomethyl)pyrimidin-5-yl]-3,3-dimethyl-6-(2-methylpyrimidin-5-yl)indol-2-on
3-(3,3-Dimethyl-6-(2-methylpyrimidin-5-yl)-2-oxoindolin-1-yl)-6-methylpyridazin-1-oxid
3-(3,3-Dimethyl-6-(2-methyl-1-oxidopyrimidin-5-yl)-2-oxoindolin-1-yl)-6-methylpyridazin-1-oxid
1-(2-(Fluormethyl)pyrimidin-5-yl)-3,3-dimethyl-6-(2-methylpyrimidin-5-yl)indolin-2-on
3,3-Dimethyl-1-(6-methylpyridazin-3-yl)-6-(2-methylpyridin-4-yl)-1H-pyrrolo[3,2-c]pyridin-2(3H)-on
6-(4-Fluorphenyl)-3,3-dimethyl-1-(2-methylpyrimidin-5-yl)-1H-pyrrolo[3,2-c]pyridin-2(3H)-on

1-(5-Chlorpyrimidin-2-yl)-3,3-dimethyl-6-(2-methylpyrimidin-5-yl)indolin-2-on
1-(2-Chlorpyrimidin-5-yl)-3,3-dimethyl-6-(2-methylpyrimidin-5-yl)indolin-2-on
1-(2,6-Dichlorpyridin-4-yl)-3,3-dimethyl-6-(2-methylpyrimidin-5-yl)indolin-2-on
1-(2-Cyclopropylpyrimidin-5-yl)-3,3-dimethyl-6-(2-methylpyrimidin-5-yl)indolin-2-on
1-(5-Chlorpyrazin-2-yl)-3,3-dimethyl-6-(2-methylpyrimidin-5-yl)indol-2-on
1-(6-Chlorpyridazin-3-yl)-3,3-dimethyl-6-(2-methylpyrimidin-5-yl)indolin-2-on
1-(2-Chlor-6-methylpyridin-4-yl)-3,3-dimethyl-6-(2-methylpyrimidin-5-yl)indolin-2-on
3,3-Dimethyl-6-(2-methylpyrimidin-5-yl)-1-(pyridazin-4-yl)indolin-2-on
1-(6-Chlor-2-methylpyrimidin-4-yl)-3,3-dimethyl-6-(2-methylpyrimidin-5-yl)indolin-2-on
3,3-Dimethyl-1-(5-methylpyridazin-3-yl)-6-(2-methylpyrimidin-5-yl)indolin-2-on
1-(6-Chlorpyridazin-4-yl)-3,3-dimethyl-6-(2-methylpyrimidin-5-yl)indolin-2-on
3,3-Dimethyl-1-(3-methylpyrazin-2-yl)-6-(2-methylpyrimidin-5-yl)indolin-2-on
1-(4-Chlorpyrimidin-2-yl)-3,3-dimethyl-6-(2-methylpyrimidin-5-yl)indolin-2-on
1-(6-(Methoxymethyl)pyridin-3-yl)-3,3-dimethyl-6-(2-methylpyrimidin-5-yl)indolin-2-on
1-(5-Cyclopropylpyridazin-3-yl)-3,3-dimethyl-6-(2-methylpyrimidin-5-yl)indolin-2-on
3,3-Dimethyl-6-(5-methylpyrazin-2-yl)-1-(6-methylpyrazin-2-yl)indolin-2-on
3,3-Dimethyl-6-(5-methylpyrazin-2-yl)-1-(2-methylpyrimidin-5-yl)indolin-2-on
1-(6-Cyclopropylpyridazin-3-yl)-3,3-dimethyl-6-(2-methylpyrimidin-5-yl)indolin-2-on
3,3-Dimethyl-1-(6-methylpyrazin-2-yl)-6-(6-methyl-2-pyridyl)indolin-2-on
3,3-Dimethyl-1-(5-methylpyrazin-2-yl)-6-(2-methylpyrimidin-4-yl)indolin-2-on
3,3-Dimethyl-6-(5-methylpyrazin-2-yl)-1-(6-methylpyridazin-3-yl)indolin-2-on
1-(5-Fluorpyridin-3-yl)-3,3-dimethyl-6-(2-methylpyrimidin-5-yl)indolin-2-on
5-(3,3-Dimethyl-6-(2-methylpyrimidin-5-yl)-2-oxoindolin-1-yl)nicotinonitril
3,3-Dimethyl-1-(6-methylpyrazin-2-yl)-6-(2-methyl-4-pyridyl)indolin-2-on
3,3-Dimethyl-1-(6-methylpyrazin-2-yl)-6-(5-methyl-3-pyridyl)indolin-2-on
6-(5-Fluor-3-pyridyl)-3,3-dimethyl-1-(6-methylpyrazin-2-yl)indolin-2-on
3,3-Dimethyl-1-(5-methylpyrazin-2-yl)-6-(2-methyl-4-pyridyl)indolin-2-on
3,3-Dimethyl-1-(5-methylpyrazin-2-yl)-6-(6-methyl-3-pyridyl)indolin-2-on
3,3-Dimethyl-1-(5-methylpyrazin-2-yl)-6-(5-methyl-3-pyridyl)indolin-2-on
6-(5-Fluor-3-pyridyl)-3,3-dimethyl-1-(5-methylpyrazin-2-yl)indolin-2-on
6-(5-Fluor-6-methyl-3-pyridyl)-3,3-dimethyl-1-(5-methylpyrazin-2-yl)indolin-2-on
3,3-Dimethyl-6-(2-methylpyrimidin-5-yl)-1-(5-(trifluormethyl)pyridin-3-yl)indolin-2-on
1-(5-(Hydroxymethyl)pyridin-3-yl)-3,3-dimethyl-6-(2-methylpyrimidin-5-yl)indolin-2-on
3,3-Dimethyl-1-(6-methylpyrazin-2-yl)-6-(2-methylpyrimidin-4-yl)indolin-2-on
5-[3,3-Dimethyl-1-(6-methylpyrazin-2-yl)-2-oxoindolin-6-yl]pyrimidin-2-carbonitril oder
1-(5-Ethylpyridin-3-yl)-3,3-dimethyl-6-(2-methylpyrimidin-5-yl)indolin-2-on.

**5.** Verbindung der Formel I nach Anspruch 2, wobei $Ar^1$ für eine sechsgliedrige Heteroarylgruppe steht, die ein, zwei oder drei Heteroatome enthält, die aus N, S oder O ausgewählt sind, und $Ar^2$ für eine fünfgliedrige Heteroarylgruppe steht, die ein, zwei oder drei Heteroatome enthält, die aus N, S oder O ausgewählt sind.

**6.** Verbindung der Formel I nach Anspruch 5, wobei die Verbindungen Folgende sind:

3,3-Dimethyl-1-(1-methyl-1H-imidazol-4-yl)-6-(2-methylpyrimidin-5-yl)indolin-2-on
3,3-Dimethyl-1-(1-methyl-1H-pyrazol-3-yl)-6-(2-methylpyrimidin-5-yl)indolin-2-on
1-(1,5-Dimethyl-1H-pyrazol-3-yl)-3,3-dimethyl-6-(2-methylpyrimidin-5-yl)indolin-2-on
3,3-Dimethyl-1-(1-methyl-1H-imidazol-4-yl)-6-(6-methylpyridin-3-yl)indolin-2-on
3,3-Dimethyl-1-(1-methyl-1H-imidazol-4-yl)-6-(2-methylpyridin-4-yl)indolin-2-on
3,3-Dimethyl-1-(1-methyl-1H-pyrazol-3-yl)-6-(6-methylpyridin-3-yl)indolin-2-on
6-(4-Fluorpyridin-3-yl)-3,3-dimethyl-1-(1-methyl-1H-imidazol-4-yl)indolin-2-on
6-(4-Fluorpyridin-3-yl)-3,3-dimethyl-1-(1-methyl-1H-pyrazol-3-yl)indolin-2-on
3,3-Dimethyl-6-(2-methylpyrimidin-5-yl)-1-(5-methylthiophen-2-yl)indolin-2-on
3,3-Dimethyl-1-(1-methyl-1H-imidazol-5-yl)-6-(2-methylpyrimidin-5-yl)indolin-2-on
3,3-Dimethyl-1-(5-methyl-1,3,4-oxadiazol-2-yl)-6-(2-methylpyrimidin-5-yl)indolin-2-on
3,3-Dimethyl-6-(2-methylpyrimidin-5-yl)-1-(1H-pyrazol-3-yl)indolin-2-on
1-(1,2-Dimethyl-1H-imidazol-4-yl)-3,3-dimethyl-6-(2-methylpyrimidin-5-yl)indolin-2-on
1-(5-Ethylpyridin-3-yl)-3,3-dimethyl-6-(2-methylpyrimidin-5-yl)indolin-2-on
1-(1,5-Dimethyl-1H-1,2,4-triazol-3-yl)-3,3-dimethyl-6-(2-methylpyrimidin-5-yl)indolin-2-on

3,3-Dimethyl-1-(1-methyl-1H-pyrazol-4-yl)-6-(2-methylpyrimidin-5-yl)indolin-2-on
3,3-Dimethyl-1-(2-methyl-1H-imidazol-4-yl)-6-(2-methylpyrimidin-5-yl)indolin-2-on
3,3-Dimethyl-1-(1-methyl-1H-1,2,4-triazol-3-yl)-6-(2-methylpyrimidin-5-yl)indolin-2-on
3,3-Dimethyl-6-(2-methylpyrimidin-5-yl)-1-(1H-pyrazol-4-yl)indolin-2-on
1-(1H-Imidazol-4-yl)-3,3-dimethyl-6-(2-methylpyrimidin-5-yl)indolin-2-on
3,3-Dimethyl-1-(3-methyl-1H-pyrazol-5-yl)-6-(2-methylpyrimidin-5-yl)indolin-2-on
3,3-Dimethyl-1-(1-methyl-1H-1,2,3-triazol-4-yl)-6-(2-methylpyrimidin-5-yl)indolin-2-on
3,3-Dimethyl-1-(4-methyl-1H-imidazol-2-yl)-6-(2-methylpyrimidin-5-yl)indolin-2-on
3,3-Dimethyl-1-(3-methylisoxazol-5-yl)-6-(2-methylpyrimidin-5-yl)indolin-2-on
1-(1H-Imidazol-2-yl)-3,3-dimethyl-6-(2-methylpyrimidin-5-yl)indolin-2-on
1-(1-Ethyl-1H-pyrazol-3-yl)-3,3-dimethyl-6-(2-methylpyrimidin-5-yl)indolin-2-on
3,3-Dimethyl-6-(2-methylpyrimidin-5-yl)-1-(2H-tetrazol-5-yl)indolin-2-on
3,3-Dimethyl-1-(2-methyl-2H-tetrazol-5-yl)-6-(2-methylpyrimidin-5-yl)indolin-2-on
3,3-Dimethyl-1-(1-methyl-1H-pyrazol-4-yl)-6-(2-methylpyridin-4-yl)-1H-pyrrolo[3,2-c]pyridin-2(3H)-on
3,3-Dimethyl-1-(1-methyl-1H-imidazol-4-yl)-6-(2-methylpyridin-4-yl)-1H-pyrrolo[3,2-c]pyridin-2(3H)-on
3,3-Dimethyl-1-(3-methyl-1,2,4-thiadiazol-5-yl)-6-(2-methylpyrimidin-5-yl)indolin-2-on
3,3-Dimethyl-1-(3-methylisothiazol-5-yl)-6-(2-methylpyrimidin-5-yl)indolin-2-on
3,3-Dimethyl-6-(2-methylpyrimidin-5-yl)-1-(5-methylthiazol-2-yl)indolin-2-on
1-(1-Isopropyl-1H-pyrazol-3-yl)-3,3-dimethyl-6-(2-methylpyrimidin-5-yl)indolin-2-on
3,3-Dimethyl-1-(1-methyl-1H-pyrazol-3-yl)-6-(5-methylpyrazin-2-yl)indolin-2-on
3,3-Dimethyl-6-(2-methylpyrimidin-5-yl)-1-(1-(2,2,2-trifluorethyl)-1H-pyrazol-3-yl)indolin-2-on
1-(1-(2-Methoxyethyl)-1H-pyrazol-3-yl)-3,3-dimethyl-6-(2-methylpyrimidin-5-yl)indolin-2-on
oder
2-(3-(3,3-Dimethyl-6-(2-methylpyrimidin-5-yl)-2-oxoindolin-1-yl)-1H-pyrazol-1-yl)-N,N-dimethylacetamid.

**7.** Verbindung der Formel I nach Anspruch 2, wobei $Ar^1$ für eine fünfgliedrige Heteroarylgruppe steht, die ein, zwei oder drei Heteroatome enthält, die aus N, S oder O ausgewählt sind, und $Ar^2$ für eine sechsgliedrige Heteroarylgruppe steht, die ein, zwei oder drei Heteroatome enthält, die aus N, S oder O ausgewählt sind.

**8.** Verbindung der Formel I nach Anspruch 7, wobei die Verbindungen Folgende sind:

3,3-Dimethyl-6-(4-methylimidazol-1-yl)-1-(6-methyl-3-pyridyl)indolin-2-on
6-(4-Cyclopropylimidazol-1-yl)-3,3-dimethyl-1-(2-methyl-4-pyridyl)indolin-2-on
3,3-Dimethyl-6-(4-methylimidazol-1-yl)-1-(2-methyl-4-pyridyl)indolin-2-on
3,3-Dimethyl-6-(1-methyl-1H-pyrazol-3-yl)-1-(2-methylpyrimidin-5-yl)indolin-2-on
3,3-Dimethyl-6-(1-methyl-1H-imidazol-4-yl)-1-(2-methylpyrimidin-5-yl)indolin-2-on
6-(4-Isopropylimidazol-1-yl)-3,3-dimethyl-1-(6-methyl-3-pyridyl)indolin-2-on
3,3-Dimethyl-6-(5-methyl-1,3,4-oxadiazol-2-yl)-1-(6-methyl-3-pyridyl)indolin-2-on
3,3-Dimethyl-6-(5-methyl-1,3,4-oxadiazol-2-yl)-1-(2-methyl-4-pyridyl)indolin-2-on
6-(4-Isopropylimidazol-1-yl)-3,3-dimethyl-1-(2-methyl-4-pyridyl)indolin-2-on
6-(4-Isopropylimidazol-1-yl)-3,3-dimethyl-1-(2-methylpyrimidin-5-yl)indolin-2-on
6-(4-Cyclopropylimidazol-1-yl)-3,3-dimethyl-1-(2-methylpyrimidin-5-yl)indolin-2-on
3,3-Dimethyl-6-(5-methyl-1,3,4-oxadiazol-2-yl)-1-(2-methylpyrimidin-5-yl)indolin-2-on
3,3-Dimethyl-6-(4-methylimidazol-1-yl)-1-(2-methylpyrimidin-5-yl)indolin-2-on
3,3-Dimethyl-6-(2-methyloxazol-5-yl)-1-(2-methylpyrimidin-5-yl)indolin-2-on oder
3,3-Dimethyl-6-(3-methyl-1,2,4-oxadiazol-5-yl)-1-(2-methylpyrimidin-5-yl)indolin-2-on.

**9.** Verbindung der Formel I nach Anspruch 2, wobei sowohl $Ar^1$ als auch $Ar^2$ für eine fünfgliedrige Heteroarylgruppe stehen, die ein, zwei oder drei Heteroatome enthält, die aus N, S oder O ausgewählt sind.

**10.** Verbindung der Formel I nach Anspruch 9, wobei die Verbindungen Folgende sind:

6-(4-Isopropylimidazol-1-yl)-3,3-dimethyl-1-(1-methylpyrazol-3-yl)indolin-2-on
6-(4-Cyclopropylimidazol-1-yl)-3,3-dimethyl-1-(1-methylimidazol-4-yl)indolin-2-on
6-(4-Cyclopropylimidazol-1-yl)-3,3-dimethyl-1-(1-methylpyrazol-3-yl)indolin-2-on
3,3-Dimethyl-1-(1-methylimidazol-4-yl)-6-(2-methyloxazol-5-yl)indolin-2-on
3,3-Dimethyl-6-(2-methyloxazol-5-yl)-1-(1-methylpyrazol-3-yl)indolin-2-on
3,3-Dimethyl-1-(1-methylimidazol-4-yl)-6-(5-methyl-1,3,4-oxadiazol-2-yl)indolin-2-on oder

3,3-Dimethyl-6-(4-methyl-1H-imidazol-1-yl)-1-(1-methyl-1H-imidazol-4-yl)indolin-2-on.

**11.** Verbindung der Formel I nach Anspruch 2, wobei $Ar^2$ für Benzo[b]thiophenyl steht und die anderen Substituenten wie in Anspruch 1 beschrieben sind.

**12.** Verbindung der Formel I nach Anspruch 11, wobei die Verbindung 1-(Benzo[b]thiophen-4-yl)-3,3-dimethyl-6-(2-methylpyrimidin-5-yl)indolin-2-on ist.

**13.** Verbindung der Formel I nach Anspruch 1, wobei X für N steht und die anderen Substituenten wie in Anspruch 1 beschrieben sind.

**14.** Verbindung der Formel I nach Anspruch 13, wobei die Verbindungen Folgende sind:

3,3-Dimethyl-1-(1-methyl-1H-imidazol-4-yl)-6-(2-methylpyrimidin-5-yl)-1H-pyrrolo[3,2-c]pyridin-2(3H)-on
3,3-Dimethyl-1-(1-methyl-1H-pyrazol-3-yl)-6-(2-methylpyrimidin-5-yl)-1H-pyrrolo[3,2-c]pyridin-2(3H)-on
3,3-Dimethyl-1-(2-methylpyridin-4-yl)-6-(2-methylpyrimidin-5-yl)-1H-pyrrolo[3,2-c]pyridin-2(3H)-on
3,3-Dimethyl-1-(6-methylpyridazin-3-yl)-6-(2-methylpyridin-4-yl)-1H-pyrrolo[3,2-c]pyridin-2(3H)-on
3,3-Dimethyl-1-(1-methyl-1H-pyrazol-4-yl)-6-(2-methylpyridin-4-yl)-1H-pyrrolo[3,2-c]pyridin-2(3H)-on
3,3-Dimethyl-1-(1-methyl-1H-imidazol-4-yl)-6-(2-methylpyridin-4-yl)-1H-pyrrolo[3,2-c]pyridin-2(3H)-on oder
6-(4-Fluorphenyl)-3,3-dimethyl-1-(2-methylpyrimidin-5-yl)-1H-pyrrolo[3,2-c]pyridin-2(3H)-on.

**15.** Kombination einer Verbindung der Formel I nach einem der Ansprüche 1-14, zusammen mit einem bekannten, marktgängigen Antipsychotikum, Antidepressivum, Anxiolytikum oder Stimmungsstabilisierer.

**16.** Kombination nach Anspruch 15, wobei das marktgängige antipsychotische Arzneimittel Olanzapin (Zyprexa), Clozapin (Clozaril), Risperidon (Risperdal), Aripiprazol (Abilify) oder Ziprasidon ist.

**17.** Kombination nach Anspruch 15, wobei das marktgängige antidepressive Arzneimittel Citalopram (Celexa), Escitalopram (Lexapro, Cipralex), Paroxetin (Paxil, Seroxat), Fluoxetin (Prozac), Sertralin (Zoloft, Lustral), Duloxetin (Cymbalta), Milnacipran (Ixel, Savella), Venlafaxin (Effexor) oder Mirtazapin (Remeron) ist.

**18.** Kombination nach Anspruch 15, wobei das marktgängige anxiolytische Arzneimittel Alprazolam (Helex, Xanax, Xanor, Onax, Alprox, Restyl, Tafil, Paxal), Chlordiazepoxid (Librium, Risolid, Elenium), Clonazepam (Rivotril, Klonopin, Iktorivil, Paxam), Diazepam (Antenex, Apaurin, Apzepam, Apozepam, Hexalid, Pax, Stesolid, Stedon, Valium, Vival, Valaxona), Estazolam (ProSom), Eszopiclon (Lunesta), Zaleplon (Sonata, Starnoc), Zolpidem (Ambien, Nytamel, Stilnoct, Stilnox, Zoldem, Zolnod), Pregabalin (Lyrica) oder Gabapentin (Fanatrex, Gabaron, Gralise, Neurontin, Nupentin) ist.

**19.** Kombination nach Anspruch 15, wobei der marktgängige Stimmungsstabilisierer Carbamazepin (Tegretol), Lamotrigin (Lamictal), Lithium (Eskalith, Lithane, Lithobid) und Valproinsäure (Depakote) ist.

**20.** Verfahren zum Herstellen einer Verbindung der Formel I, wie in einem der Ansprüche 1 bis 14 beschrieben, umfassend das Umsetzen
einer Verbindung der Formel

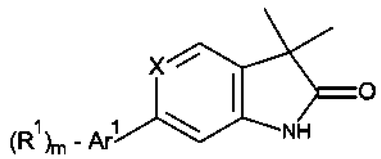

7

mit einer Verbindung der Formel

$(R^2)_n$-$Ar^2$-Y 8

zu einer Verbindung der Formel

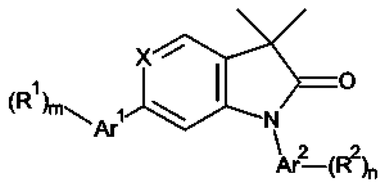

I,

wobei Y für Cl, Br oder I steht und die anderen Gruppen dieselben Bedeutungen wie die in Anspruch 1 beschriebenen haben, und, falls erwünscht, die erhaltenen Verbindungen in pharmazeutisch annehmbare Säureadditionssalze umgewandelt werden.

**21.** Verbindung nach einem der Ansprüche 1-14 zur Verwendung als therapeutisch aktive Substanz.

**22.** Pharmazeutische Zusammensetzung, umfassend eine Verbindung nach einem der Ansprüche 1-14 und einen therapeutisch wirksamen Träger zur Verwendung in der Behandlung von ZNS-Erkrankungen in Verbindung mit positiven (Psychose) und negativen Symptomen von Schizophrenie, Drogenmissbrauch, Alkohol- und Medikamentenabhängigkeit, Zwangsstörungen, kognitiver Beeinträchtigung, bipolaren Störungen, Stimmungsschwankungen, schwerer Depression, therapieresistenter Depression, Angstzuständen, Alzheimer-Krankheit, Autismus, Parkinson-Krankheit, chronischen Schmerzen, Borderline-Persönlichkeitsstörung, neurodegenerativen Erkrankungen, Schlafstörungen, chronischem Erschöpfungssyndrom, Steifheit, entzündlichen Erkrankungen, Asthma, Huntington-Krankheit, ADHD, amyotropher Lateralsklerose, Epilepsie, Wirkungen bei Arthritis, Autoimmunerkrankungen, viralen und mykotischen Infektionen, Herz-Kreislauf-Erkrankungen, Erkrankungen im Bereich der Ophthalmologie und entzündlichen Netzhauterkrankungen und Gleichgewichtsproblemen.

**23.** Verwendung einer Verbindung der Formel I nach einem der Ansprüche 1-14 für die Herstellung eines Medikaments zur Behandlung von ZNS-Erkrankungen in Verbindung mit positiven (Psychose) und negativen Symptomen von Schizophrenie, Drogenmissbrauch, Alkohol- und Medikamentenabhängigkeit, Zwangsstörungen, kognitiver Beeinträchtigung, bipolaren Störungen, Stimmungsschwankungen, schwerer Depression, therapieresistenter Depression, Angstzuständen, Alzheimer-Krankheit, Autismus, Parkinson-Krankheit, chronischen Schmerzen, Borderline-Persönlichkeitsstörung, neurodegenerativen Erkrankungen, Schlafstörungen, chronischem Erschöpfungssyndrom, Steifheit, entzündlichen Erkrankungen, Asthma, Huntington-Krankheit, ADHD, amyotropher Lateralsklerose, Epilepsie, Wirkungen bei Arthritis, Autoimmunerkrankungen, viralen und mykotischen Infektionen, Herz-Kreislauf-Erkrankungen, Erkrankungen im Bereich der Ophthalmologie und entzündlichen Netzhauterkrankungen und Gleichgewichtsproblemen.

**24.** Verbindung nach einem der Ansprüche 1-14 zur Verwendung bei der Behandlung von ZNS-Erkrankungen in Verbindung mit positiven (Psychose) und negativen Symptomen von Schizophrenie, Drogenmissbrauch, Alkohol- und Medikamentenabhängigkeit, Zwangsstörungen, kognitiver Beeinträchtigung, bipolaren Störungen, Stimmungsschwankungen, schwerer Depression, therapieresistenter Depression, Angstzuständen, Alzheimer-Krankheit, Autismus, Parkinson-Krankheit, chronischen Schmerzen, Borderline-Persönlichkeitsstörung, neurodegenerativen Erkrankungen, Schlafstörungen, chronischem Erschöpfungssyndrom, Steifheit, entzündlichen Erkrankungen, Asthma, Huntington-Krankheit, ADHD, amyotropher Lateralsklerose, Epilepsie, Wirkungen bei Arthritis, Autoimmunerkrankungen, viralen und mykotischen Infektionen, Herz-Kreislauf-Erkrankungen, Erkrankungen im Bereich der Ophthalmologie und entzündlichen Netzhauterkrankungen und Gleichgewichtsproblemen.

## Revendications

**1.** Composé de formule

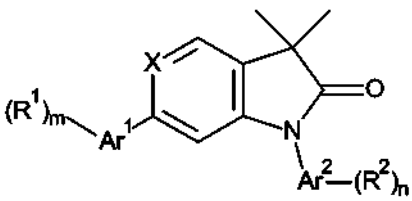

I

dans laquelle

$Ar^1$ représente un phényle ou un groupe hétéroaryle à cinq ou six chaînons, contenant un, deux ou trois hétéroatomes, choisis parmi N, S ou O, l'hétéroatome N dans le groupe hétéroaryle pouvant être oxydé en $N^+$-($O^-$) ;
$R^1$ représente un alkyle en $C_{1-7}$, un halogène, un cyano ou un cycloalkyle ;
$Ar^2$ représente un groupe hétéroaryle à cinq ou six chaînons, contenant un, deux, trois ou quatre hétéroatomes, choisis parmi N, S ou O, l'hétéroatome N dans le groupe hétéroaryle pouvant être oxydé en $N^+$-($O^-$), ou représente un benzo[b]thiophényle ;
$R^2$ représente un hydrogène, un alkyle en $C_{1-7}$, un halogène, un cyano, un alkyle en $C_{1-7}$ substitué par un hydroxyle, un alkyle en $C_{1-7}$ substitué par un halogène, un alkyle en $C_{1-7}$ substitué par un groupe amino, un alkyle en $C_{1-7}$ substitué par un alcoxy en $C_{1-7}$, un alkyle en $C_{1-7}$ substitué par un amide, ou représente un cycloalkyle ;
X représente CH ou N ;
n représente 1 ou 2 ;
m représente 1 ou 2 ;

ainsi qu'un sel pharmaceutiquement acceptable de celui-ci, un mélange racémique, ou son énantiomère et/ou isomère optique et/ou stéréoisomère correspondants.

**2.** Composé de formule I selon la revendication 1, dans laquelle X représente CH.

**3.** Composé de formule I selon la revendication 2, dans laquelle $Ar^1$ et $Ar^2$ représentent tous les deux un groupe hétéroaryle à six chaînons, contenant un, deux ou trois hétéroatomes, choisis parmi N, S ou O.

**4.** Composé de formule I selon la revendication 3, lesdits composés étant

la 3,3-diméthyl-6-(2-méthylpyrimidin-5-yl)-1-(pyridin-4-yl)indolin-2-one
la 3,3-diméthyl-6-(2-méthylpyrimidin-5-yl)-1-(pyridin-3-yl)indolin-2-one
la 3,3-diméthyl-1-(2-méthylpyridin-4-yl)-6-(2-méthylpyrimidin-5-yl)indolin-2-one
la 3,3-diméthyl-1-(6-méthylpyrimidin-4-yl)-6-(2-méthylpyrimidin-5-yl)indolin-2-one
la 3,3-diméthyl-6-(2-méthylpyrimidin-5-yl)-1-(pyrimidin-5-yl)indolin-2-one
la 3,3-diméthyl-1,6-bis(2-méthylpyrimidin-5-yl)indolin-2-one
la 3,3-diméthyl-1-(6-méthylpyridin-3-yl)-6-(2-méthylpyrimidin-5-yl)indolin-2-one
la 3,3-diméthyl-6-(6-méthylpyridin-3-yl)-1-(2-méthylpyridin-4-yl)indolin-2-one
la 3,3-diméthyl-1,6-bis(2-méthylpyridin-4-yl)indolin-2-one
la 6-(4-fluoropyridin-3-yl)-3,3-diméthyl-1-(2-méthylpyridin-4-yl)indolin-2-one
la 1-(5-fluoro-2-méthylpyridin-4-yl)-3,3-diméthyl-6-(2-méthylpyrimidin-5-yl)indolin-2-one
le 5-(3,3-diméthyl-6-(2-méthylpyrimidin-5-yl)-2-oxoindolin-1-yl)picolinonitrile
la 1-(6-(hydroxyméthyl)pyridin-3-yl)-3,3-diméthyl-6-(2-méthylpyrimidin-5-yl)indolin-2-one
la 1-(6-cyclopropylpyridin-3-yl)-3,3-diméthyl-6-(2-méthylpyrimidin-5-yl)indolin-2-one
la 3,3-diméthyl-6-(2-méthylpyrimidin-5-yl)-1-(pyridin-2-yl)indolin-2-one
la 1-(2-fluoropyridin-4-yl)-3,3-diméthyl-6-(2-méthylpyrimidin-5-yl)indolin-2-one
la 1-(3-fluoropyridin-4-yl)-3,3-diméthyl-6-(2-méthylpyrimidin-5-yl)indolin-2-one
la 1-(2-fluoro-5-méthylpyridin-4-yl)-3,3-diméthyl-6-(2-méthylpyrimidin-5-yl)indolin-2-one
la 3,3-diméthyl-1-(6-méthylpyridazin-3-yl)-6-(2-méthylpyrimidin-5-yl)indolin-2-one
la 1-(3-chloropyridin-4-yl)-3,3-diméthyl-6-(2-méthylpyrimidin-5-yl)indolin-2-one
la 3,3-diméthyl-1-(5-méthylpyrimidin-2-yl)-6-(2-méthylpyrimidin-5-yl)indolin-2-one
la 3,3-diméthyl-1-(5-méthylpyridin-2-yl)-6-(2-méthylpyrimidin-5-yl)indolin-2-one
la 3,3-diméthyl-1-(5-méthylpyrazin-2-yl)-6-(2-méthylpyrimidin-5-yl)indolin-2-one
la 3,3-diméthyl-1-(5-méthylpyridin-3-yl)-6-(2-méthylpyrimidin-5-yl)indolin-2-one
la 3,3-diméthyl-1-(4-méthylpyridin-2-yl)-6-(2-méthylpyrimidin-5-yl)indolin-2-one
la 3,3-diméthyl-1-(6-méthylpyridin-2-yl)-6-(2-méthylpyrimidin-5-yl)indolin-2-one
la 3,3-diméthyl-1-(2-méthylpyrimidin-4-yl)-6-(2-méthylpyrimidin-5-yl)indolin-2-one
la 3,3-diméthyl-1-(6-méthylpyrazin-2-yl)-6-(2-méthylpyrimidin-5-yl)indolin-2-one
la 3,3-diméthyl-1-(4-méthylpyrimidin-2-yl)-6-(2-méthylpyrimidin-5-yl)indolin-2-one
la 1-(2,6-diméthylpyridin-4-yl)-3,3-diméthyl-6-(2-méthylpyrimidin-5-yl)indolin-2-one
la -(4,6-diméthylpyrimidin-2-yl)-3,3-diméthyl-6-(2-méthylpyrimidin-5-yl)indolin-2-one

la 1-(2,6-diméthylpyrimidin-4-yl)-3,3-diméthyl-6-(2-méthylpyrimidin-5-yl)indolin-2-one
la 1-(4,5-diméthylpyridin-2-yl)-3,3-diméthyl-6-(2-méthylpyrimidin-5-yl)indolin-2-one
la 1-(5,6-diméthylpyridin-2-yl)-3,3-diméthyl-6-(2-méthylpyrimidin-5-yl)indolin-2-one
la 1-(5,6-diméthylpyridin-3-yl)-3,3-diméthyl-6-(2-méthylpyrimidin-5-yl)indolin-2-one
la 3,3-diméthyl-6-(2-méthylpyrimidin-5-yl)-1-(pyridazin-3-yl)indolin-2-one
la 3,3-diméthyl-6-(2-méthylpyrimidin-5-yl)-1-(pyrazin-2-yl)indolin-2-one
la 3,3-diméthyl-6-(2-méthylpyrimidin-5-yl)-1-(pyrimidin-2-yl)indolin-2-one
la 3,3-diméthyl-6-(2-méthylpyrimidin-5-yl)-1-(4-méthylpyrimidin-5-yl)indolin-2-one
la 3,3-diméthyl-1,6-bis(5-méthylpyrimidin-2-yl)indolin-2-one
la 3,3-diméthyl-1,6-bis(5-méthylpyrazin-2-yl)indolin-2-one
la 3,3-diméthyl-6-(2-méthylpyrimidin-5-yl)-1-(pyrimidin-4-yl)indolin-2-one
la 1-(5-cyclopropylpyrazin-2-yl)-3,3-diméthyl-6-(2-méthylpyrimidin-5-yl)indolin-2-one
le 5-(3,3-diméthyl-6-(2-méthylpyrimidin-5-yl)-2-oxoindolin-1-yl)pyrazine-2-carbonitrile
la 1-(6-cyclopropylpyrazin-2-yl)-3,3-diméthyl-6-(2-méthylpyrimidin-5-yl)indolin-2-one
la 1-(4,5-diméthylpyridin-3-yl)-3,3-diméthyl-6-(2-méthylpyrimidin-5-yl)indolin-2-one
la 1-(4,5-diméthylpyridin-3-yl)-3,3-diméthyl-6-(2-méthylpyrimidin-5-yl)indolin-2-one
la 1-(4,6-diméthylpyridin-3-yl)-3,3-diméthyl-6-(2-méthylpyrimidin-5-yl)indolin-2-one
le 1-oxyde de 5-(3,3-diméthyl-1-(5-méthylpyrimidin-2-yl)-2-oxoindolin-6-yl)-2-méthylpyrimidine
la 1-(2-(hydroxyméthyl)pyrimidin-5-yl)-3,3-diméthyl-6-(2-méthylpyrimidin-5-yl)indolin-2-one
la 1-[2-(aminométhyl)pyrimidin-5-yl]-3,3-diméthyl-6-(2-méthylpyrimidin-5-yl)indol-2-one
le 1-oxyde de 3-(3,3-diméthyl-6-(2-méthylpyrimidin-5-yl)-2-oxoindolin-1-yl)-6-méthylpyridazine
le 1-oxyde de 3-(3,3-diméthyl-6-(2-méthyl-1-oxydopyrimidin-5-yl)-2-oxoindolin-1-yl)-6-méthylpyridazinela 1-(2-(fluorométhyl)pyrimidin-5-yl)-3,3-diméthyl-6-(2-méthylpyrimidin-5-yl)indolin-2-one
la 3,3-diméthyl-1-(6-méthylpyridazin-3-yl)-6-(2-méthylpyridin-4-yl)-1H-pyrrolo [3,2-c]pyridin-2(3H)-one
la 6-(4-fluorophényl)-3,3-diméthyl-1-(2-méthylpyrimidin-5-yl)-1H-pyrrolo[3,2-c]pyridin-2(3H)-one
la 1-(5-chloropyrimidin-2-yl)-3,3-diméthyl-6-(2-méthylpyrimidin-5-yl)indolin-2-one
la 1-(2-chloropyrimidin-5-yl)-3,3-diméthyl-6-(2-méthylpyrimidin-5-yl)indolin-2-one
la 1-(2,6-dichloropyridin-4-yl)-3,3-diméthyl-6-(2-méthylpyrimidin-5-yl)indolin-2-one
la 1-(2-cyclopropylpyrimidin-5-yl)-3,3-diméthyl-6-(2-méthylpyrimidin-5-yl)indolin-2-one
la 1-(5-chloropyrazin-2-yl)-3,3-diméthyl-6-(2-méthylpyrimidin-5-yl)indol-2-one
la 1-(6-chloropyridazin-3-yl)-3,3-diméthyl-6-(2-méthylpyrimidin-5-yl)indolin-2-one
la 1-(2-chloro-6-méthylpyridin-4-yl)-3,3-diméthyl-6-(2-méthylpyrimidin-5-yl)indolin-2-one
la 3,3-diméthyl-6-(2-méthylpyrimidin-5-yl)-1-(pyridazin-4-yl)indolin-2-one
la 1-(6-chloro-2-méthylpyrimidin-4-yl)-3,3-diméthyl-6-(2-méthylpyrimidin-5-yl)indolin-2-one
la 3,3-diméthyl-1-(5-méthylpyridazin-3-yl)-6-(2-méthylpyrimidin-5-yl)indolin-2-one
la 1-(6-chloropyridazin-4-yl)-3,3-diméthyl-6-(2-méthylpyrimidin-5-yl)indolin-2-one
la 3,3-diméthyl-1-(3-méthylpyrazin-2-yl)-6-(2-méthylpyrimidin-5-yl)indolin-2-one
la 1-(4-chloropyrimidin-2-yl)-3,3-diméthyl-6-(2-méthylpyrimidin-5-yl)indolin-2-one
la 1-(6-(méthoxyméthyl)pyridin-3-yl)-3,3-diméthyl-6-(2-méthylpyrimidin-5-yl)indolin-2-one
la 1-(5-cyclopropylpyridazin-3-yl)-3,3-diméthyl-6-(2-méthylpyrimidin-5-yl)indolin-2-one
la 3,3-diméthyl-6-(5-méthylpyrazin-2-yl)-1-(6-méthylpyrazin-2-yl)indolin-2-one
la 3,3-diméthyl-6-(5-méthylpyrazin-2-yl)-1-(2-méthylpyrimidin-5-yl)indolin-2-one
la 1-(6-cyclopropylpyridazin-3-yl)-3,3-diméthyl-6-(2-méthylpyrimidin-5-yl)indolin-2-one
la 3,3-diméthyl-1-(6-méthylpyrazin-2-yl)-6-(6-méthyl-2-pyridyl)indolin-2-one
la 3,3-diméthyl-1-(5-méthylpyrazin-2-yl)-6-(2-méthylpyrimidin-4-yl)indolin-2-one
la 3,3-diméthyl-6-(5-méthylpyrazin-2-yl)-1-(6-méthylpyridazin-3-yl)indolin-2-one
la 1-(5-fluoropyridin-3-yl)-3,3-diméthyl-6-(2-méthylpyrimidin-5-yl)indolin-2-one
le 5-(3,3-diméthyl-6-(2-méthylpyrimidin-5-yl)-2-oxoindolin-1-yl)nicotinonitrile
la 3,3-diméthyl-1-(6-méthylpyrazin-2-yl)-6-(2-méthyl-4-pyridyl)indolin-2-one
la 3,3-diméthyl-1-(6-méthylpyrazin-2-yl)-6-(5-méthyl-3-pyridyl)indolin-2-one
la 6-(5-fluoro-3-pyridyl)-3,3-diméthyl-1-(6-méthylpyrazin-2-yl)indolin-2-one
la 3,3-diméthyl-1-(5-méthylpyrazin-2-yl)-6-(2-méthyl-4-pyridyl)indolin-2-one
la 3,3-diméthyl-1-(5-méthylpyrazin-2-yl)-6-(6-méthyl-3-pyridyl)indolin-2-one
la 3,3-diméthyl-1-(5-méthylpyrazin-2-yl)-6-(5-méthyl-3-pyridyl)indolin-2-one
la 6-(5-fluoro-3-pyridyl)-3,3-diméthyl-1-(5-méthylpyrazin-2-yl)indolin-2-one
la 6-(5-fluoro-6-méthyl-3-pyridyl)-3,3-diméthyl-1-(5-méthylpyrazin-2-yl)indolin-2-one
la 3,3-diméthyl-6-(2-méthylpyrimidin-5-yl)-1-(5-(trifluorométhyl)pyridin-3-yl)indolin-2-one
la 1-(5-(hydroxyméthyl)pyridin-3-yl)-3,3-diméthyl-6-(2-méthylpyrimidin-5-yl)indolin-2-one

la 3,3-diméthyl-1-(6-méthylpyrazin-2-yl)-6-(2-méthylpyrimidin-4-yl)indolin-2-one
le 5-[3,3-diméthyl-1-(6-méthylpyrazin-2-yl)-2-oxo-indolin-6-yl]pyrimidine-2-carbonitrile ou
la 1-(5-éthylpyridin-3-yl)-3,3-diméthyl-6-(2-méthylpyrimidin-5-yl)indolin-2-one.

**5.** Composé de formule I selon la revendication 2, dans laquelle $Ar^1$ représente un groupe hétéroaryle à six chaînons, contenant un, deux ou trois hétéroatomes, choisis parmi N, S ou O, et $Ar^2$ représente un groupe hétéroaryle à cinq chaînons, contenant un, deux ou trois hétéroatomes, choisis parmi N, S ou O.

**6.** Composé de formule I selon la revendication 5, lesdits composés étant

la 3,3-diméthyl-1-(1-méthyl-1H-imidazol-4-yl)-6-(2-méthylpyrimidin-5-yl)indolin-2-one
la 3,3-diméthyl-1-(1-méthyl-1H-pyrazol-3-yl)-6-(2-méthylpyrimidin-5-yl)indolin-2-one
la 1-(1,5-diméthyl-1H-pyrazol-3-yl)-3,3-diméthyl-6-(2-méthylpyrimidin-5-yl)indolin-2-one
la 3,3-diméthyl-1-(1-méthyl-1H-imidazol-4-yl)-6-(6-méthylpyridin-3-yl)indolin-2-one
la 3,3-diméthyl-1-(1-méthyl-1H-imidazol-4-yl)-6-(2-méthylpyridin-4-yl)indolin-2-one
la 3,3-diméthyl-1-(1-méthyl-1H-pyrazol-3-yl)-6-(6-méthylpyridin-3-yl)indolin-2-one
la 6-(4-fluoropyridin-3-yl)-3,3-diméthyl-1-(1-méthyl-1H-imidazol-4-yl)indolin-2-one
la 6-(4-fluoropyridin-3-yl)-3,3-diméthyl-1-(1-méthyl-1H-pyrazol-3-yl)indolin-2-one
la 3,3-diméthyl-6-(2-méthylpyrimidin-5-yl)-1-(5-méthylthiophén-2-yl) indolin-2-one
la 3,3-diméthyl-1-(1-méthyl-1H-imidazol-5-yl)-6-(2-méthylpyrimidin-5-yl)indolin-2-one
la 3,3-diméthyl-1-(5-méthyl-1,3,4-oxadiazol-2-yl)-6-(2-méthylpyrimidin-5-yl)indolin-2-one
la 3,3-diméthyl-6-(2-méthylpyrimidin-5-yl)-1-(1H-pyrazol-3-yl)indolin-2-one
la 1-(1,2-diméthyl-1H-imidazol-4-yl)-3,3-diméthyl-6-(2-méthylpyrimidin-5-yl)indolin-2-one
la 1-(5-éthylpyridin-3-yl)-3,3-diméthyl-6-(2-méthylpyrimidin-5-yl)indolin-2-one
la 1-(1,5-diméthyl-1H-1,2,4-triazol-3-yl)-3,3-diméthyl-6-(2-méthylpyrimidin-5-yl)indolin-2-one
la 3,3-diméthyl-1-(1-méthyl-1H-pyrazol-4-yl)-6-(2-méthylpyrimidin-5-yl)indolin-2-one
la 3,3-diméthyl-1-(2-méthyl-1H-imidazol-4-yl)-6-(2-méthylpyrimidin-5-yl)indolin-2-one
la 3,3-diméthyl-1-(1-méthyl-1H-1,2,4-triazol-3-yl)-6-(2-méthylpyrimidin-5-yl)indolin-2-one
la 3,3-diméthyl-6-(2-méthylpyrimidin-5-yl)-1-(1H-pyrazol-4-yl)indolin-2-one
la 1-(1H-imidazol-4-yl)-3,3-diméthyl-6-(2-méthylpyrimidin-5-yl)indolin-2-one
la 3,3-diméthyl-1-(3-méthyl-1H-pyrazol-5-yl)-6-(2-méthylpyrimidin-5-yl)indolin-2-one
la 3,3-diméthyl-1-(1-méthyl-1H-1,2,3-triazol-4-yl)-6-(2-méthylpyrimidin-5-yl)indolin-2-one
la 3,3-diméthyl-1-(4-méthyl-1H-imidazol-2-yl)-6-(2-méthylpyrimidin-5-yl)indolin-2-one
la 3,3-diméthyl-1-(3-méthylisoxazol-5-yl)-6-(2-méthylpyrimidin-5-yl)indolin-2-one
la 1-(1H-imidazol-2-yl)-3,3-diméthyl-6-(2-méthylpyrimidin-5-yl)indolin-2-one
la 1-(1-éthyl-1H-pyrazol-3-yl)-3,3-diméthyl-6-(2-méthylpyrimidin-5-yl)indolin-2-one
la 3,3-diméthyl-6-(2-méthylpyrimidin-5-yl)-1-(2H-tétrazol-5-yl)indolin-2-one
la 3,3-diméthyl-1-(2-méthyl-2H-tétrazol-5-yl)-6-(2-méthylpyrimidin-5-yl)indolin-2-one
la 3,3-diméthyl-1-(1-méthyl-1H-pyrazol-4-yl)-6-(2-méthylpyridin-4-yl)-1H-pyrrolo [3,2-c]pyridin-2(3H)-one
la 3,3-diméthyl-1-(1-méthyl-1H-imidazol-4-yl)-6-(2-méthylpyridin-4-yl)-1H-pyrrolo [3,2-c]pyridin-2(3H)-one
la 3,3-diméthyl-1-(3-méthyl-1,2,4-thiadiazol-5-yl)-6-(2-méthylpyrimidin-5-yl)indolin-2-one
la 3,3-diméthyl-1-(3-méthylisothiazol-5-yl)-6-(2-méthylpyrimidin-5-yl)indolin-2-one
la 3,3-diméthyl-6-(2-méthylpyrimidin-5-yl)-1-(5-méthylthiazol-2-yl)indolin-2-one
la 1-(1-isopropyl-1H-pyrazol-3-yl)-3,3-diméthyl-6-(2-méthylpyrimidin-5-yl)indolin-2-one
la 3,3-diméthyl-1-(1-méthyl-1H-pyrazol-3-yl)-6-(5-méthylpyrazin-2-yl)indolin-2-one
la 3,3-diméthyl-6-(2-méthylpyrimidin-5-yl)-1-(1-(2,2,2-trifluoroéthyl)-1H-pyrazol-3-yl)indolin-2-one
la 1-(1-(2-méthoxyéthyl)-1H-pyrazol-3-yl)-3,3-diméthyl-6-(2-méthylpyrimidin-5-yl) indolin-2-one
ou
le 2-(3-(3,3-diméthyl-6-(2-méthylpyrimidin-5-yl)-2-oxoindolin-1-yl)-1H-pyrazol-1-yl)-N,N-diméthylacétamide.

**7.** Composé de formule I selon la revendication 2, dans laquelle $Ar^1$ représente un groupe hétéroaryle à cinq chaînons, contenant un, deux ou trois hétéroatomes, choisis parmi N, S ou O, et $Ar^2$ représente un groupe hétéroaryle à six chaînons, contenant un, deux ou trois hétéroatomes, choisis parmi N, S ou O.

**8.** Composé de formule I selon la revendication 7, lesdits composés étant

la 3,3-diméthyl-6-(4-méthylimidazol-1-yl)-1-(6-méthyl-3-pyridyl)indolin-2-one

la 6-(4-cyclopropylimidazol-1-yl)-3,3-diméthyl-1-(2-méthyl-4-pyridyl)indolin-2-one
la 3,3-diméthyl-6-(4-méthylimidazol-1-yl)-1-(2-méthyl-4-pyridyl)indolin-2-one
la 3,3-diméthyl-6-(1-méthyl-1H-pyrazol-3-yl)-1-(2-méthylpyrimidin-5-yl)indolin-2-one
la 3,3-diméthyl-6-(1-méthyl-1H-imidazol-4-yl)-1-(2-méthylpyrimidin-5-yl)indolin-2-one
la 6-(4-isopropylimidazol-1-yl)-3,3-diméthyl-1-(6-méthyl-3-pyridyl)indolin-2-one
la 3,3-diméthyl-6-(5-méthyl-1,3,4-oxadiazol-2-yl)-1-(6-méthyl-3-pyridyl)indolin-2-one
la 3,3-diméthyl-6-(5-méthyl-1,3,4-oxadiazol-2-yl)-1-(2-méthyl-4-pyridyl)indolin-2-one
la 6-(4-isopropylimidazol-1-yl)-3,3-diméthyl-1-(2-méthyl-4-pyridyl)indolin-2-one
la 6-(4-isopropylimidazol-1-yl)-3,3-diméthyl-1-(2-méthylpyrimidin-5-yl)indolin-2-one
la 6-(4-cyclopropylimidazol-1-yl)-3,3-diméthyl-1-(2-méthylpyrimidin-5-yl)indolin-2-one
la 3,3-diméthyl-6-(5-méthyl-1,3,4-oxadiazol-2-yl)-1-(2-méthylpyrimidin-5-yl)indolin-2-one
la 3,3-diméthyl-6-(4-méthylimidazol-1-yl)-1-(2-méthylpyrimidin-5-yl)indolin-2-one
la 3,3-diméthyl-6-(2-méthyloxazol-5-yl)-1-(2-méthylpyrimidin-5-yl)indolin-2-one ou
la 3,3-diméthyl-6-(3-méthyl-1,2,4-oxadiazol-5-yl)-1-(2-méthylpyrimidin-5-yl)indolin-2-one.

**9.** Composé de formule I selon la revendication 2, dans laquelle $Ar^1$ et $Ar^2$ représentent tous les deux un groupe hétéroaryle à cinq chaînons, contenant un, deux ou trois hétéroatomes, choisis parmi N, S ou O.

**10.** Composé de formule I selon la revendication 9, lesdits composés étant

la 6-(4-isopropylimidazol-1-yl)-3,3-diméthyl-1-(1-méthylpyrazol-3-yl)indolin-2-one
la 6-(4-cyclopropylimidazol-1-yl)-3,3-diméthyl-1-(1-méthylimidazol-4-yl)indolin-2-one
la 6-(4-cyclopropylimidazol-1-yl)-3,3-diméthyl-1-(1-méthylpyrazol-3-yl)indolin-2-one
la 3,3-diméthyl-1-(1-méthylimidazol-4-yl)-6-(2-méthyloxazol-5-yl)indolin-2-one
la 3,3-diméthyl-6-(2-méthyloxazol-5-yl)-1-(1-méthylpyrazol-3-yl)indolin-2-one
la 3,3-diméthyl-1-(1-méthylimidazol-4-yl)-6-(5-méthyl-1,3,4-oxadiazol-2-yl)indolin-2-one ou
la 3,3-diméthyl-6-(4-méthyl-1H-imidazol-1-yl)-1-(1-méthyl-1H-imidazol-4-yl)indolin-2-one.

**11.** Composé de formule I selon la revendication 2, dans laquelle $Ar^2$ représente un benzo[b]thiophényle, et les autres substituants sont tels que décrits dans la revendication 1.

**12.** Composé de formule I selon la revendication 11, ledit composé étant la 1-(benzo[b]thiophén-4-yl)-3,3-diméthyl-6-(2-méthylpyrimidin-5-yl)indolin-2-one.

**13.** Composé de formule I selon la revendication 1, dans laquelle X représente N et les autres substituants sont tels que décrits dans la revendication 1.

**14.** Composé de formule I selon la revendication 13, lesdits composés étant

la 3,3-diméthyl-1-(1-méthyl-1H-imidazol-4-yl)-6-(2-méthylpyrimidin-5-yl)-1H-pyrrolo [3,2-c]pyridin-2(3H)-one
la 3,3-diméthyl-1-(1-méthyl-1H-pyrazol-3-yl)-6-(2-méthylpyrimidin-5-yl)-1H-pyrrolo [3,2-c]pyridin-2(3H)-one
la 3,3-diméthyl-1-(2-méthylpyridin-4-yl)-6-(2-méthylpyrimidin-5-yl)-1H-pyrrolo [3,2-c]pyridin-2(3H)-one
la 3,3-diméthyl-1-(6-méthylpyridazin-3-yl)-6-(2-méthylpyridin-4-yl)-1H-pyrrolo [3,2-c]pyridin-2(3H)-one
la 3,3-diméthyl-1-(1-méthyl-1H-pyrazol-4-yl)-6-(2-méthylpyridin-4-yl)-1H-pyrrolo [3,2-c]pyridin-2(3H)-one
la 3,3-diméthyl-1-(1-méthyl-1H-imidazol-4-yl)-6-(2-méthylpyridin-4-yl)-1H-pyrrolo [3,2-c]pyridin-2(3H)-one ou
la 6-(4-fluorophényl)-3,3-diméthyl-1-(2-méthylpyrimidin-5-yl)-1H-pyrrolo[3,2-c]pyridin-2(3H)-one.

**15.** Association d'un composé de formule I selon l'une quelconque des revendications 1 à 14 avec un médicament antipsychotique, antidépresseur, anxiolytique ou psychorégulateur commercialisé connu.

**16.** Association selon la revendication 15, dans laquelle le médicament antipsychotique commercialisé est l'olanzapine (Zyprexa), la clozapine (Clozaril), la rispéridone (Risperdal), l'aripiprazole (Abilify) ou la ziprasidone.

**17.** Association selon la revendication 15, dans laquelle le médicament antidépresseur commercialisé est le citalopram (Celexa), l'escitalopram (Lexapro, Cipralex), la paroxétine (Paxil, Seroxat), la fluoxétine (Prozac), la sertraline (Zoloft, Lustral), la duloxétine (Cymbalta), le milnacipran (Ixel, Savella), la venlafaxine (Effexor) ou la mirtazapine (Remeron).

**18.** Association selon la revendication 15, dans laquelle le médicament anxiolytique commercialisé est l'alprazolam

(Helex, Xanax, Xanor, Onax, Alprox, Restyl, Tafil, Paxal), le chlordiazépoxide (Librium, Risolid, Elenium), le clonazépam (Rivotril, Klonopin, Iktorivil, Paxam), le diazépam (Antenex, Apaurin, Apzepam, Apozepam, Hexalid, Pax, Stesolid, Stedon, Valium, Vival, Valaxona), l'estazolam (ProSom), l'eszopiclone (Lunesta), le zaleplon (Sonata, Starnoc), le zolpidem (Ambien, Nytamel, Stilnoct, Stilnox, Zoldem, Zolnod), la prégabaline (Lyrica) ou la gabapentine (Fanatrex, Gabarone, Gralise, Neurontin, Nupentin).

**19.** Association selon la revendication 15, dans laquelle le médicament psychorégulateur commercialisé est la carbamazépine (Tegretol), la lamotrigine (Lamictal), le lithium (Eskalith, Lithane, Lithobid) et l'acide valproïque (Dépakote).

**20.** Procédé de préparation d'un composé de formule I tel que décrit dans l'une quelconque des revendications 1 à 14, comprenant :
la réaction d'un composé de formule

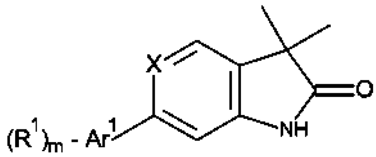

7

avec un composé de formule

$(R^2)_n$ - $Ar^2$ - Y 8

pour obtenir un composé de formule

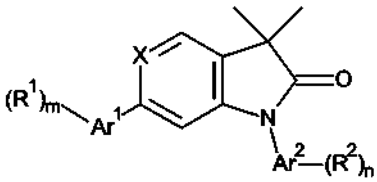

I

dans laquelle Y représente Cl, Br ou I et les autres groupes ont la signification telle que décrite dans la revendication 1, et,
si souhaité, la conversion des composés obtenus en des sels d'addition d'acide pharmaceutiquement acceptables.

**21.** Composé selon l'une quelconque des revendications 1 à 14 destiné à être utilisé comme substance thérapeutiquement active.

**22.** Composition pharmaceutique comprenant un composé selon l'une quelconque des revendications 1 à 14 et un excipient thérapeutiquement actif, destinée à être utilisée dans le traitement de maladies du système nerveux central associées aux symptômes positifs (psychose) et négatifs de la schizophrénie, de la toxicomanie, de l'alcoolisme et de la dépendance aux médicaments, de troubles obsessionnels compulsifs, de troubles cognitifs, de troubles bipolaires, de troubles de l'humeur, de la dépression majeure, de la dépression résistante au traitement, de troubles de l'anxiété, de la maladie d'Alzheimer, de l'autisme, de la maladie de Parkinson, de la douleur chronique, du trouble de la personnalité limite, d'une maladie neurodégénérative, de troubles du sommeil, du syndrome de fatigue chronique, de raideurs, d'une maladie inflammatoire, de l'asthme, de la maladie de Huntington, d'un trouble de déficit de l'attention/hyperactivité (TDAH), d'une sclérose latérale amyotrophique, de l'épilepsie, des effets de l'arthrite, d'une maladie auto-immune, d'infections virales et fongiques, de maladies cardiovasculaires, de maladies ophtalmologiques et de maladies rétiniennes inflammatoires et de problèmes d'équilibre.

**23.** Utilisation d'un composé de formule I selon l'une quelconque des revendications 1 à 14, pour la fabrication d'un médicament destiné au traitement de maladies du système nerveux central associées aux symptômes positifs (psychose) et négatifs de la schizophrénie, de la toxicomanie, de l'alcoolisme et de la dépendance aux médicaments, de troubles obsessionnels compulsifs, de troubles cognitifs, de troubles bipolaires, de troubles de l'humeur, de la dépression majeure, de la dépression résistante au traitement, de troubles de l'anxiété, de la maladie d'Alzheimer,

de l'autisme, de la maladie de Parkinson, de la douleur chronique, du trouble de la personnalité limite, d'une maladie neurodégénérative, de troubles du sommeil, du syndrome de fatigue chronique, de raideurs, d'une maladie inflammatoire, de l'asthme, de la maladie de Huntington, d'un trouble de déficit de l'attention/hyperactivité (TDAH), d'une sclérose latérale amyotrophique, de l'épilepsie, des effets de l'arthrite, d'une maladie auto-immune, d'infections virales et fongiques, de maladies cardiovasculaires, de maladies ophtalmologiques et de maladies rétiniennes inflammatoires et de problèmes d'équilibre.

**24.** Composé selon l'une quelconque des revendications 1 à 14 destiné à être utilisé dans le traitement de maladies du système nerveux central associées aux symptômes positifs (psychose) et négatifs de la schizophrénie, de la toxicomanie, de l'alcoolisme et de la dépendance aux médicaments, de troubles obsessionnels compulsifs, de troubles cognitifs, de troubles bipolaires, de troubles de l'humeur, de la dépression majeure, de la dépression résistante au traitement, de troubles de l'anxiété, de la maladie d'Alzheimer, de l'autisme, de la maladie de Parkinson, de la douleur chronique, du trouble de la personnalité limite, d'une maladie neurodégénérative, de troubles du sommeil, du syndrome de fatigue chronique, de raideurs, d'une maladie inflammatoire, de l'asthme, de la maladie de Huntington, d'un trouble de déficit de l'attention/hyperactivité (TDAH), d'une sclérose latérale amyotrophique, de l'épilepsie, des effets de l'arthrite, d'une maladie auto-immune, d'infections virales et fongiques, de maladies cardiovasculaires, de maladies ophtalmologiques et de maladies rétiniennes inflammatoires et de problèmes d'équilibre.

Figure 1

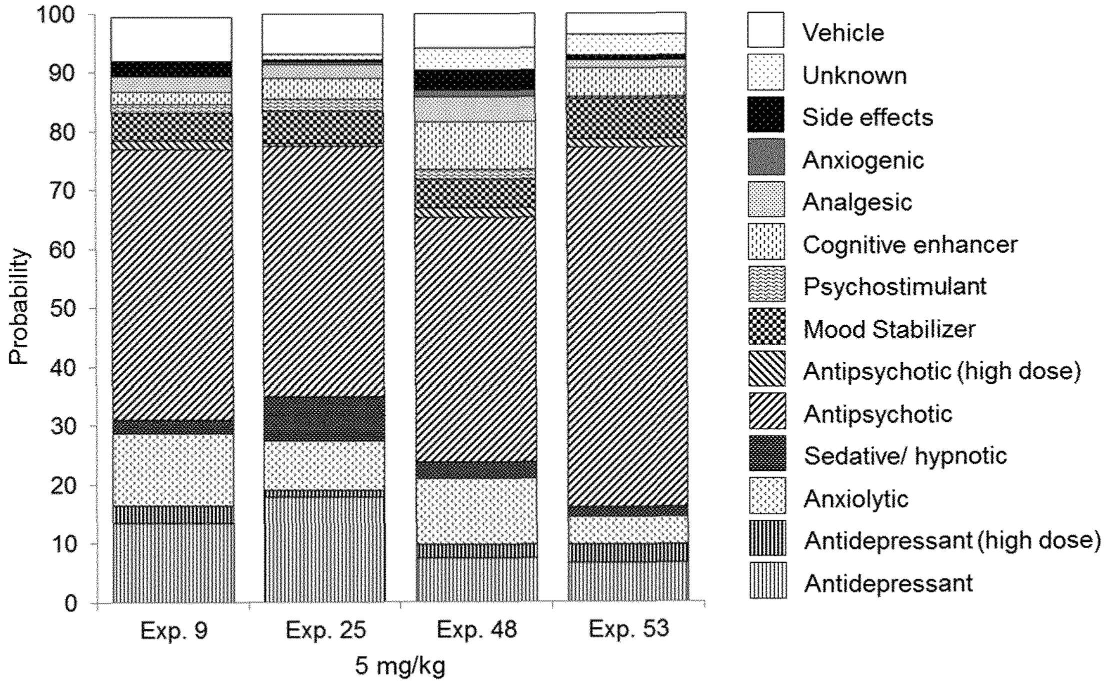


Figure 2

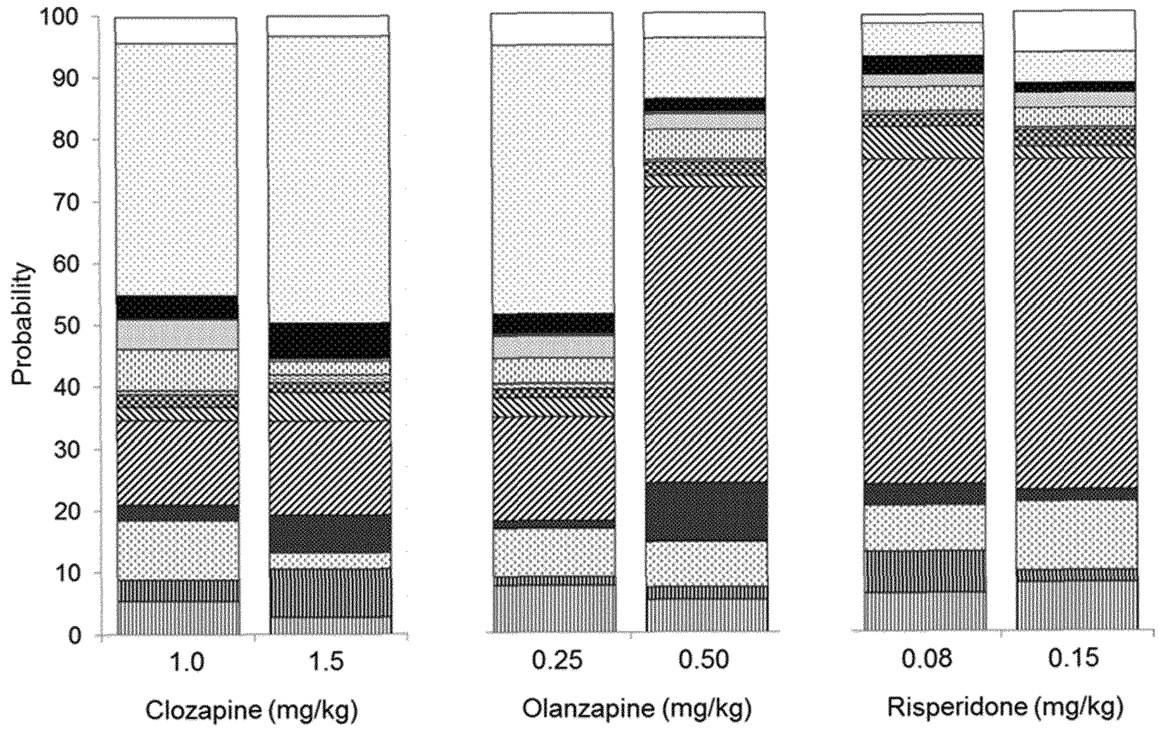

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9106545 A
- EP 2108641 A
- WO 2008046083 A
- US 2006253263 A
- WO 9857643 A
- WO 2004060902 A
- WO 0168105 A
- EP 1252910 A
- EP 1612210 A
- WO 2009018275 A
- WO 2009062990 A
- WO 2009064497 A
- US 2007213296 A
- WO 2012143726 A, Woolford
- WO 2010096395 A, Chen, Y.
- US 20050101785 A, Dolby


### Non-patent literature cited in the description


- **D. ALBERATI et al.** *Pharmacology, Biochemistry and Behavior,* 2010, vol. 97, 185-191
- **ROBERDS et al.** *Frontiers in Neuroscience,* 2011, vol. 5, 1-4
- **VADIM ALEXANDROV ; DANI BRUNNER ; TALEEN HANANIA ; EMER LEAHY.** *Eur. J. Pharmacol.,* 2015, vol. 750, 82-99
- **SUSAN A. MASINOA ; MASAHITO KAWAMURA JR. ; JESSICA L. COTEA ; REBECCA B. WILLIAMS ; DAVID N. RUSKINA.** *Neuropharmacology,* 2013, vol. 68, 116-121
- *Inflammation research,* 2011, vol. 60, 75-76
- *Trends in Pharmacological science,* 2006, vol. 27, 416-425
- *Psychopharmacology,* 1987, vol. 91, 434-439
- *World Journal of Diabetes,* vol. 1, 12-18
- *CNS Drugs,* 2012, vol. 26 (2), 135-53
- *J. Clin. Psychiatry,* 2010, vol. 71, 14-9
- *Pediatr. Drugs,* 2011, vol. 13 (5), 291-302
- *BMC Psychiatry,* 2011, vol. 11, 86
- *Journal of Psychopharmacology,* vol. 0 (0), 1-16
- *European Neuropsychopharmacology,* 2011, vol. 21, 429-449
- *Encephale, International J. of Neuropsychopharmacology,* 2011, vol. 14, 1029-104
- *International J. of Neuropsychopharmacology,* 2012, 1-12
- *J. of Neuropsychopharmacology,* 2011, vol. 0 (0), 1-15
- *J. Psychopharmacol.,* 11 January 2012
- *CNS Drugs,* 2010, vol. 2, 131-61
- *Current opinion in pediatrics,* 2011, vol. 23, 621-627
- *J. Clin. Psychiatry,* 2011, vol. 72 (9), 1270-1276
- *J. Clin. Psychiatry,* 2012, vol. 73 (1), 121-128
- *Movement Disorders,* 2011, vol. 26, 6
- *European Neuropsychopharmacology,* 2011, vol. 21, 282-286
- *J. Clin. Psychiatry,* 2011, vol. 72 (10), 1363-1365
- *J. Clin. Psychiatry,* 2011, vol. 72 (10), 1353-1362
- *European J. of Pharmacology,* 2012, vol. 678, 55-60
- *CHEMICAL ABSTRACTS,* 99365-40-9
- *CHEMICAL ABSTRACTS,* 158326-84-2
- *CHEMICAL ABSTRACTS,* 88491-61-6
- *CHEMICAL ABSTRACTS,* 56616-93-4
- *CHEMICAL ABSTRACTS,* 4595-60-2
- *CHEMICAL ABSTRACTS,* 15803-02-8
- *CHEMICAL ABSTRACTS,* 91749-26-7
- **HASNIK, ZBYNEK et al.** *Synlett,* 2008, vol. 4, 543-546
- *CHEMICAL ABSTRACTS,* 1618-47-9
- **MANN, H. B. ; WHITNEY, D. R.** On a Test of Whether one of Two Random Variables is Stochastically Larger than the Other. *Annals of Mathematical Statistics,* 1947, vol. 18 (1), 50-60